# EUROPEAN PATENT APPLICATION

(11) **EP 4 186 909 A1**
(43) Date of publication of application: **31.05.2023**
(21) Application number: 21846928.6
(22) Date of filing: 20.07.2021
(51) Int. Cl.: C07D 495/14, C07D 495/22, C07B 61/00, C09D 11/52, H01L 51/42, H01L 31/10, H01L 27/146, H01L 27/30, C09B 57/00

(54) **COMPOUND AND PHOTOELECTRIC CONVERSION ELEMENT USING SAME**

(30) Priority: 22.07.2020 JP 2020125820
(71) Applicant: SUMITOMO CHEMICAL COMPANY, LIMITED, Tokyo 103-6020 (JP)
(72) Inventor: ABE, Masanori, Osaka 554-8558 (JP); ARAKI, Takafumi, Osaka 554-8558 (JP); NISHI, Miki, Ehime 792-0015 (JP); YOSHIKAWA, Eiji, Osaka 554-8558 (JP)
(74) Representative: Vossius & Partner Patentanwälte Rechtsanwälte mbB
(86) International application number: PCT/JP2021/027111
(87) International publication number: WO 2022/019300

(57) **Abstract**

Provided is a novel semiconductor material. A compound represented by the following formula (I): A¹-B¹-A¹ (I) (In the formula (I), A¹ represents an electron-withdrawing group, B¹ is a divalent group including two or more constituent units that are linked by a single bond to constitute a π-conjugated system, at least one of the two or more constituent units is a first constituent unit represented by the following formula (II), and the remaining second constituent unit other than the first constituent unit is a divalent group including an unsaturated bond, an arylene group, or a heteroarylene group.) 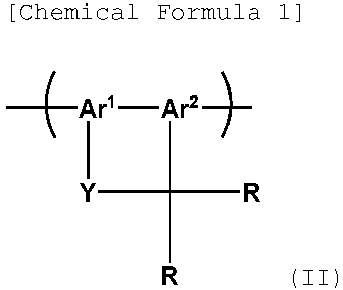 (In the formula (II), Ar¹, Ar², Y, and R are as defined in the specification.).

## Description

### TECHNICAL FIELD

The present invention relates to a compound and a photoelectric conversion element with the compound used as a semiconductor material.

### BACKGROUND ART

Photoelectric conversion elements are extremely useful devices from the viewpoints of, for example, energy saving and reduced carbon dioxide emission, and have been attracting attention.

The photoelectric conversion element is an element including at least a pair of electrodes of an anode and a cathode, and an active layer provided between the pair of electrodes. In the case of the photoelectric conversion element, at least one of the pair of electrodes is made from a transparent or translucent material, and light is made incident on the active layer from the transparent or translucent electrode side. Charges (holes and electrons) are generated in the active layer by the energy (hv) of the light made incident on the active layer, and the generated holes move toward the anode, whereas the electrons move toward the cathode. Then, the charges reaching the anode and the cathode are extracted to the outside of the element.

In recent years, photoelectric conversion elements have been required to have characteristics and the like further improved. Thus, various semiconductor materials have been further developed and reported (see Patent Document 1).

### PRIOR ART DOCUMENT

### PATENT DOCUMENT

Patent Document 1: Chinese Patent Application Publication No. 107652304

### SUMMARY OF THE INVENTION

### PROBLEMS TO BE SOLVED BY THE INVENTION

The n-type semiconductor material reported in Patent Document 1 mentioned above is, however, not sufficient to satisfy the characteristics required for recent photoelectric conversion elements.

Accordingly, semiconductor materials capable of satisfying the characteristics required for the photoelectric conversion elements have been further required.

### MEANS FOR SOLVING THE PROBLEMS

As a result of intensive studies carried out to solve the problem mentioned above, the inventor has found that the problem can be solved with a compound that has a predetermined structure as described later, and have achieved the present invention.

Accordingly, the present invention provides the following [1] to [14].
[1] A compound represented by the following formula (I):

   A¹-B¹-A¹ (I)

   (In the formula (I),
   A¹ represents an electron-withdrawing group,
   B¹ is a divalent group including two or more constituent units that are linked by a single bond to constitute a π-conjugated system, at least one of the two or more constituent units is a first constituent unit represented by the following formula (II), and the remaining second constituent unit other than the first constituent unit is a divalent group including an unsaturated bond, an arylene group, or a heteroarylene group.
   The two groups A¹ may be identical to or different from each other. In the case of having two or more first constituent units, the two or more first constituent units may be identical to or different from each other. In the case of having two or more second constituent units, the two or more second constituent units may be identical to or different from each other.) 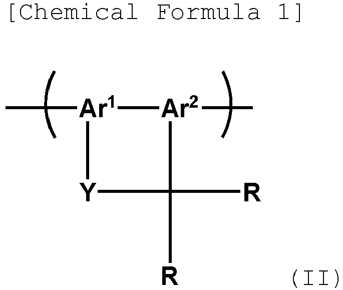 (In the formula (II),
      Ar¹ and Ar² each independently represent an aromatic carbocyclic ring optionally having a substituent or an aromatic heterocyclic ring optionally having a substituent,
      Y represents a group represented by -C(=O)- or an oxygen atom,
      the groups R each independently represent
      a hydrogen atom,
      a halogen atom,
      an alkyl group optionally having a substituent,
      a cycloalkyl group optionally having a substituent,
      an aryl group optionally having a substituent,
      an alkyloxy group optionally having a substituent,
      a cycloalkyloxy group optionally having a substituent,
      an aryloxy group optionally having a substituent,
      an alkylthio group optionally having a substituent,
      a cycloalkylthio group optionally having a substituent,
      an arylthio group optionally having a substituent,
      a monovalent heterocyclic group optionally having a substituent,
      a substituted amino group optionally having a substituent,
      an acyl group optionally having a substituent,
      an imine residue optionally having a substituent,
      an amide group optionally having a substituent,
      an acid imide group optionally having a substituent,
      a substituted oxycarbonyl group optionally having a substituent,
      an alkenyl group optionally having a substituent,
      a cycloalkenyl group optionally having a substituent,
      an alkynyl group optionally having a substituent,
      a cycloalkynyl group optionally having a substituent,
      a cyano group,
      a nitro group,
      a group represented by -C(=O)-R^{a}, or
      a group represented by -SO₂-R^{b}, and
      R^{a} and R^{b} each independently represent
      a hydrogen atom,
      an alkyl group optionally having a substituent,
      an aryl group optionally having a substituent,
      an alkyloxy group optionally having a substituent, an aryloxy group optionally having a substituent, or
      a monovalent heterocyclic group optionally having a substituent.
      the multiple groups R may be identical to or different from each other.)
[2] The compound according to [1], where B¹ includes two or more of the first constituent units.
[3] The compound according to [1] or [2], where the first constituent unit is a constituent unit represented by the following formula (III): 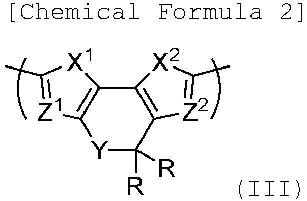 (In the formula (III),
   Y and R are as defined above,
   X¹ and X² each independently represent a sulfur atom or an oxygen atom, and
   Z¹ and Z² each independently represent a group represented by =C(R)- or a nitrogen atom.)
[4] The compound according to any one of [1] to [3], where B¹ includes three or more of the first constituent units.
[5] The compound according to any one of [1] to [4], where the second constituent unit is selected from the group consisting of a divalent group including an unsaturated bond and groups represented by the following formulas (IV-1) to (IV-9): 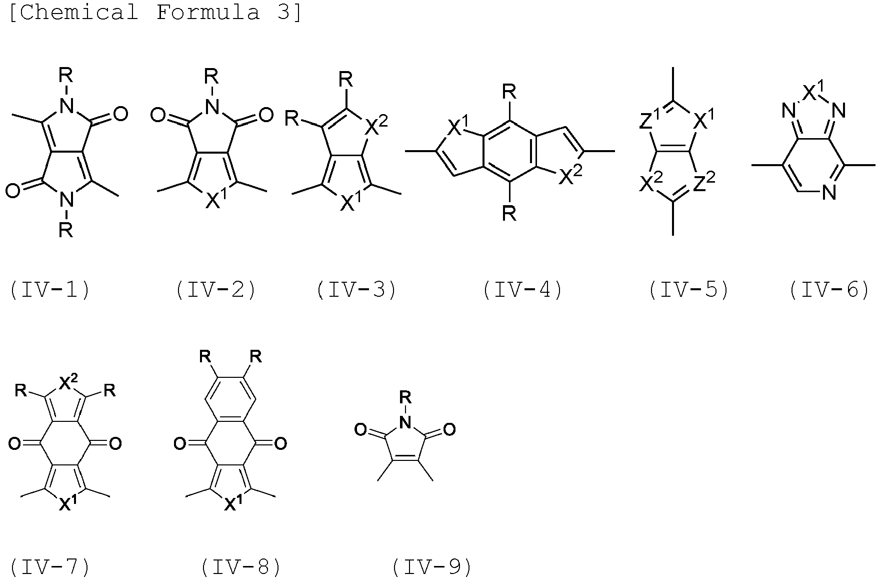 (In the formulas (IV-1) to (IV-9), X¹, X², Z¹, Z², and R are as defined above.
   In the case of having two groups R, the two groups R may be identical to or different from each other.)
[6] The compound according to any one of [1] to [3], where B¹ is a divalent group having the two or more and four or less first constituent units linked.
[7] The compound according to [5], where B¹ is a divalent group that has any one structure selected from the group consisting of structures represented by the following formulas (V-1) to (V-9):

   -CU1-CU2-CU1- (V-1)

   -CU1-CU2-CU1-CU2-CU1- (V-2)

   -CU2-CU1-CU2-CU1-CU2- (V-3)

   -CU1-CU2-CU1-CU2-CU1-CU2-CU1- (V-4)

   -CU1-CU1- (V-5)

   -CU2-CU1-CU2- (V-6)

   -CU1-CU1-CU1- (V-7)

   -CU2-CU1-CU1-CU2- (V-8)

   -CU2-CU1-CU1-CU1-CU2- (V-9)

   (In the formulas (V-1) to (V-9),
   CU1 represents the first constituent unit, and
   CU2 represents the second constituent unit.
   In the case of having two or more units CU1, the two or more units CU1 may be identical to or different from each other, and in the case of having two or more units CU2, the two or more units CU2 may be identical to or different from each other.)
[8] A composition including a p-type semiconductor material and an n-type semiconductor material, where the n-type semiconductor material includes the compound according to any one of [1] to [7].
[9] An ink including the composition according to [8] and a solvent.
[10] A photoelectric conversion element including: an anode; a cathode; and an active layer provided between the anode and the cathode and including a p-type semiconductor material and an n-type semiconductor material, where the compound according to any one of [1] to [7] is included as the n-type semiconductor material.
[11] The photoelectric conversion element according to [10], where the photoelectric conversion element is a photodetection element.
[12] An image sensor including the photoelectric conversion element according to [11].
[13] A fingerprint authentication device including the photoelectric conversion element according to [11].
[14] A vein authentication device including the photoelectric conversion element according to [11].

In addition, the compound according to the present invention may be the following aspect [X].
[X] The compound according to [1], excluding cases where the compound is any compound represented by the following formulas X-1 to X-3.

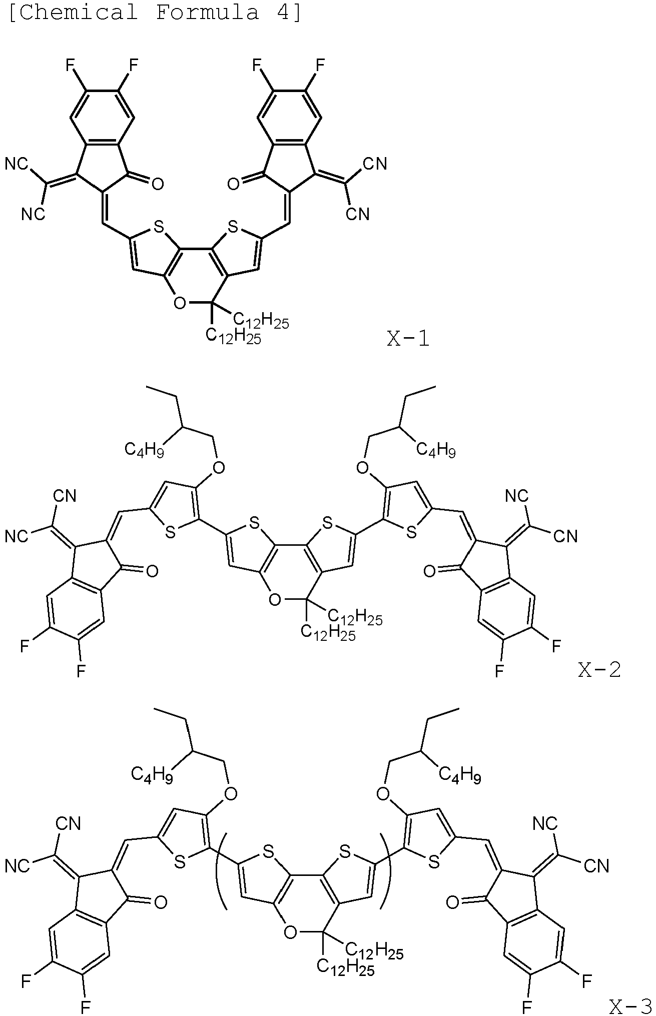

### EFFECT OF THE INVENTION

The present invention makes it possible to provide a novel compound capable of suppressing a decrease in external quantum efficiency of a photoelectric conversion element with respect to a heat treatment in a step of manufacturing the photoelectric conversion element or a step of incorporating the photoelectric conversion element into a device to which the photoelectric conversion element is applied, and improving the heat resistance, and a photoelectric conversion element with the compound used.

### BRIEF DESCRIPTION OF THE DRAWINGS

Fig. 1 is a diagram schematically illustrating a configuration example of a photoelectric conversion element.
Fig. 2 is a diagram schematically illustrating a configuration example of an image detection unit.
Fig. 3 is a diagram schematically illustrating a configuration example of a fingerprint detection unit.
Fig. 4 is a diagram schematically illustrating a configuration example of an image detection unit for an X-ray imaging device.
Fig. 5 is a diagram schematically illustrating a configuration example of a vein detection unit for a vein authentication device.
Fig. 6 is a diagram schematically illustrating a configuration example of an image detection unit for an indirect TOF-type distance measuring device.

### MODE FOR CARRYING OUT THE INVENTION

Hereinafter, a compound according to an embodiment of the present invention will be described, and a photoelectric conversion element with the compound according to the present embodiment used will be further described with reference to the drawings. It is to be noted that the drawings merely schematically illustrate the shapes, sizes, and layouts of the constituent element to the extent that the invention can be understood. The present invention is not to be considered limited by the following description, and each constituent element can be appropriately changed without departing from the scope of the present invention. In addition, configurations according to the embodiments of the present invention are not necessarily manufactured or used in the layouts illustrated in the drawings.

The terms commonly used in the following description will be described first.

The "non-fullerene compound" refers to a compound that is neither fullerene nor a fullerene derivative.

The "π-conjugated system" means a system in which π electrons are delocalized at multiple bonds.

The "polymer compound" means a polymer that has a molecular weight distribution and a polystyrene-equivalent number average molecular weight of 1 × 10³ or more and 1 × 10⁸ or less. It is to be noted that the constituent unit included in the polymer compound is 100 mol% in total.

The "constituent unit" means one or more residues derived from a raw material compound (monomer), present in the compound according to the present embodiment and the polymer compound.

The "hydrogen atom" may be a light hydrogen atom or a deuterium atom.

Examples of the "halogen atom" include a fluorine atom, a chlorine atom, a bromine atom, and an iodine atom.

The aspect of "optionally having a substituent" includes both aspects in which all of hydrogen atoms constituting a compound or a group are unsubstituted and aspects in which some or all of one or more hydrogen atoms are substituted with a substituent.

Examples of the "substituent" include a halogen atom, an alkyl group, a cycloalkyl group, an alkenyl group, a cycloalkenyl group, an alkynyl group, a cycloalkynyl group, an alkyloxy group, a cycloalkyloxy group, an alkylthio group, a cycloalkylthio group, an aryl group, an aryloxy group, an arylthio group, a monovalent heterocyclic group, a substituted amino group, an acyl group, an imine residue, an amide group, an acid imide group, a substituted oxycarbonyl group, a cyano group, an alkylsulfonyl group, and a nitro group. It is to be noted that the number of carbon atoms in this specification excludes the number of carbon atoms of the substituent.

In this specification, unless otherwise specified, the "alkyl group" may be linear, branched, or cyclic. The number of carbon atoms of the linear alkyl group is typically 1 to 50, preferably 1 to 30, more preferably 1 to 20, excluding the number of carbon atoms of the substituent. The number of carbon atoms of the branched or cyclic alkyl group is typically 3 to 50, preferably 3 to 30, more preferably 4 to 20, excluding the number of carbon atoms of the substituent.

Specific examples of the alkyl group include a methyl group, an ethyl group, an n-propyl group, an isopropyl group, an n-butyl group, an isobutyl group, a tert-butyl group, an n-pentyl group, an isoamyl group, a 2-ethylbutyl group, an n-hexyl group, a cyclohexyl group, an n-heptyl group, a cyclohexylmethyl group, a cyclohexylethyl group, an n-octyl group, a 2-ethylhexyl group, a 3-n-propylheptyl group, an adamantyl group, an n-decyl group, a 3,7-dimethyloctyl group, a 2-ethyloctyl group, a 2-n-hexyldecyl group, an n-dodecyl group, a tetradecyl group, a hexadecyl group, an octadecyl group, and an icosyl group.

The alkyl group may have a substituent. The alkyl group having a substituent is, for example, the alkyl group exemplified above in which a hydrogen atom is substituted with a substituent such as an alkyloxy group, an aryl group, or a fluorine atom.

Specific examples of the alkyl having a substituent include a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-dihexylphenyl)propyl group, and a 6-ethyloxyhexyl group.

The "cycloalkyl group" may be a monocyclic group or a polycyclic group. The cycloalkyl group may have a substituent. The number of carbon atoms of the cycloalkyl group is typically 3 to 30, preferably 12 to 19, excluding the number of carbon atoms of the substituent.

Examples of the cycloalkyl group include alkyl groups having no substituent, such as a cyclopentyl group, a cyclohexyl group, a cycloheptyl group, and an adamantyl group, and these groups in which a hydrogen atom is substituted with a substituent such as an alkyl group, an alkyloxy group, an aryl group, or a fluorine atom.

Specific examples of the cycloalkyl group having a substituent include a methylcyclohexyl group and an ethylcyclohexyl group.

The "p-valent aromatic carbocyclic group" means a remaining atomic group excluding p hydrogen atoms directly bonded to carbon atoms constituting the ring from an aromatic hydrocarbon optionally having a substituent. The p-valent aromatic carbocyclic group may further have a substituent.

The "aryl group" is a monovalent aromatic carbocyclic group, which means a remaining atomic group excluding one hydrogen atom directly bonded to a carbon atom constituting the ring from an aromatic hydrocarbon optionally having a substituent.

The aryl group may have a substituent. Specific examples of the aryl group include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group, a 4-phenylphenyl group, and these groups in which a hydrogen atom is substituted with a substituent such as an alkyl group, an alkyloxy group, an aryl group, or a fluorine atom.

The "alkyloxy group" may be linear, branched, or cyclic. The number of carbon atoms of the linear alkyloxy group is typically 1 to 40, preferably 1 to 10, excluding the number of carbon atoms of the substituent. The number of carbon atoms of the branched or cyclic alkyloxy group is typically 3 to 40, preferably 4 to 10, excluding the number of carbon atoms of the substituent.

The alkyloxy group may have a substituent. Specific examples of the alkyloxy group include a methoxy group, an ethoxy group, an n-propyloxy group, an isopropyloxy group, an n-butyloxy group, an isobutyloxy group, a tert-butyloxy group, an n-pentyloxy group, an n-hexyloxy group, a cyclohexyloxy group, an n-heptyloxy group, an n-octyloxy group, a 2-ethylhexyloxy group, an n-nonyloxy group, an n-decyloxy group, a 3,7-dimethyloctyloxy group, a 3-heptyldodecyloxy group, a lauryloxy group, and these groups in which a hydrogen atom is substituted with an alkyloxy group, an aryl group, or a fluorine atom.

The cycloalkyl group included in the "cycloalkyloxy group" may be a monocyclic group or a polycyclic group. The cycloalkyloxy group may have a substituent. The number of carbon atoms of the cycloalkyloxy group is typically 3 to 30, preferably 12 to 19, excluding the number of carbon atoms of the substituent.

Examples of the cycloalkyloxy group include cycloalkyloxy groups having no substituent, such as a cyclopentyloxy group, a cyclohexyloxy group, and a cycloheptyloxy group, and these groups in which a hydrogen atom is substituted with a fluorine atom or an alkyl group.

The number of carbon atoms of the "aryloxy group" is typically 6 to 60, preferably 6 to 48, excluding the number of carbon atoms of the substituent.

The aryloxy group may have a substituent. Specific examples of the aryloxy group include a phenoxy group, a 1-naphthyloxy group, a 2-naphthyloxy group, a 1-anthracenyloxy group, a 9-anthracenyloxy group, a 1-pyrenyloxy group, and these groups in which a hydrogen atom is substituted with a substituent such as an alkyl group, an alkyloxy group, or a fluorine atom.

The "alkylthio group" may be linear, branched, or cyclic. The number of carbon atoms of the linear alkylthio group is typically 1 to 40, preferably 1 to 10, excluding the number of carbon atoms of the substituent. The numbers of carbon atoms of the branched and cyclic alkylthio groups are typically 3 to 40, preferably 4 to 10, excluding the number of carbon atoms of the substituent.

The alkylthio group may have a substituent. Specific examples of the alkylthio group include a methylthio group, an ethylthio group, a propylthio group, an isopropylthio group, a butylthio group, an isobutylthio group, a tert-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, a laurylthio group, and a trifluoromethylthio group.

The cycloalkyl group included in the "cycloalkylthio group" may be a monocyclic group or a polycyclic group. The cycloalkylthio group may have a substituent. The number of carbon atoms of the cycloalkylthio group is typically 3 to 30, preferably 12 to 19, excluding the number of carbon atoms of the substituent.

Examples of the cycloalkylthio group optionally having a substituent include a cyclohexylthio group.

The number of carbon atoms of the "arylthio group" is typically 6 to 60, preferably 6 to 48, excluding the number of carbon atoms of the substituent.

The arylthio group may have a substituent. Examples of the arylthio group include a phenylthio group and C1 to C12 alkyloxyphenylthio groups (C1 to C12 indicate that the numbers of carbon atoms of the groups immediately thereafter are 1 to 12. The same applies hereinafter.), C1 to C 12 alkylphenylthio groups, a 1-naphthylthio group, a 2-naphthylthio group, and a pentafluorophenylthio group.

The "p-valent heterocyclic group" (p represents an integer of 1 or more) means a remaining atomic group excluding p hydrogen atoms of hydrogen atoms directly bonded to carbon atoms or heteroatoms constituting the ring from a heterocyclic compound optionally having a substituent.

The p-valent heterocyclic group may further have a substituent. The number of carbon atoms of the p-valent heterocyclic group is typically 2 to 30, preferably 2 to 6, excluding the number of carbon atoms of the substituent.

Examples of the substituent optionally included in the heterocyclic compound include a halogen atom, an alkyl group, an aryl group, an alkyloxy group, an aryloxy group, an alkylthio group, an arylthio group, a monovalent heterocyclic group, a substituted amino group, an acyl group, an imine residue, an amide group, an acid imide group, a substituted oxycarbonyl group, an alkenyl group, an alkynyl group, a cyano group, and a nitro group. The p-valent heterocyclic group encompasses a "p-valent aromatic heterocyclic group".

The "p-valent aromatic heterocyclic group" means a remaining atomic group excluding p hydrogen atoms of hydrogen atoms directly bonded to carbon atoms or heteroatoms constituting the ring from an aromatic heterocyclic compound optionally having a substituent. The p-valent aromatic heterocyclic group may further have a substituent.

The aromatic heterocyclic compound encompasses a compound with an aromatic ring condensed to a heterocyclic ring, even if the heterocyclic ring itself exhibits no aromaticity, in addition to a compound in which the heterocyclic ring itself exhibits aromaticity.

Specific examples of the compound in which the heterocycle itself exhibits aromaticity, among the aromatic heterocyclic compounds, include an oxadiazole, a thiadiazole, a thiazole, an oxazole, a thiophene, a pyrrole, a phosphole, a furan, a pyridine, a pyrazine, a pyrimidine, a triazine, a pyridazine, a quinoline, an isoquinoline, a carbazole, and a dibenzophosphole.

Specific examples of the compound with the aromatic ring condensed to the heterocyclic ring, in which the heterocyclic ring itself exhibits no aromaticity, among the aromatic heterocyclic compounds, include a phenoxazine, a phenothiazine, a dibenzoborol, a dibenzosilole, and a benzopyran.

The number of carbon atoms of the monovalent heterocyclic group is typically 2 to 60, preferably 4 to 20, excluding the number of carbon atoms of the substituent.

The monovalent heterocyclic group may have a substituent, and specific examples of the monovalent heterocyclic group include a thienyl group, a pyrrolyl group, a furyl group, a pyridyl group, a piperidyl group, a quinolyl group, an isoquinolyl group, a pyrimidinyl group, a triazinyl group, and these groups in which a hydrogen atom is substituted with an alkyl group, an alkyloxy group, or the like.

The "substituted amino group" means an amino group having a substituent. Examples of the substituent included in the amino group include an alkyl group, an aryl group, and a monovalent heterocyclic group, and an alkyl group, an aryl group, or a monovalent heterocyclic group is preferred. The number of carbon atoms of the substituted amino group is typically 2 to 30.

Examples of the substituted amino group include dialkylamino groups such as a dimethylamino group and a diethylamino group; and diarylamino groups such as a diphenylamino group, a bis(4-methylphenyl)amino group, a bis(4-tert-butylphenyl)amino group, and a bis(3,5-di-tert-butylphenyl)amino group.

The "acyl group" may have a substituent. The number of carbon atoms of the acyl group is typically 2 to 20, preferably 2 to 18, excluding the number of carbon atoms of the substituent. Specific examples of the acyl group include an acetyl group, a propionyl group, a butyryl group, an isobutyryl group, a pivaloyl group, a benzoyl group, a trifluoroacetyl group, and a pentafluorobenzoyl group.

The "imine residue" means a remaining atomic group excluding one hydrogen atom directly bonded to a carbon atom or a nitrogen atom constituting a carbon atom-nitrogen atom double bond from an imine compound. The "imine compound" means an organic compound having a carbon atom-nitrogen atom double bond in the molecule. Examples of the imine compound include an aldimine, a ketimine, and a compound in which a hydrogen atom bonded to a nitrogen atom constituting a carbon atom-nitrogen atom double bond in an aldimine is substituted with an alkyl group or the like.

The number of carbon atoms of the imine residue is typically 2 to 20, preferably 2 to 18. Examples of the imine residue include a group represented by the following structural formula.

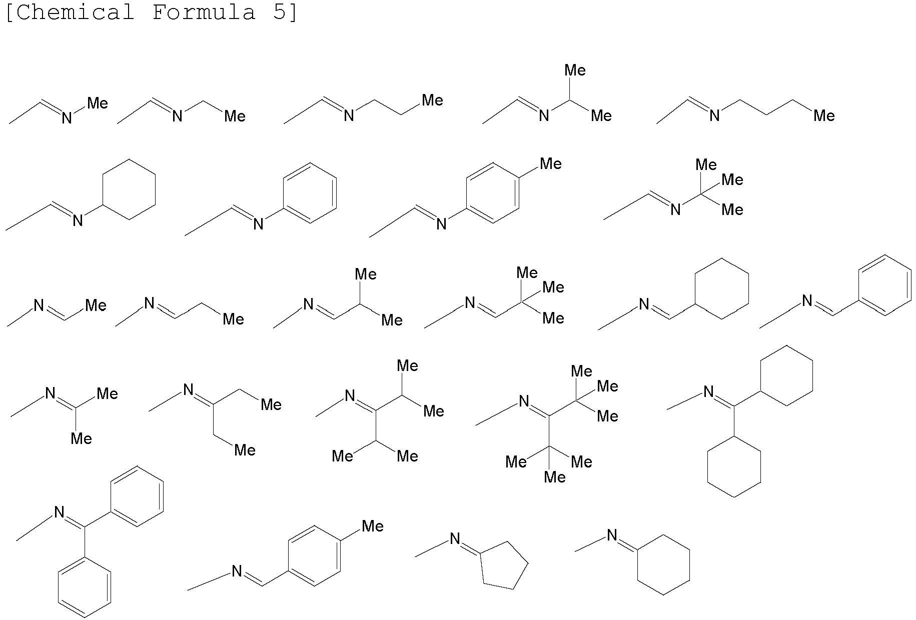

The "amide group" means a remaining atomic group excluding one hydrogen atom bonded to a nitrogen atom from an amide. The number of carbon atoms of the amide group is typically 1 to 20, preferably 1 to 18. Specific examples of the amide group include a formamide group, an acetamide group, a propionamide group, a butyroamide group, a benzamide group, a trifluoroacetamide group, a pentafluorobenzamide group, a diformamide group, a diacetamide group, a dipropionamide group, a dibutyroamide group, a dibenzamide group, a ditrifluoroacetamide group, and a dipentafluorobenzamide group.

The "acid imide group" means a remaining atomic group excluding one hydrogen atom bonded to a nitrogen atom from an acid amide. The number of carbon atoms of the acid imide group is typically 4 to 20. Specific examples of the acid imide group include a group represented by the following structural formula.

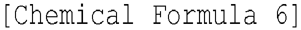

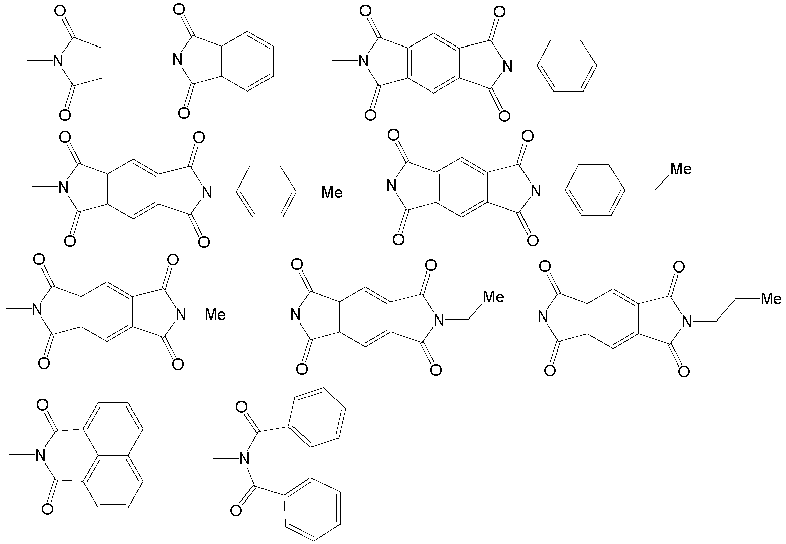

The "substituted oxycarbonyl group" means a group represented by R'-O-(C=O)-. In this regard, R' represents an alkyl group, an aryl group, an arylalkyl group, or a monovalent heterocyclic group.

The number of carbon atoms of the substituted oxycarbonyl group is typically 2 to 60, preferably 2 to 48, excluding the number of carbon atoms of the substituent.

Specific examples of the substituted oxycarbonyl group include a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentyloxycarbonyl group, a hexyloxycarbonyl group, a cyclohexyloxycarbonyl group, a heptyloxycarbonyl group, an octyloxycarbonyl group, a 2-ethylhexyloxycarbonyl group, a nonyloxycarbonyl group, a decyloxycarbonyl group, a 3,7-dimethyloctyloxycarbonyl group, a dodecyloxycarbonyl group, a trifluoromethoxycarbonyl group, a pentafluoroethoxycarbonyl group, a perfluorobutoxycarbonyl group, a perfluorohexyloxycarbonyl group, a perfluorooctyloxycarbonyl group, a phenoxycarbonyl group, a naphthoxycarbonyl group, and a pyridyloxycarbonyl group.

The "alkenyl group" may be linear, branched, or cyclic. The number of carbon atoms of the linear alkenyl group is typically 2 to 30, preferably 3 to 20, excluding the number of carbon atoms of the substituent. The number of carbon atoms of the branched or cyclic alkenyl group is typically 3 to 30, preferably 4 to 20, excluding the number of carbon atoms of the substituent.

The alkenyl group may have a substituent. Specific examples of the alkenyl group include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group, a 7-octenyl group, and these groups in which a hydrogen atom is substituted with an alkyl group, an alkyloxy group, an aryl group, or a fluorine atom.

The "cycloalkenyl group" may be a monocyclic group or a polycyclic group. The cycloalkenyl group may have a substituent. The number of carbon atoms of the cycloalkenyl group is typically 3 to 30, preferably 12 to 19, excluding the number of carbon atoms of the substituent.

Examples of the cycloalkenyl group include cycloalkenyl groups having no substituent, such as a cyclohexenyl group, and these groups in which a hydrogen atom is substituted with an alkyl group, an alkyloxy group, an aryl group, or a fluorine atom.

Examples of the cycloalkenyl group having a substituent include a methylcyclohexenyl group and an ethylcyclohexenyl group.

The "alkynyl group" may be linear, branched, or cyclic. The number of carbon atoms of the linear alkenyl group is typically 2 to 20, preferably 3 to 20, excluding the number of carbon atoms of the substituent. The number of carbon atoms of the branched or cyclic alkenyl group is typically 4 to 30, preferably 4 to 20, excluding the number of carbon atoms of the substituent.

The alkynyl group may have a substituent. Specific examples of the alkynyl group include an ethynyl group, a 1-propynyl group, a 2-propynyl group, a 2-butynyl group, a 3-butynyl group, a 3-pentynyl group, a 4-pentynyl group, a 1-hexynyl group, a 5-hexynyl group, and these groups in which a hydrogen atom is substituted with an alkyloxy group, an aryl group, or a fluorine atom.

The "cycloalkynyl group" may be a monocyclic group or a polycyclic group. The cycloalkynyl group may have a substituent. The number of carbon atoms of the cycloalkynyl group is typically 4 to 30, preferably 12 to 19, excluding the number of carbon atoms of the substituent.

Examples of the cycloalkynyl group include cycloalkynyl groups having no substituent, such as a cyclohexynyl group, and these groups in which a hydrogen atom is substituted with an alkyl group, an alkyloxy group, an aryl group, or a fluorine atom.

Examples of the cycloalkynyl group having a substituent include a methylcyclohexynyl group and an ethylcyclohexynyl group.

The "alkylsulfonyl group" may be linear or branched. The alkylsulfonyl group may have a substituent. The number of carbon atoms of the alkylsulfonyl group is typically 1 to 30, excluding the number of carbon atoms of the substituent. Specific examples of the alkylsulfonyl group include a methylsulfonyl group, an ethylsulfonyl group, and a dodecylsulfonyl group.

The symbol "*" that can be attached to a chemical formula represents a bond.

The "ink" means a liquid for use in a coating method, which is not limited to a colored liquid. In addition, the "coating method" encompasses a method of forming a film (layer) with the use of a liquid substance, and examples thereof include a slot die coating method, a slit coating method, a knife coating method, a spin coating method, a casting method, a micro-gravure coating method, a gravure coating method, a bar coating method, a roll coating method, a wire bar coating method, a dip coating method, a spray coating method, a screen printing method, a gravure printing method, a flexographic printing method, an offset printing method, an inkjet coating method, a dispenser printing method, a nozzle coating method, and a capillary coating method.

The ink may be a solution, or may be a dispersion such as a dispersion, an emulsion (emulsion), or a suspension (suspension).

The "absorption peak wavelength" is a parameter specified based on an absorption peak of an absorption spectrum measured in a predetermined wavelength range, which refers to the wavelength of an absorption peak with the highest absorbance among the absorption peaks of the absorption spectrum.

The "external quantum efficiency" is also referred to as EQE (External Quantum Efficiency), which refers to a value that indicates the ratio (%) of the number of electrons that can be extracted to the outside of a photoelectric conversion element among electrons generated with respect to the number of photons emitted to the photoelectric conversion element.

### 1. Compound

First, the compound according to the present embodiment will be described. The compound according to the present embodiment can be suitably used as a semiconductor material particularly for an active layer of a photoelectric conversion element. It is to be noted that whether the compound according to the present embodiment functions as either a p-type semiconductor material or an n-type semiconductor material in the active layer can be relatively determined from the value of the HOMO energy level of the selected compound or the value of the LUMO energy level thereof. The compound according to the present embodiment can be suitably used particularly as an n-type semiconductor material in an active layer of a photoelectric conversion element.

The relationship between the values of the HOMO and LUMO energy levels of the p-type semiconductor material contained in the active layer and the values of the HOMO and LUMO energy levels of the n-type semiconductor material can be appropriately set to the extent that the photoelectric conversion element (photodetection element) operates.

The compound according to the present embodiment is a compound represented by the following formula (I).

A¹-B¹-A¹ (I)

In the formula (I),
A¹ represents an electron-withdrawing group,
B¹ is a divalent group including two or more constituent units that are linked by a single bond to constitute a π-conjugated system, at least one of the two or more constituent units is a first constituent unit (hereinafter, also referred to as a first constituent unit CU1) represented by the following formula (II), and the remaining second constituent unit (hereinafter, also referred to as a second constituent unit CU2) other than the first constituent unit is a divalent group including an unsaturated bond, an arylene group, or a heteroarylene group.

The two groups A¹ may be identical to or different from each other. In the case of having two or more first constituent units, the two or more first constituent units may be identical to or different from each other. In the case of having two or more second constituent units, the two or more second constituent units may be identical to or different from each other.

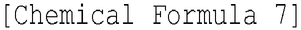

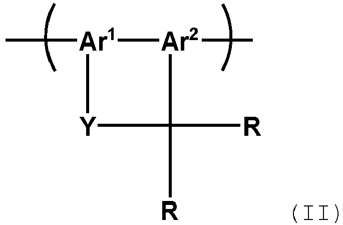

In the formula (II),
Ar¹ and Ar² each independently represent an aromatic carbocyclic ring optionally having a substituent or an aromatic heterocyclic ring optionally having a substituent,
Y represents a group represented by -C(=O)- or an oxygen atom,
the groups R each independently represent
a hydrogen atom,
a halogen atom,
an alkyl group optionally having a substituent,
a cycloalkyl group optionally having a substituent,
an aryl group optionally having a substituent,
an alkyloxy group optionally having a substituent,
a cycloalkyloxy group optionally having a substituent,
an aryloxy group optionally having a substituent,
an alkylthio group optionally having a substituent,
a cycloalkylthio group optionally having a substituent,
an arylthio group optionally having a substituent,
a monovalent heterocyclic group optionally having a substituent,
a substituted amino group optionally having a substituent,
an acyl group optionally having a substituent,
an imine residue optionally having a substituent,
an amide group optionally having a substituent,
an acid imide group optionally having a substituent,
a substituted oxycarbonyl group optionally having a substituent,
an alkenyl group optionally having a substituent,
a cycloalkenyl group optionally having a substituent,
an alkynyl group optionally having a substituent,
a cycloalkynyl group optionally having a substituent,
a cyano group,
a nitro group,
a group represented by -C(=O)-R^{a}, or
a group represented by -SO₂-R^{b},
R^{a} and R^{b} each independently represent
a hydrogen atom,
an alkyl group optionally having a substituent,
an aryl group optionally having a substituent,
an alkyloxy group optionally having a substituent,
an aryloxy group optionally having a substituent, or
a monovalent heterocyclic group optionally having a substituent.
the multiple groups R may be identical to or different from each other.

The compound according to the present embodiment is a non-fullerene compound represented by the formula (I) mentioned above, in which two groups A¹ that are electron-withdrawing monovalent group are bonded to both terminals of B¹ that is a divalent group including two or more constituent units that are linked by a single bond to constitute a π-conjugated system. A¹ and B¹ that can constitute the compound according to the present embodiment will be specifically described below.

### (1) Regarding A¹

A¹ is an electron-withdrawing monovalent group.

Examples of A¹ that is an electron-withdrawing monovalent group include a group represented by -CH=C(-CN)₂ and groups represented by the following formulas (a-1) to (a-9).

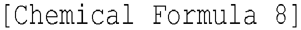

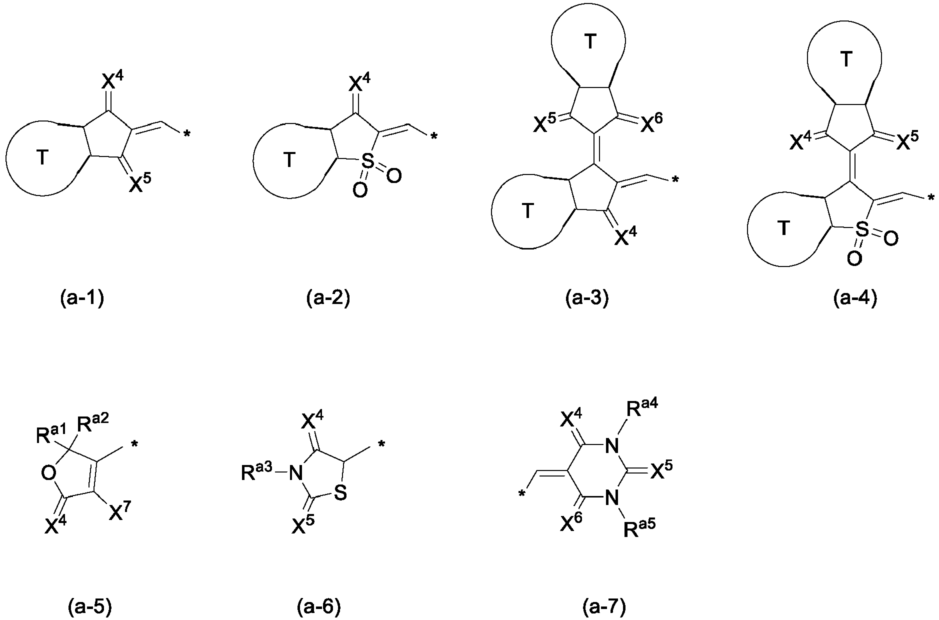

In the formulas (a-1) to (a-7),
T represents a carbocyclic ring optionally having a substituent or a heterocyclic ring optionally having a substituent. The carbocyclic ring and the heterocyclic ring may be a monocyclic ring or a condensed ring. When these rings have multiple substituents, the multiple substituents may be identical to or different from each other.

Examples of the carbocyclic ring optionally having a substituent, represented by T include aromatic carbocyclic rings, and preferred are aromatic carbocyclic rings. Specific examples of the carbocyclic ring optionally having a substituent, represented by T include a benzene ring, a naphthalene ring, an anthracene ring, a tetracene ring, a pentacene ring, a pyrene ring, and a phenanthrene ring, and preferred are a benzene ring, a naphthalene ring, and a phenanthrene ring, more preferred are a benzene ring and a naphthalene ring, and still more preferred is a benzene ring. These rings may have a substituent.

Examples of the heterocyclic ring optionally having a substituent, represented by T include aromatic heterocyclic rings, and preferred are aromatic carbocyclic rings. Specific examples of the heterocyclic ring optionally having a substituent, represented by T include a pyridine ring, a pyridazine ring, a pyrimidine ring, a pyrazine ring, a pyrrole ring, a furan ring, a thiophene ring, an imidazole ring, an oxazole ring, a thiazole ring, and a thienothiophene ring, and preferred are a thiophene ring, a pyridine ring, a pyrazine ring, a thiazole ring, and a thienothiophene ring, and more preferred us a thiophene ring. These rings may have a substituent.

Examples of the substituent that can be included in the carbocyclic ring or heterocyclic ring represented by T include a halogen atom, an alkyl group, an alkyloxy group, an aryl group, and a monovalent heterocyclic group, and preferred are a fluorine atom, a chlorine atom, an alkyloxy group having 1 to 6 carbon atoms, and/or an alkyl group having 1 to 6 carbon atoms.

X⁴, X⁵, and X⁶ each independently represent an oxygen atom, a sulfur atom, an alkylidene group, or a group represented by =C(-CN)₂, and preferably represent an oxygen atom, a sulfur atom, or a group represented by =C(-CN)₂.

X⁷ represents a hydrogen atom or a halogen atom, a cyano group, an alkyl group optionally having a substituent, an alkyloxy group optionally having a substituent, an aryl group optionally having a substituent, or a monovalent heterocyclic group. X⁷ is preferably a cyano group.

R^{a1}, R^{a2}, R^{a3}, R^{a4}, and R^{a5} are each independently a hydrogen atom, an alkyl group optionally having a substituent, a halogen atom, an alkyloxy group optionally having a substituent, an aryl group optionally having a substituent, or a monovalent heterocyclic group, and preferably an alkyl group optionally having a substituent or an aryl group optionally having a substituent.

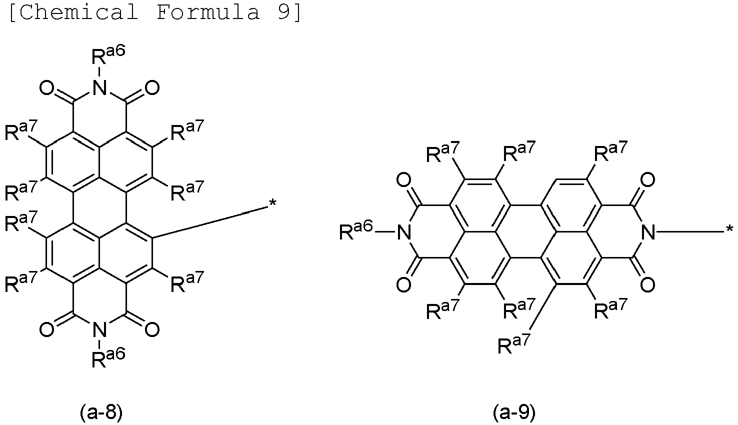

In the formula (a-8) and the formula (a-9),
R^{a6} and R^{a7} each independently represent a hydrogen atom, a halogen atom, an alkyl group optionally having a substituent, a cycloalkyl group optionally having a substituent, an alkyloxy group optionally having a substituent, a cycloalkyloxy group optionally having a substituent, a monovalent aromatic carbocyclic group optionally having a substituent, or a monovalent aromatic heterocyclic group optionally having a substituent, and multiple groups R^{a6} and R^{a7} may be identical to or different from each other.

Specific examples of the electron-withdrawing group represented by A¹ include groups represented by the following formulas (a-1-1) to (a-1-4), and formulas (a-5-1), (a-6-1), and (a-7-1).

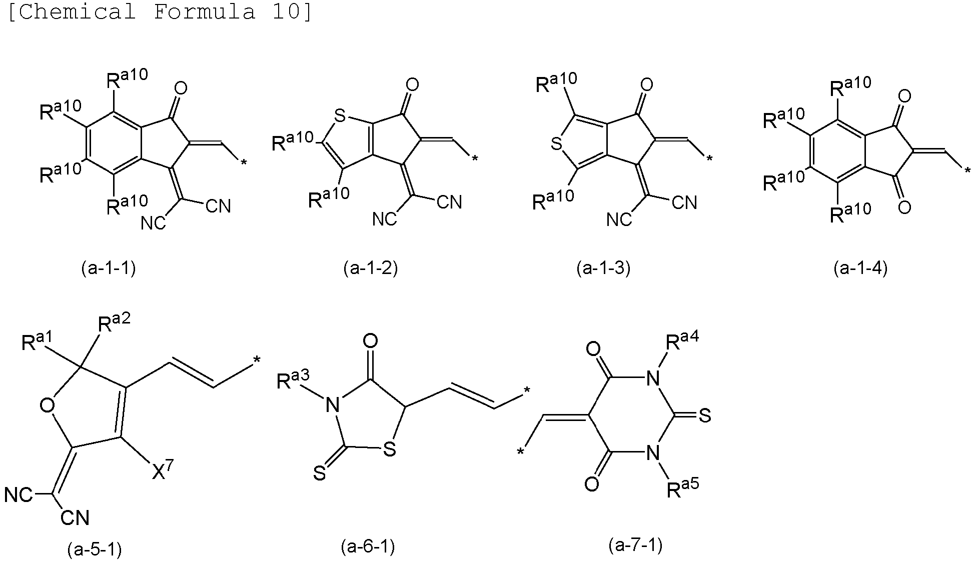

In the formulas (a-1-1) to (a-1 -4), and the formulas (a-5-1), (a-6-1), and (a-7-1),
multiple groups R^{a10} each independently represent a hydrogen atom or a substituent,
R^{a1}, R^{a2}, R^{a3}, R^{a4}, and R^{a5} each independently have the same meaning as mentioned above.

R^{a10} is preferably a hydrogen atom, a halogen atom, an alkyloxy group, a cyano group, or an alkyl group. R^{a1}, R^{a2}, R^{a3}, R^{a4}, and R^{a5} are preferably an alkyl group optionally having a substituent or an aryl group optionally having a substituent.

Preferred examples of the electron-withdrawing group represented by A¹ include groups represented by the following formulas.

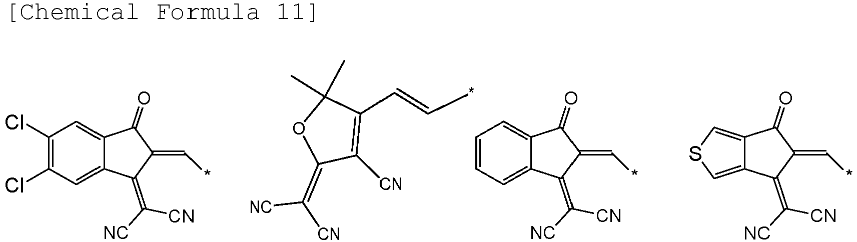

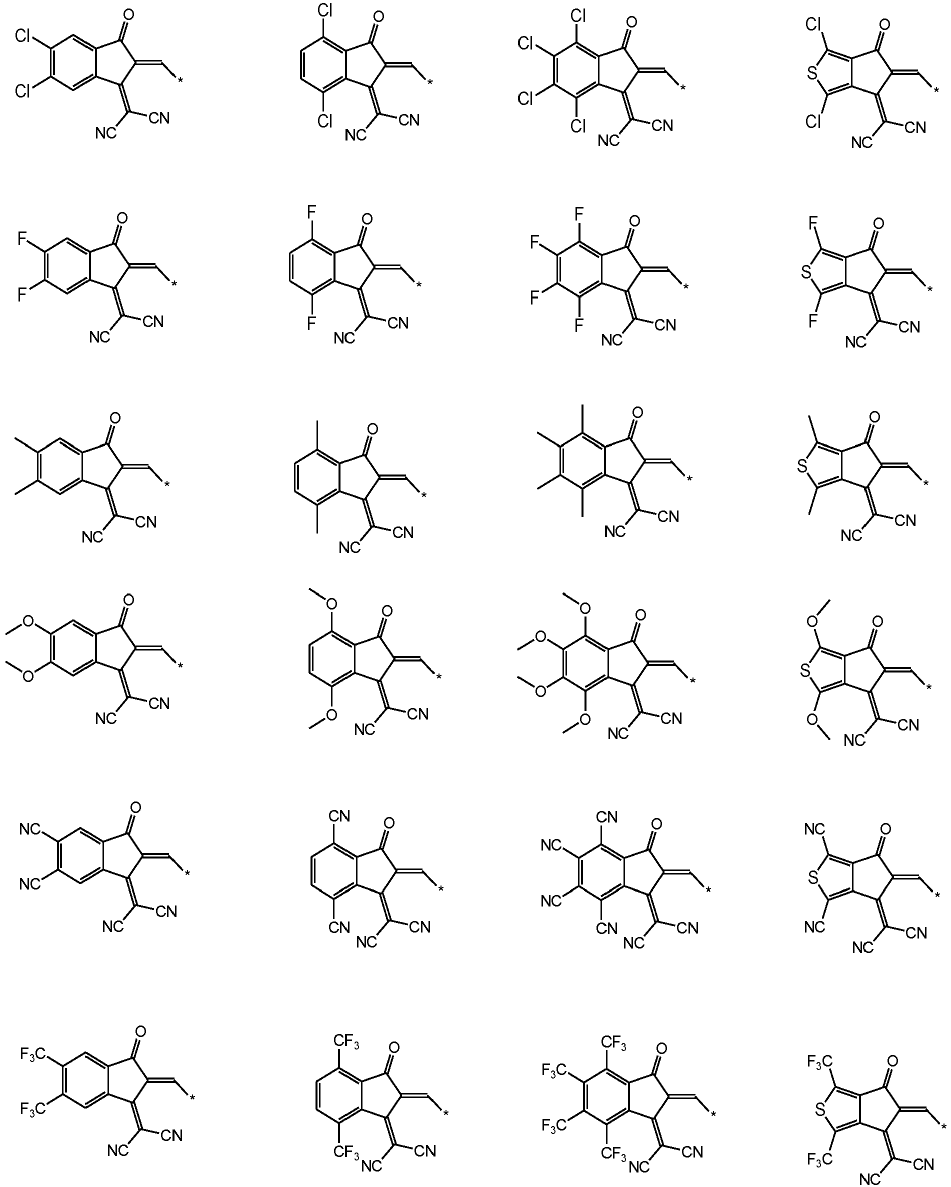

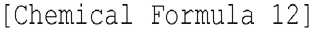

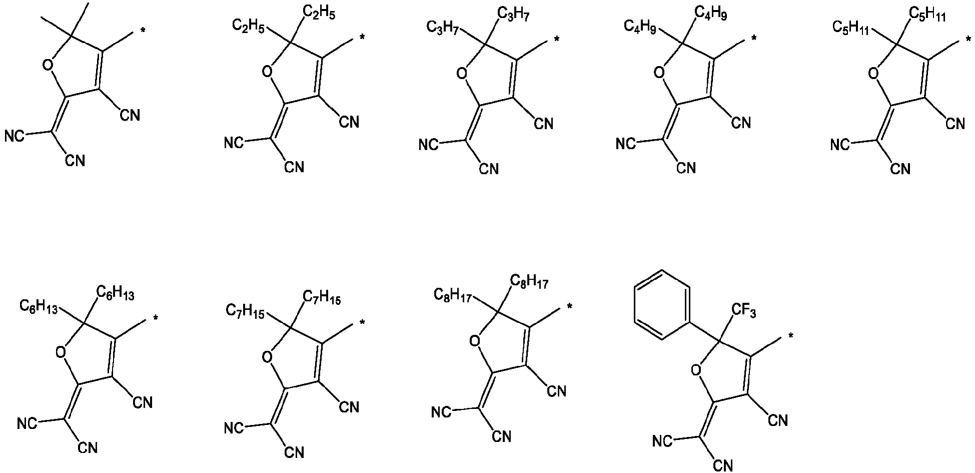

### (2) Regarding B¹

B¹ is a divalent group including two or more constituent units that are linked by a single bond to constitute a π-conjugated system. B¹ is a divalent group including one or more pairs of atoms π-bonded to each other, with a π-electron cloud spread throughout B¹.

At least one of the two or more constituent units that can constitute B¹ is a first constituent unit CU1 represented by the formula (II) mentioned above, and the remaining second constituent unit CU2 other than the first constituent unit CU1 is a divalent group including an unsaturated bond, an arylene group, or a heteroarylene group.

In the compound according to the present embodiment, B¹ preferably includes two or more first constituent units CU1.

The first constituent unit CU1 and the second constituent unit CU2 will be specifically described below.

### (i) First Constituent Unit CU1

The first constituent unit CU1 that can constitute B¹ is a constituent unit represented by the following formula (II) .

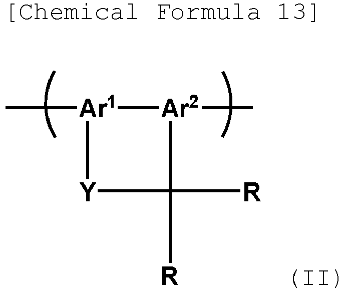

In the formula (II), Ar¹ and Ar² and Y and R are as defined above.

Aromatic carbocyclic rings that can constitute Ar¹ and Ar² are preferably a benzene ring and a naphthalene ring, more preferably a benzene ring and a naphthalene ring, still more preferably benzene rings. These rings may have a substituent.

Aromatic heterocyclic rings that can constitute Ar¹ and Ar² are preferably an oxadiazole ring, a thiadiazole ring, a thiazole ring, an oxazole ring, a thiophene ring, a thienothiophene ring, a benzothiophene ring, a pyrrole ring, a phosphole ring, a furan ring, a pyridine ring, a pyrazine ring, a pyrimidine ring, a triazine ring, a pyridazine ring, a quinoline ring, an isoquinoline ring, a carbazole ring, and a dibenzophosphole ring; and a phenoxazine ring, a phenothiazine ring, a dibenzoborol ring, a dibenzosilole ring, and a benzopyran ring. These rings may have a substituent.

The halogen atom represented by R is preferably a fluorine atom.

The alkyl group optionally having a substituent represented by R is preferably an alkyl group optionally having a substituent and having 1 to 20 carbon atoms, more preferably an alkyl group optionally having a substituent and having 1 to 15 carbon atoms, still more preferably an alkyl group optionally having a substituent and having 1 to 12 carbon atoms, yet still more preferably an alkyl group optionally having a substituent and having 1 to 10 carbon atoms.

The substituent optionally included in the alkyl group, represented by R is preferably a halogen atom, more preferably a fluorine atom and/or a chlorine atom.

The cycloalkyl group optionally having a substituent represented by R is preferably a cycloalkyl group optionally having a substituent and having 3 to 10 carbon atoms, more preferably a cycloalkyl group optionally having a substituent and having 5 to 6 carbon atoms, still more preferably a cyclohexyl group optionally having a substituent.

The aryl group optionally having a substituent represented by R is preferably an aryl group optionally having a substituent and having 6 to 15 carbon atoms, more preferably a phenyl group or a naphthyl group optionally having a substituent.

The substituent optionally included in the aryl group, represented by R is preferably a halogen atom (e.g., a chlorine atom, a fluorine atom), an alkyl group having 1 to 12 carbon atoms (e.g., a methyl group, a trifluoromethyl group, a tert-butyl group, an octyl group, a dodecyl group), an alkyloxy group having 1 to 12 carbon atoms (e.g., a methoxy group, an ethoxy group, an octyloxy group), an alkylsulfonyl group having 1 to 12 carbon atoms (e.g., a dodecylsulfonyl groups), and/or a cyano group.

The alkyloxy group optionally having a substituent represented by R is preferably an alkyloxy group optionally having a substituent and having 1 to 10 carbon atoms, more preferably an alkyloxy group optionally having a substituent and having 1 to 8 carbon atoms, still more preferably a methoxy group, an ethoxy group, a propyloxy group, 3-methylbutyloxy, or a 2-ethylhexyloxy group, which optionally has a substituent.

The aryloxy group optionally having a substituent represented by R is preferably an aryloxy group optionally having a substituent and having 6 to 15 carbon atoms, more preferably a phenyloxy group or an anthracenyloxy group optionally having a substituent.

The substituent optionally included in the aryloxy group, represented by R is preferably an alkyl group having 1 to 12 carbon atoms, more preferably an alkyl group having 1 to 6 carbon atoms, still more preferably a methyl group.

The alkylthio group optionally having a substituent represented by R is preferably an alkylthio group optionally having a substituent and having 1 to 6 carbon atoms, more preferably an alkylthio group optionally having a substituent and having 1 to 3 carbon atoms, still more preferably a methylthio group or a propylthio group optionally having a substituent.

The arylthio group optionally having a substituent represented by R is preferably an arylthio group optionally having a substituent and having 6 to 10 carbon atoms, more preferably a phenylthio group optionally having a substituent.

The substituent optionally included in the arylthio group, represented by R is preferably an alkyl group having 1 to 12 carbon atoms, more preferably an alkyl group having 1 to 6 carbon atoms, still more preferably a methyl group.

The monovalent heterocyclic group optionally having a substituent represented by R is preferably a five- or six-membered monovalent heterocyclic group optionally having a substituent. Examples of the five-membered monovalent heterocyclic group include a thienyl group, a furyl group, a pyrrolyl group, an imidazolyl group, a pyrazolyl group, a thiazolyl group, an isothiazolyl group, an oxazolyl group, an isoxazolyl group, and a pyrrolidinyl group. Examples of the six-membered monovalent heterocyclic group include a pyridyl group, a pyrazinyl group, a pyrimidinyl group, a pyridazinyl group, a piperidyl group, a piperazinyl group, a morpholinyl group, and a tetrahydropyranyl group.

The monovalent heterocyclic group optionally having a substituent represented by R is more preferably a thienyl group, a furyl group, a thiazolyl group, an oxazolyl group, a pyridyl group, or a pyrazyl group, which optionally has a substituent.

The substituent optionally included in the monovalent heterocyclic group, represented by R is preferably an alkyl group having 1 to 12 carbon atoms (e.g., a methyl group, a trifluoromethyl group, a propyl group, a hexyl group, an octyl group, a dodecyl group).

The alkenyl group optionally having a substituent represented by R is preferably an alkenyl group optionally having a substituent and having 2 to 10 carbon atoms, more preferably an alkenyl group optionally having a substituent and having 2 to 6 carbon atoms, still more preferably a 2-propenyl group or a 5-hexenyl group optionally having a substituent.

The cycloalkenyl group optionally having a substituent represented by R is preferably a cycloalkenyl group optionally having a substituent and having 3 to 10 carbon atoms, more preferably a cycloalkenyl group optionally having a substituent and having 6 to 7 carbon atoms, still more preferably a cyclohexenyl group or a cycloheptenyl group optionally having a substituent.

The substituent optionally included in the cycloalkenyl group, represented by R is preferably an alkyl group having 1 to 12 carbon atoms.

The alkynyl group optionally having a substituent represented by R is preferably an alkynyl group optionally having a substituent and having 2 to 10 carbon atoms, more preferably an alkynyl group optionally having a substituent and having 5 to 6 carbon atoms, still more preferably a 5-hexynyl group or a 3-methyl-1-butynyl group optionally having a substituent.

The cycloalkynyl group optionally having a substituent represented by R is preferably a cycloalkynyl group optionally having a substituent and having 6 to 10 carbon atoms, more preferably a cycloalkynyl group optionally having a substituent and having 7 to 8 carbon atoms, still more preferably a cycloheptynyl group or a cyclooctynyl group optionally having a substituent.

The substituent optionally included in the cycloalkynyl group, represented by R is preferably a C1 to C12 alkyl group.

The multiple groups R are each independently preferably an alkyl group optionally having a substituent, more preferably an alkyl group optionally having a substituent and having 1 to 15 carbon atoms, still more preferably an alkyl group optionally having a substituent and having 1 to 12 carbon atoms, yet still more preferably an alkyl group optionally having a substituent and having 1 to 10 carbon atoms. The multiple groups R are each particularly preferably an alkyl group optionally having a substituent having 1 to 10 carbon atoms.

In the group represented by -C(=O)-R^{a} and the group represented by -SO₂-R^{b}, represented by R, R^{a} is preferably a hydrogen atom, R^{b} is preferably an alkyl group optionally having a substituent or an alkyloxy group optionally having a substituent, more preferably an alkyl group optionally having a substituent and having 1 to 12 carbon atoms or an alkyloxy group optionally having a substituent and having 1 to 12 carbon atoms, still more preferably an alkyl group optionally having a substituent and having 1 to 12 carbon atoms or an alkyloxy group optionally having a substituent and having 1 to 6 carbon atoms, yet still more preferably a methyl group, an ethyl group, a 2-methylpropyl group, an octyl group, a dodecyl group, or an ethoxy group, which optionally has a substituent.

The first constituent unit CU1 represented by the formula (II), which can constitute B¹, is preferably a constituent unit represented by the following formula (III) .

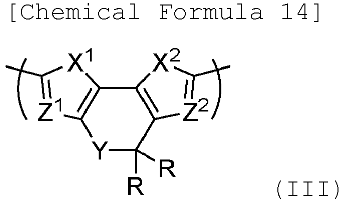

In the formula (III),
Y and R, X¹ and X², and Z¹ and Z² are as defined above.

The first constituent unit CU1 represented by the formula (III), which can constitute B¹, is preferably a constituent unit represented by the following formula (III-1) .

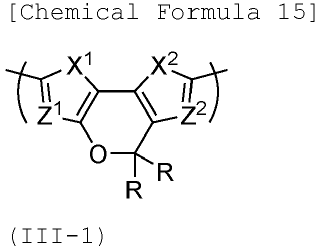

In the formula (III-1), R, X¹ and X², and Z¹ and Z² are as defined above.

Examples of the constituent unit represented by the formula (III-1) include constituent units represented by the following formulas (III-1-1) to (III-1-16).

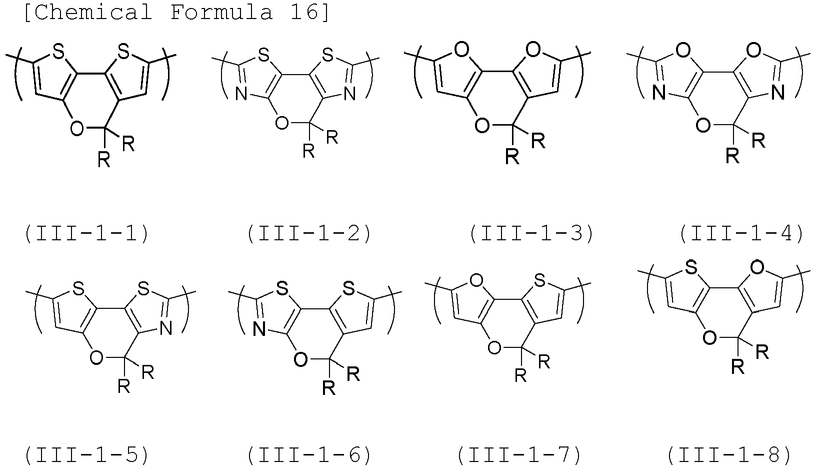

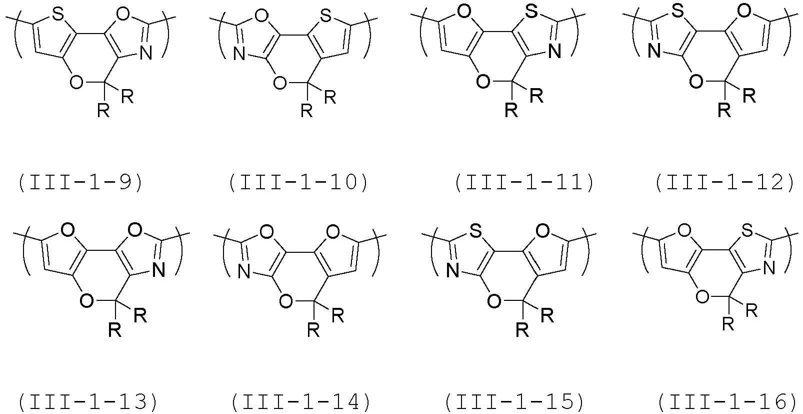

Preferred specific examples of the first constituent unit CU1 represented by the formula (III-1) include constituent units represented by the following formula.

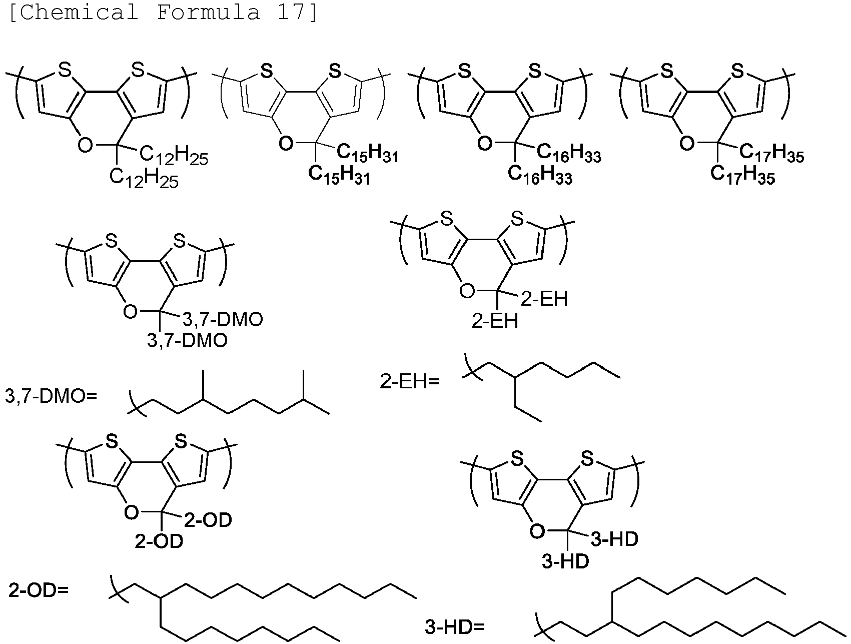

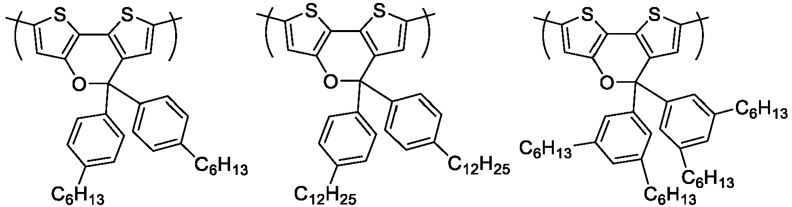

The first constituent unit CU1 represented by formula (III), which can constitute B¹, is preferably a constituent unit represented by the following formula (III-2).

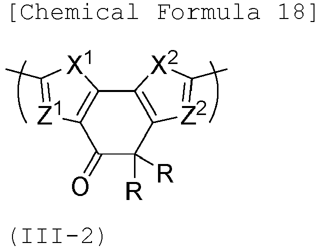

In the formula (111-2), X¹ and X², Z¹ and Z², and R are as defined above.

Examples of the constituent unit represented by the formula (III-2) include constituent units represented by the following formulas (III-2-1) to (III-2-16).

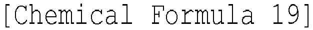

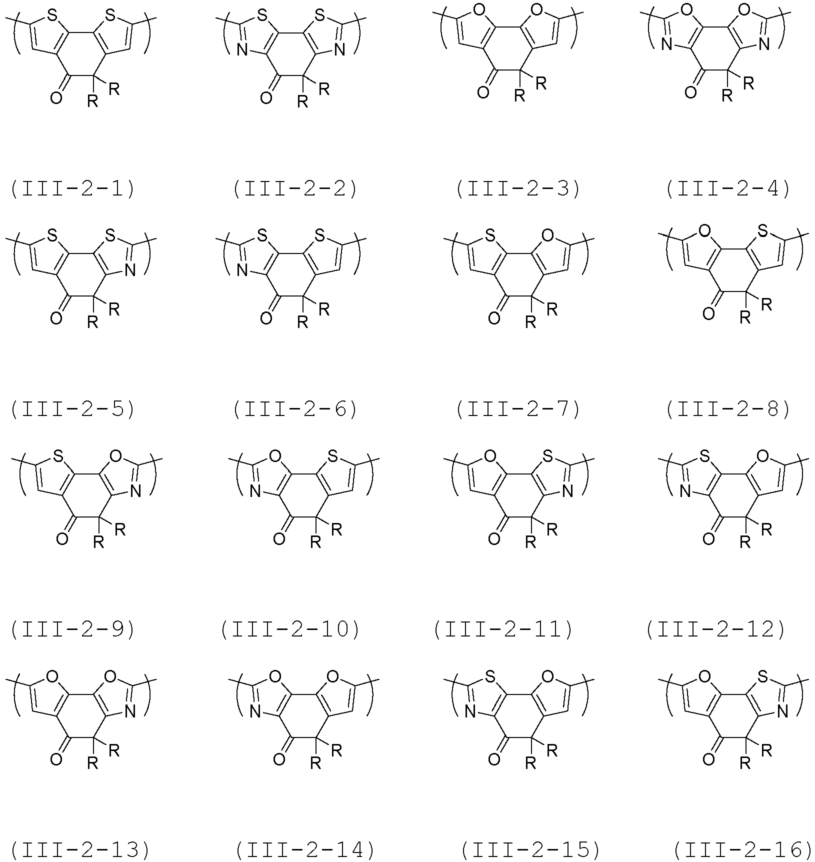

Preferred specific examples of the constituent unit represented by the formula (III-2) include constituent units represented by the following formula.

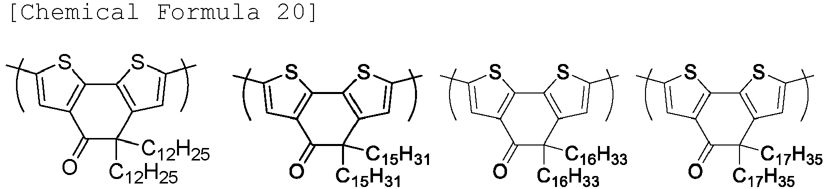

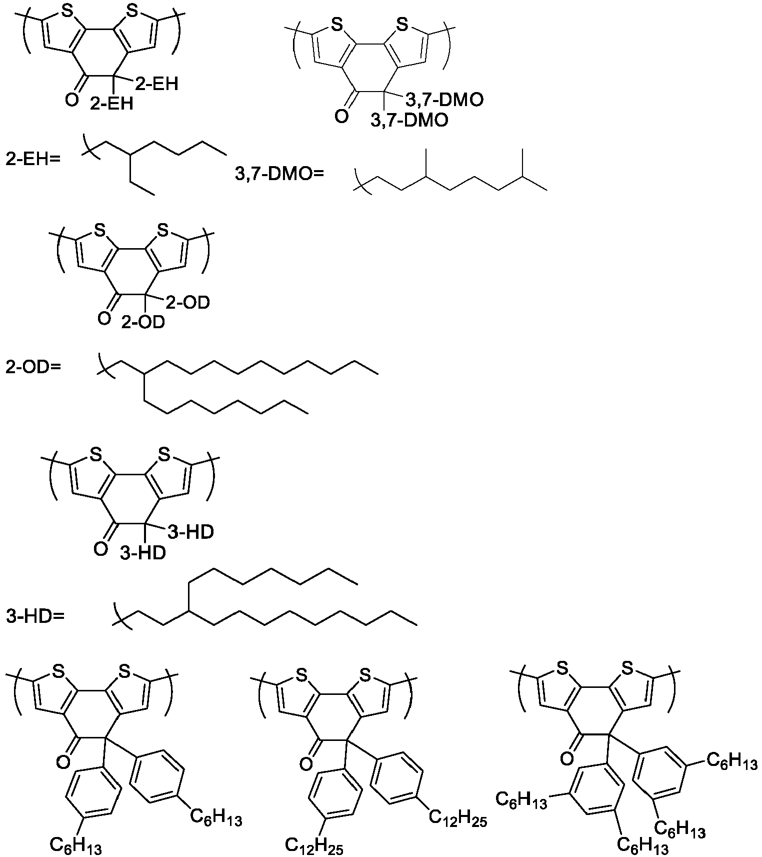

In the compound according to the present embodiment, B¹ preferably includes three or more first constituent units CU1.

In the compound according to the present embodiment, B¹ is preferably a divalent group that has two or more and four or less first constituent units CU1 linked.

### (ii) Second Constituent Unit CU2

The second constituent unit CU2 is a divalent group including an unsaturated bond, an arylene group, or a heteroarylene group.

The "divalent group including an unsaturated bond", which is a second constituent unit CU2, represents, for example, a group represented by -(CR=CR)n- (R is as defined above, and n is an integer of 1 or more. The value of n is preferably 1 or 2, more preferably 1.), or a group represented by -C≡C-.

Specific examples of the "divalent group including an unsaturated bond", which is a second constituent unit CU2, include an ethene-1,2-diyl group, a 1,3-butadiene-1,4-diyl group, and an acetylene-1, 2-diyl group.

In the compound according to the present embodiment, the second constituent unit CU2 is preferably a constituent unit selected from the group consisting of a divalent group including an unsaturated bond and groups represented by the following formulas (IV-1) to (IV-9), and above all, more preferably a constituent unit selected from the group consisting of the groups represented by formulas (IV-1) to (IV-6).

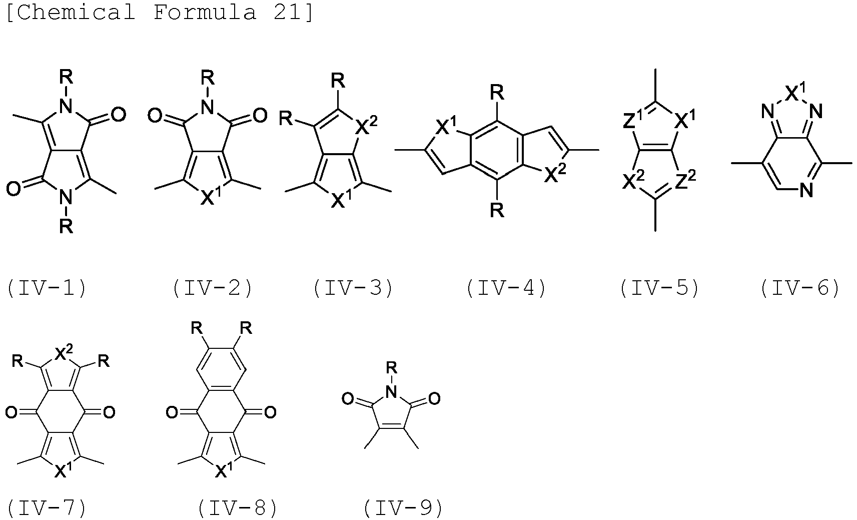

In the formulas (IV-1) to (IV-9), X¹, X², Z¹, Z², and R are as defined above.

In the case of having two groups R, the two groups R may be identical to or different from each other.

More specific preferred examples of the second constituent unit CU2 include constituent units represented by the following formula. These constituent units may further have a substituent.

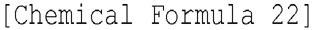

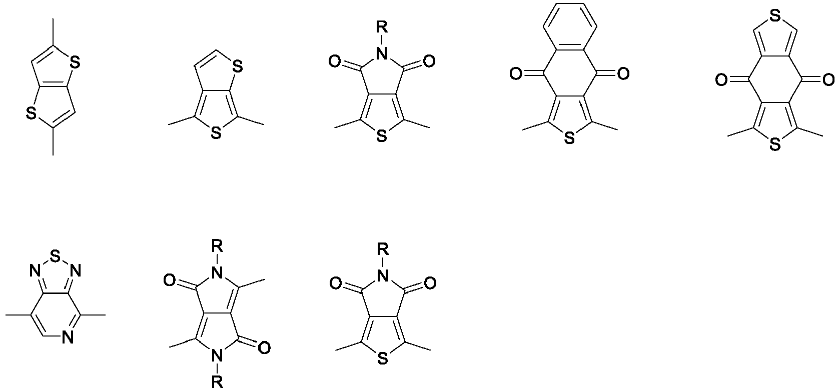

In the compound according to the present embodiment, B¹ includes, as described above, two or more constituent units, at least one of the two or more constituent units is the first constituent unit CU1, and the other constituent unit other than the first constituent unit CU1 is the second constituent units CU2.

The combination of the first constituent unit CU1 and second constituent unit CU2 included in B¹ and the aspect of the sequence thereof are not particularly limited on the condition that B¹ can constitute a π conjugated system.

B¹ is preferably a divalent group that has any one structure selected from the group consisting of structures represented by the following formulas (V-1) to (V-9).

-CU1-CU2-CU1- (V-1)

-CU1-CU2-CU1-CU2-CU1- (V-2)

-CU2-CU1-CU2-CU1-CU2- (V-3)

-CU1-CU2-CU1-CU2-CU1-CU2-CU1- (V-4)

-CU1-CU1- (V-5)

-CU2-CU1-CU2- (V-6)

-CU1-CU1-CU1- (V-7)

-CU2-CU1-CU1-CU2- (V-8)

-CU2-CU1-CU1-CU1-CU2- (V-9)

In the formulas (V-1) to (V-9),
CU1 represents the first constituent unit CU1, and
CU2 represents the second constituent unit CU2.

In the case of having two or more units CU1, the two or more units CU1 may be identical to or different from each other, and in the case of having two or more units CU2, the two or more units CU2 may be identical to or different from each other.

Among the formulas (V-1) to (V-9), divalent groups that have structures represented by the formulas (V-1), (V-3), (V-5), (V-6), (V-7), (V-8), and (V-9) are preferred, and divalent groups that have structures represented by the formulas (V-1), (V-3), (V-7), and (V-9) are more preferred.

The total number of first constituent units CU1 and second constituent units CU2 that can be included in B¹ is typically 2 or more, preferably 3 or more, and typically 7 or less, preferably 5 or less.

The number of first constituent units CU1 that can be included in B¹ is typically 1 or more, preferably 2 or more, more preferably 3 or more.

The number of second constituent units CU2 that can be included in B¹ is typically 5 or less, preferably 3 or less, more preferably 1 or more.

Specific preferred examples of B¹ include divalent groups represented by the following formulas.

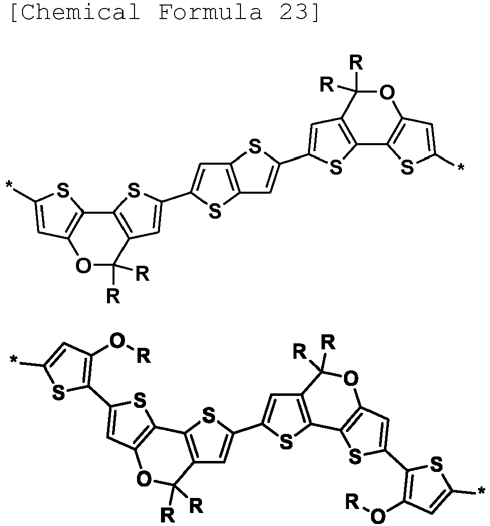

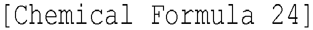

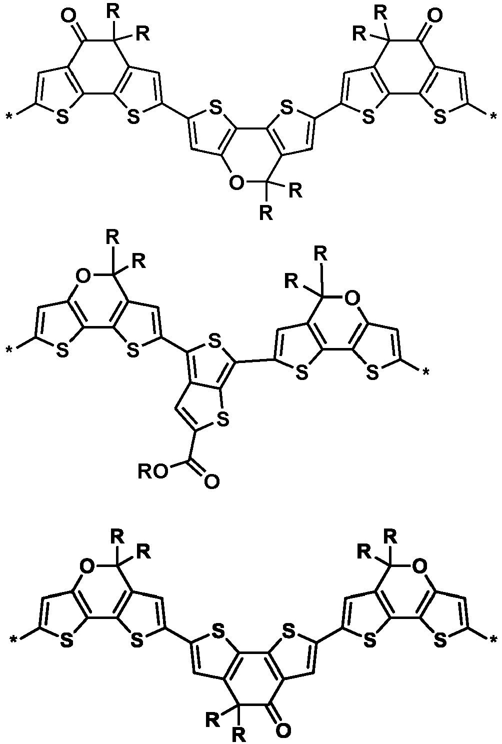

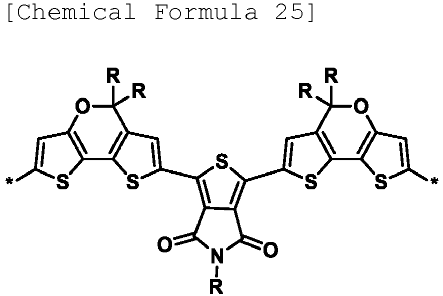

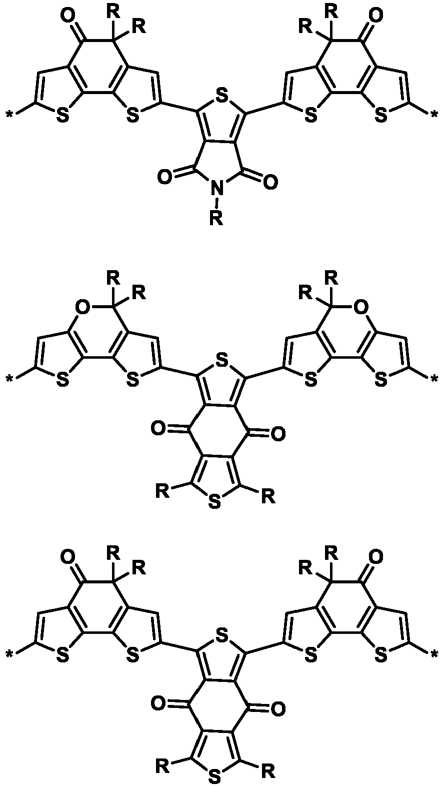

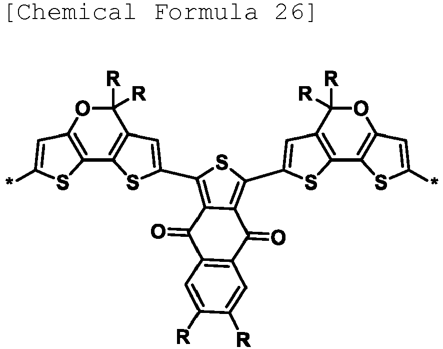

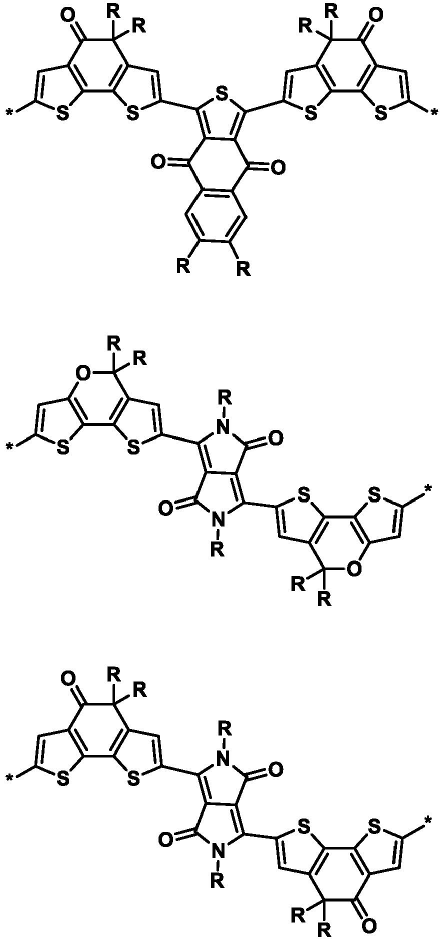

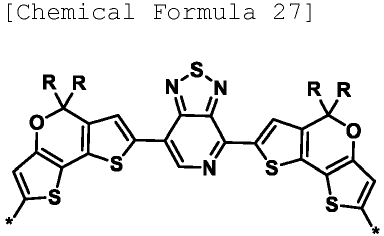

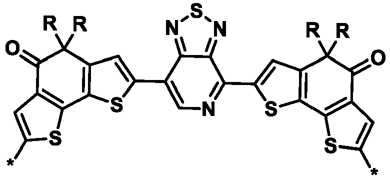

In the formulas, R is as defined above.

Specific examples of the compound represented by the formula (I) according to the present embodiment include compounds represented by the following formulas.

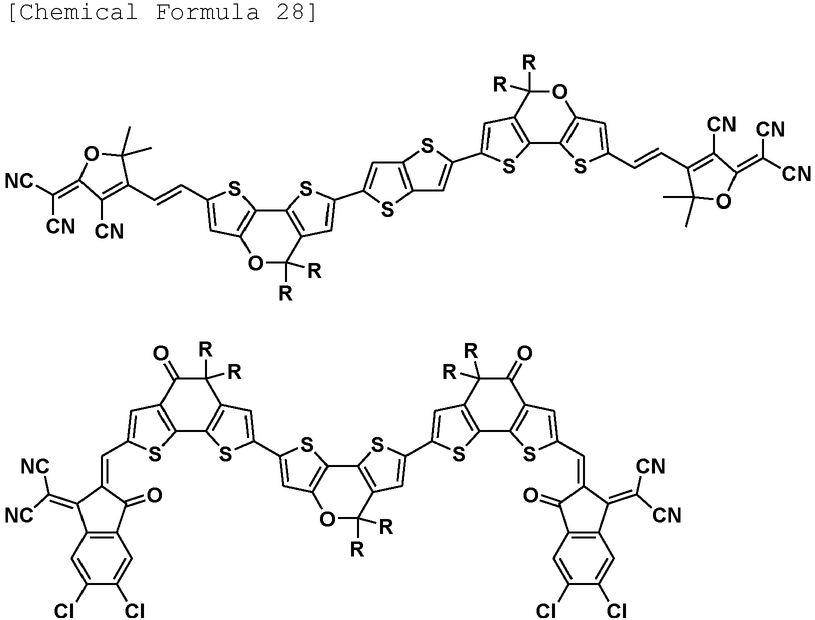

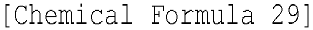

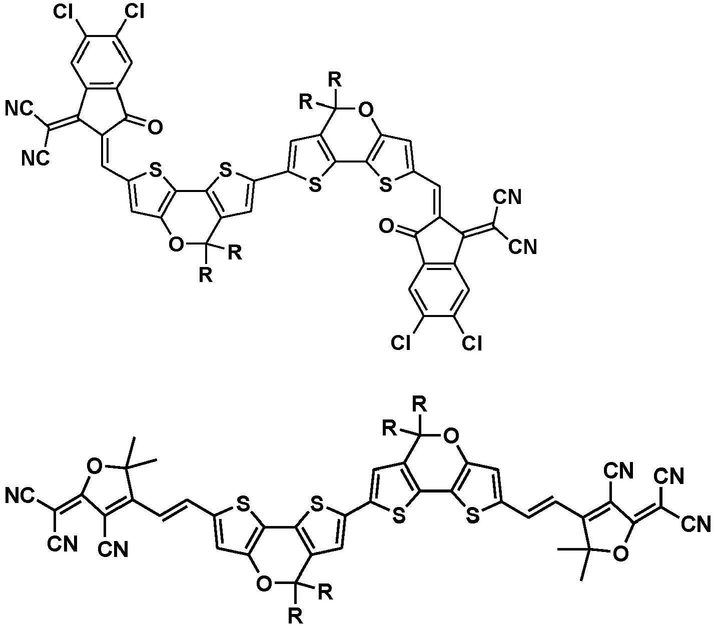

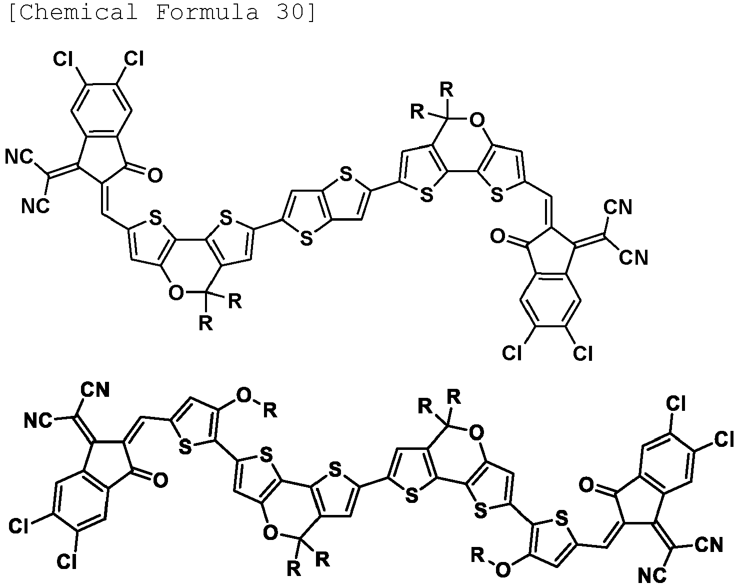

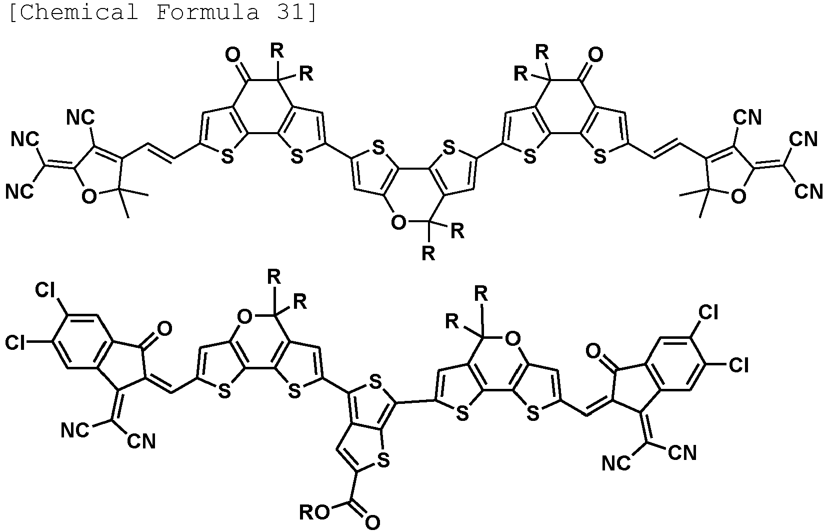

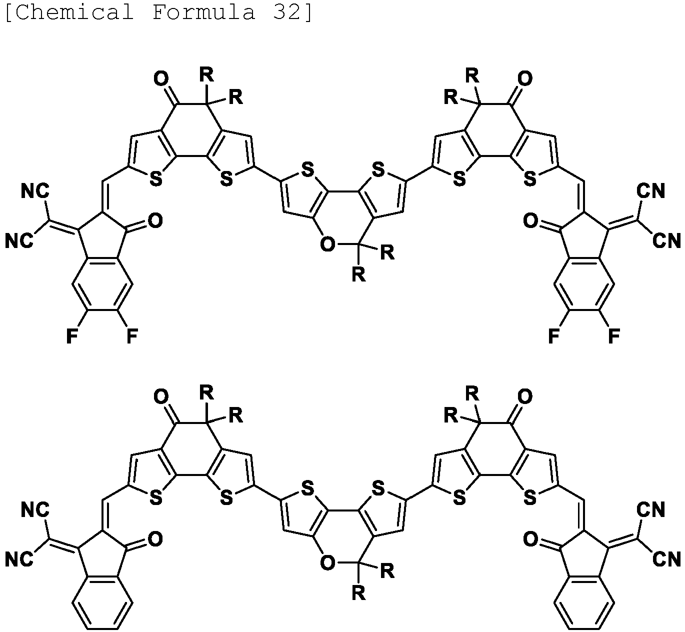

More specific preferred examples of the compound represented by the formula (I) according to the present embodiment include compounds represented by the following formulas N-1 to N-10.

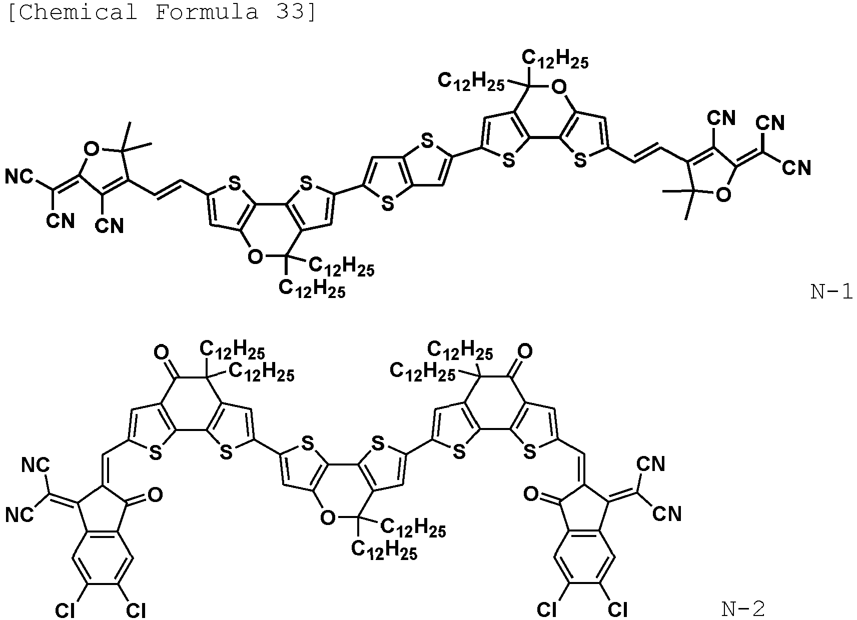

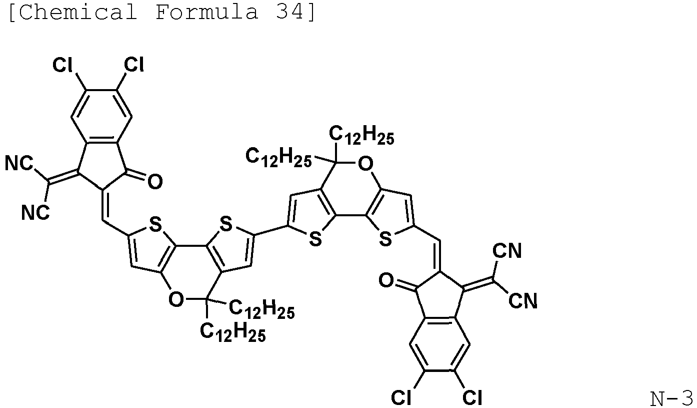

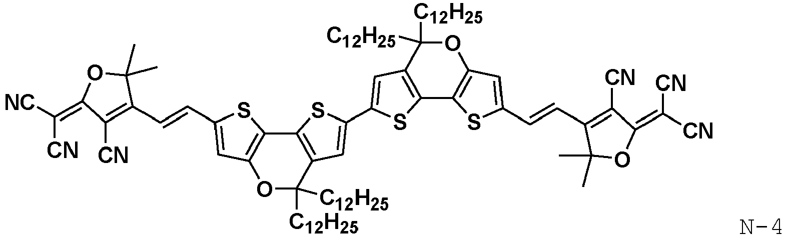

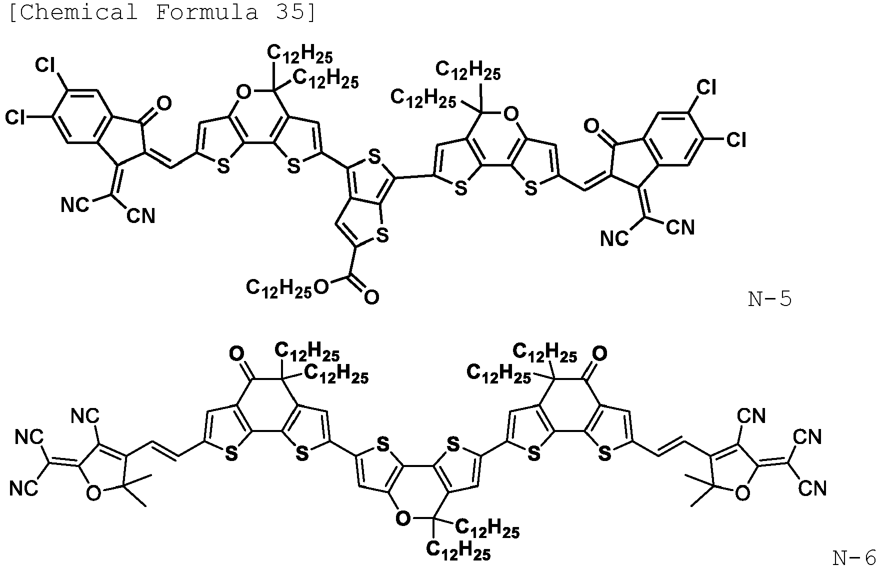

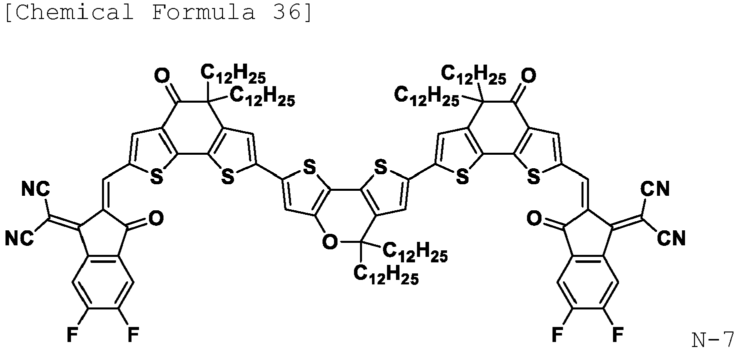

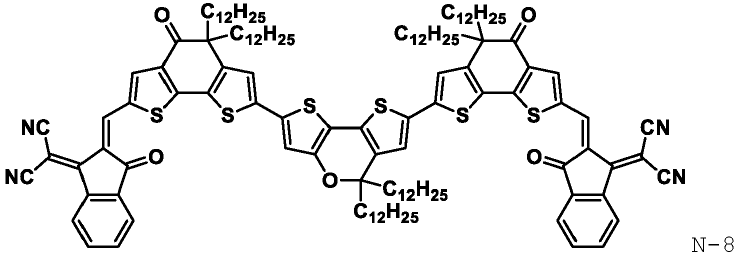

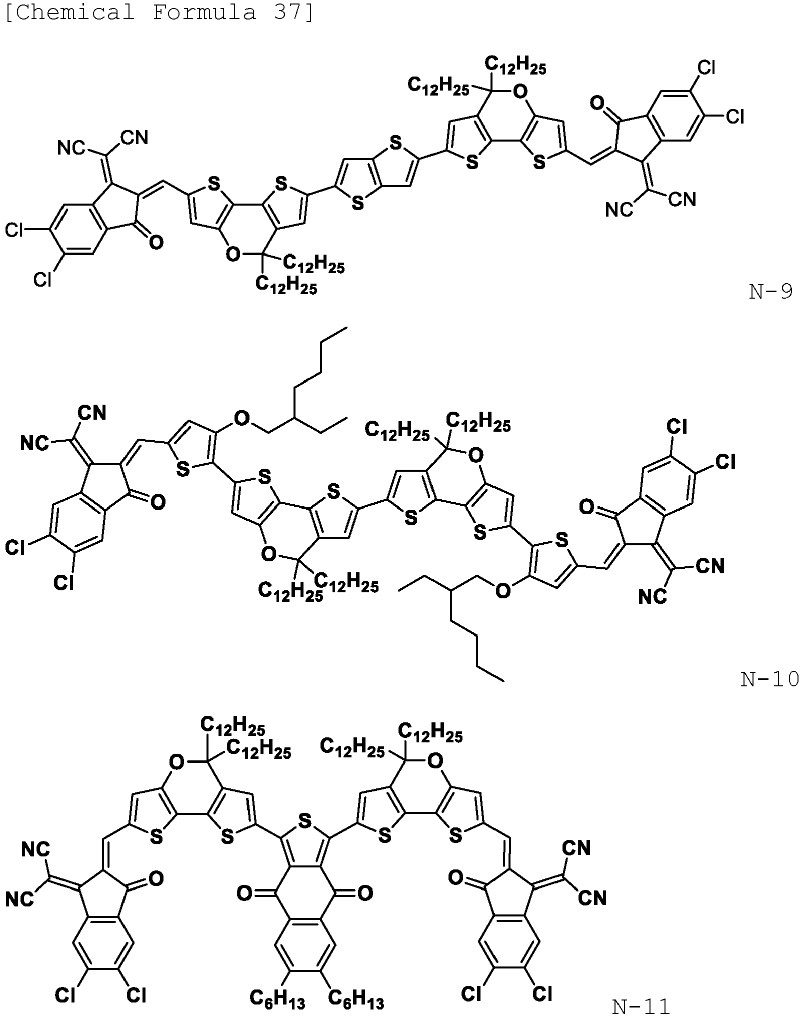

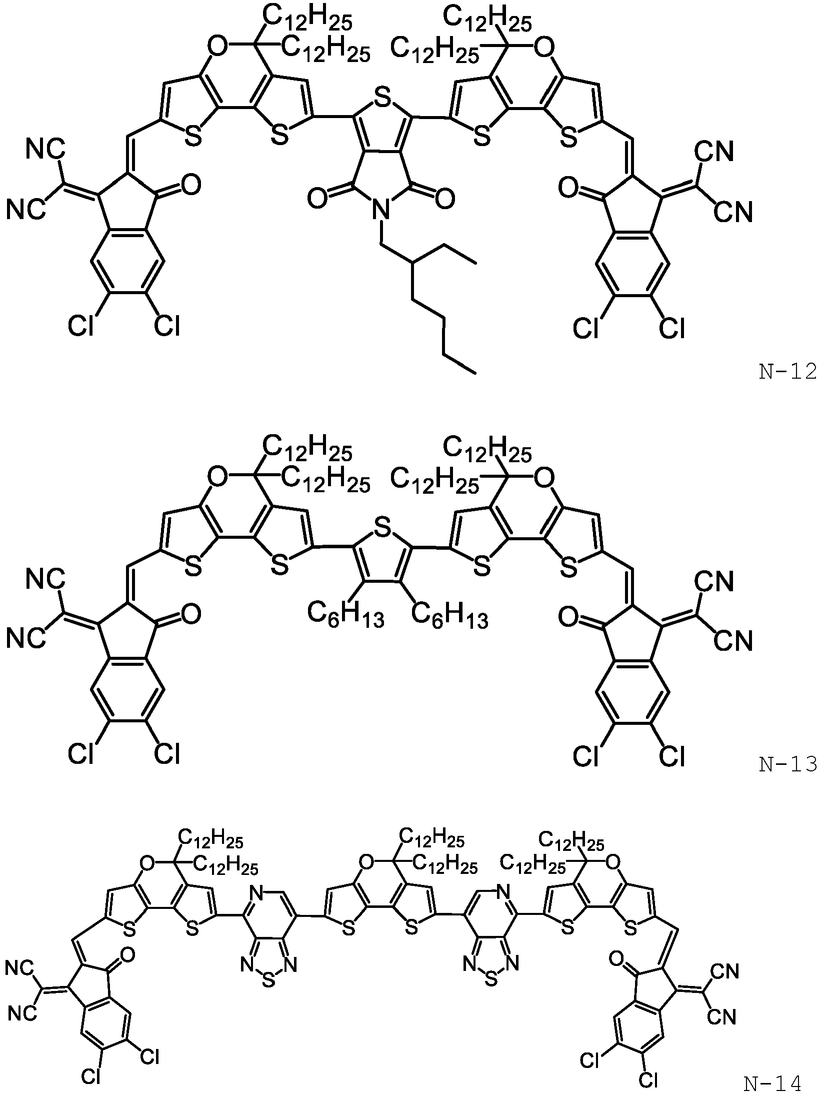

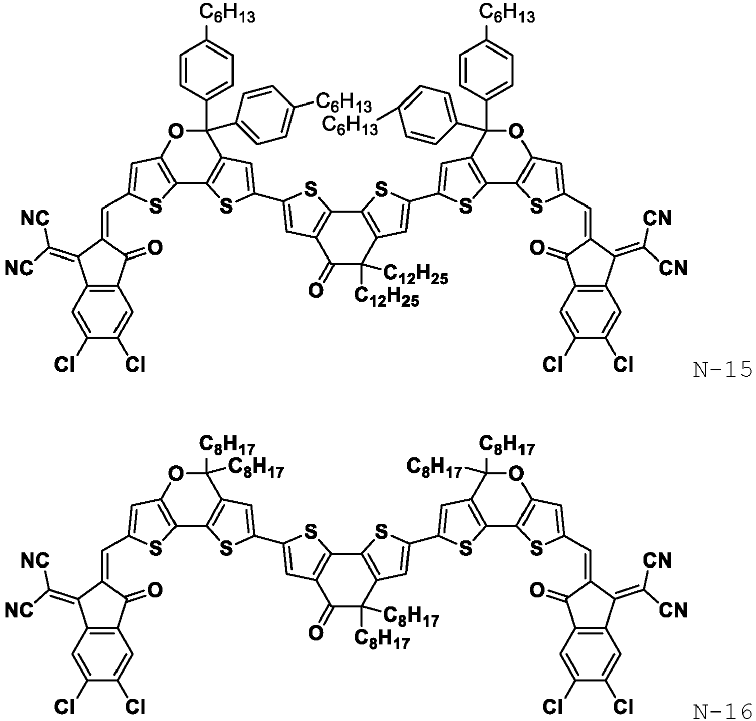

The compound according to the present embodiment can be suitably used as a semiconductor material for an active layer of a photoelectric conversion element, particularly as a non-fullerene compound, which is an n-type semiconductor material.

In particular, the use of the compound according to the present embodiment as an n-type semiconductor material for a material of an active layer can suppress a decrease in EQE with respect to a heating treatment in a step of manufacturing a photoelectric conversion element, a step of incorporating the photoelectric conversion element into a device to which the element is applied, or the like, or further improve the EQE, and improve the heat resistance.

Two or more types of compounds according to the present embodiment for use as an n-type semiconductor material may be contained as materials for an active layer.

The active layer of the photoelectric conversion element (details will be described later) may contain only the compound according to the present embodiment particularly as an n-type semiconductor material, and may contain, as an additional n-type semiconductor material, a compound other than the compound according to the present embodiment as an n-type semiconductor material. The compound other than the compound according to the present embodiment, which can be contained as an additional n-type semiconductor material, may be a low molecular weight compound or a polymer compound.

Examples of the n-type semiconductor material (electron-accepting compound) other than "the compound according to the present embodiment", which is a low molecular weight compound, include oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, metal complexes of 8-hydroxyquinoline and derivatives thereof, and phenanthrene derivatives such as a bathocuproine.

Examples of the n-type semiconductor material other than "the compound according to the present embodiment", which is a polymer compound, include polyvinylcarbazole and derivatives thereof, polysilane and derivatives thereof, polysiloxane derivatives that have an aromatic amine structure in a side chain or a main chain, polyaniline and derivatives thereof, polythiophene and derivatives thereof, polypyrrole and derivatives thereof, polyphenylenevinylene and derivatives thereof, polythienylenevinylene and derivatives thereof, polyquinoline and derivatives thereof, polyquinoxaline and derivatives thereof, and polyfluorene and derivatives thereof.

The compound other than "the compound according to the present embodiment" can encompass fullerene derivatives.

The fullerene derivative herein refers to a compound where at least a part of fullerene (C₆₀ fullerene, C₇₀ fullerene, C₇₆ fullerene, C₇₈ fullerene, and C₈₄ fullerene) is modified. In other words, the fullerene derivative refers to a compound having one or more groups added to the fullerene skeleton. Hereinafter, in particular, a fullerene derivative of C₆₀ fullerene may be referred to as a "C₆₀ fullerene derivative", and a fullerene derivative of C₇₀ fullerene may be referred to as a "C₇₀ fullerene derivative".

The fullerene derivative that can be used as an n-type semiconductor material, other than "the compound according to the present embodiment", is not particularly limited as long as the object of the present invention is not impaired.

Specific examples of the C₆₀ fullerene derivative that can be used as an n-type semiconductor material, other than "the compound according to the present embodiment", include the following compounds:

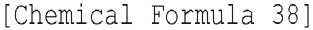

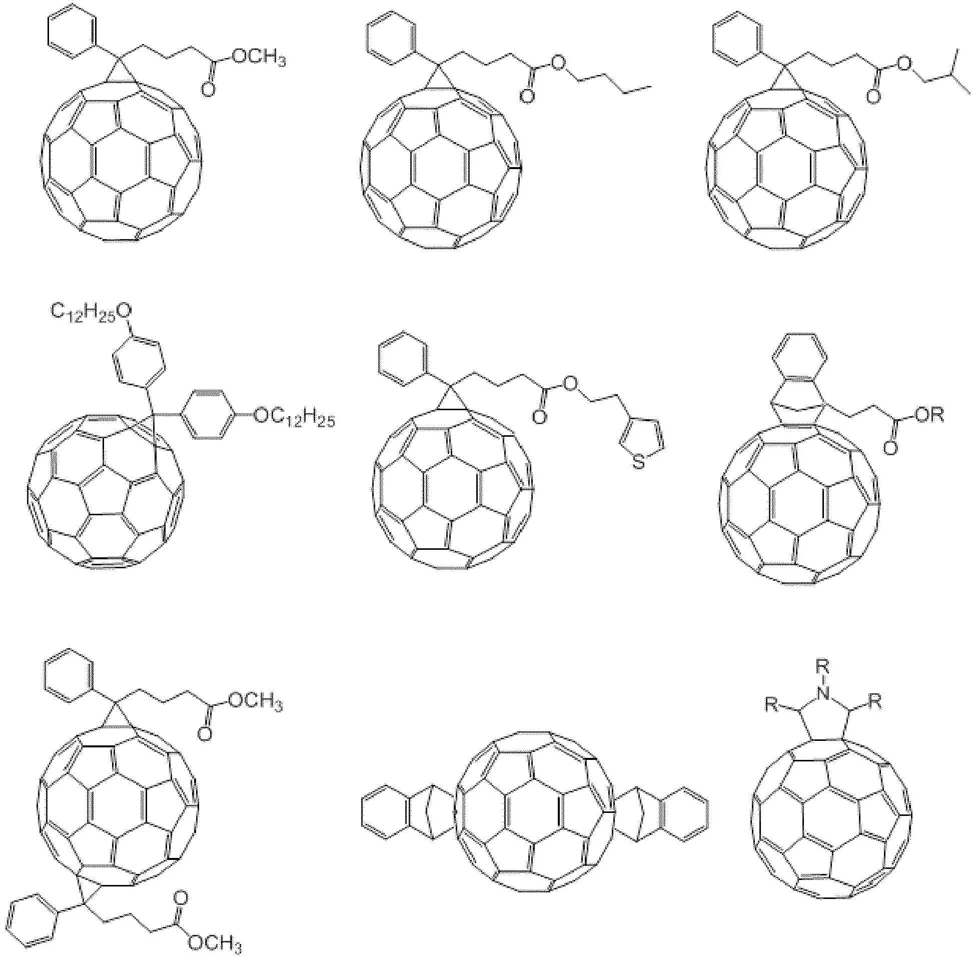

In the formulas, R is as defined above. In the case of having multiple groups R, the multiple groups R may be identical to or different from each other.

Examples of the C₇₀ fullerene derivative include the following compounds:

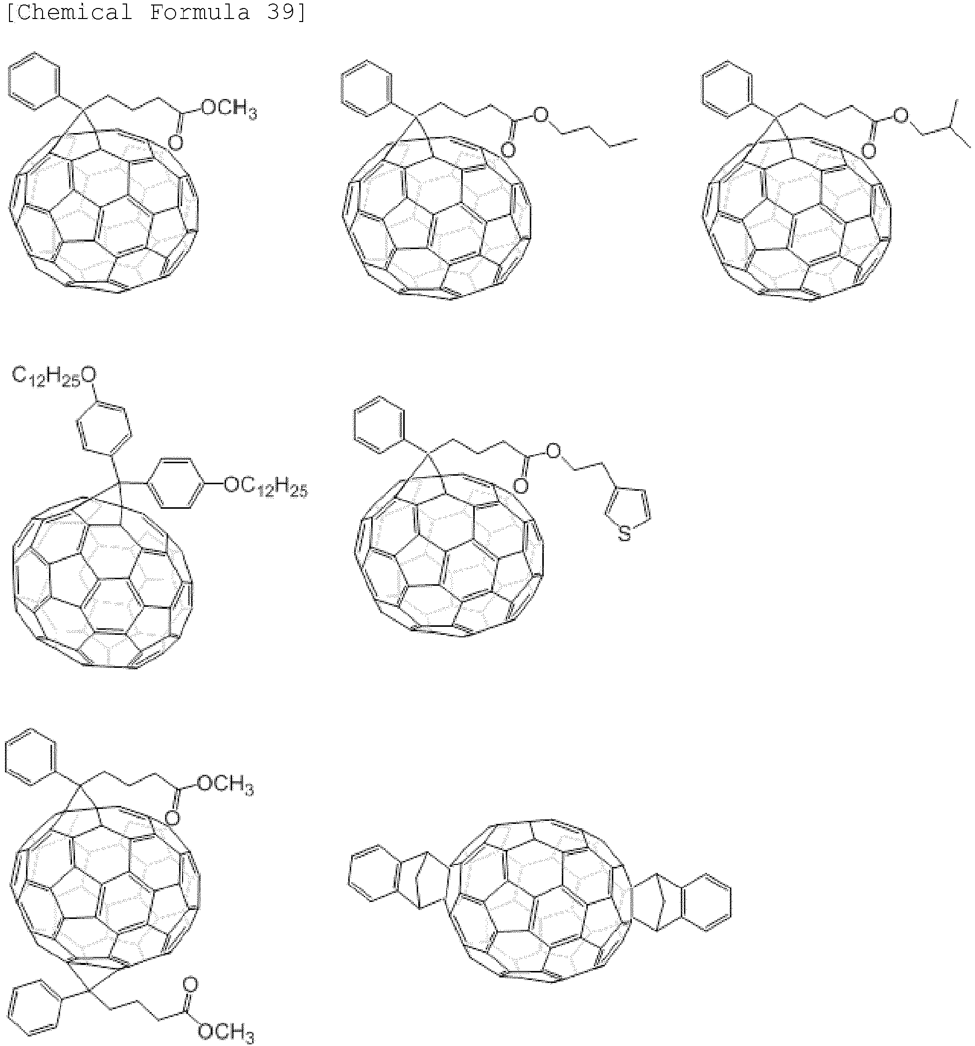

### 2. Photoelectric Conversion Element

A photoelectric conversion element according to the present embodiment is a photoelectric conversion element including an anode, a cathode, and an active layer provided between the anode and the cathode and including a p-type semiconductor material and an n-type semiconductor material, where the n-type semiconductor material contains the compound according to the present embodiment described above.

The photoelectric conversion element according to the present embodiment has the configuration mentioned above, thereby making it possible to suppress a decrease in external quantum efficiency with respect to a heating treatment in a step of manufacturing a photoelectric conversion element, a step of incorporating the photoelectric conversion element into a device to which the element is applied, or the like, and effectively improve the heat resistance.

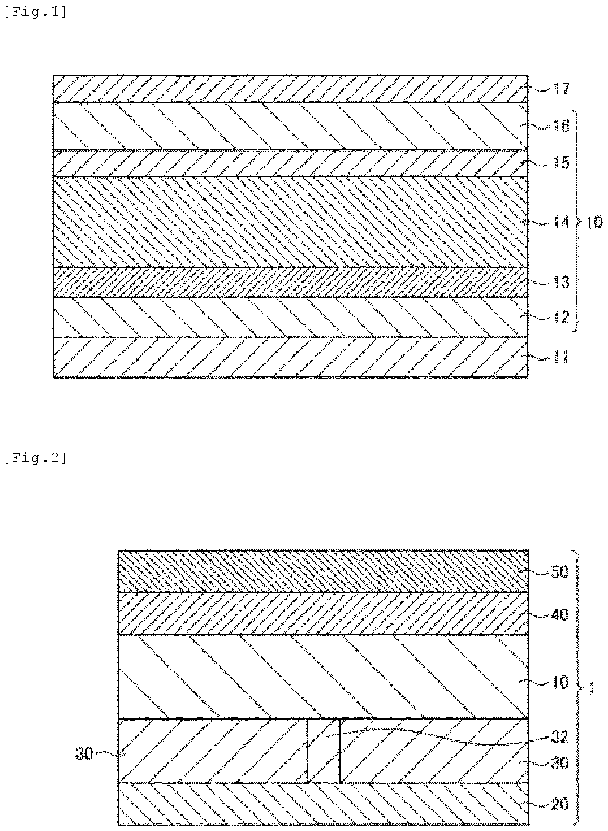

In this regard, a configuration example that can be employed by the photoelectric conversion element according to the present embodiment will be described. Fig. 1 is a diagram schematically illustrating the configuration of a photoelectric conversion element according to the present embodiment.

As illustrated in Fig. 1, the photoelectric conversion element 10 is provided on the support substrate 11. The photoelectric conversion element 10 includes an anode 12 provided in contact with the support substrate 11, a hole transport layer 13 provided in contact with the anode 12, an active layer 14 provided in contact with the hole transport layer 13, an electron transport layer 15 provided in contact with the active layer 14, and a cathode 16 provided in contact with the electron transport layer 15. In this configuration example, a sealing member 17 is further provided in contact with the cathode 16.

Constituent elements that can be included in the photoelectric conversion element according to the present embodiment will be specifically described.

### (Substrate)

The photoelectric conversion element is typically formed on a substrate (support substrate). In addition, the photoelectric conversion element may be further sealed with a substrate (sealing substrate). One of a pair of electrodes: an anode and a cathode is typically formed on the substrate. The material of the substrate is not particularly limited as long as the material undergoes no chemical change particularly in the formation of a layer containing an organic compound.

Examples of the material of the substrate include glass, plastic, a polymer film, and silicon. When an opaque substrate is used, the electrode on the side opposite to the electrode provided on the opaque substrate side (in other words, the electrode on the side far from the opaque substrate) is preferably a transparent or translucent electrode.

### (Electrodes)

The photoelectric conversion element includes an anode and a cathode as a pair of electrodes. At least one electrode of the anode and cathode is preferably a transparent or translucent electrode for allowing light to enter.

Examples of the material of the transparent or translucent electrode include a conductive metal oxide film and a translucent metal thin film. Specific examples thereof include an indium oxide, a zinc oxide, a tin oxide, and composites thereof, or conductive materials such as an indium tin oxide (ITO), an indium zinc oxide (IZO), and NESA, gold, platinum, silver, and copper. As the material of the transparent or translucent electrode, an ITO, an IZO, and a tin oxide are preferred. In addition, a transparent conductive film obtained with the use of, as a material, an organic compound such as polyaniline and derivatives thereof and polythiophene and derivatives thereof may be used as the electrode. The transparent or translucent electrode may serve as anode or a cathode.

As long as one of the pair of electrodes is transparent or translucent, the other electrode may be an electrode with low optical transparency. Examples of the material of the electrode with low optical transparency include metals and conductive polymers. Specific examples of the material of the electrode having low light permeability include metals such as lithium, sodium, potassium, rubidium, cesium, magnesium, calcium, strontium, barium, aluminum, scandium, vanadium, zinc, yttrium, indium, cerium, samarium, europium, terbium, and ytterbium, alloys of two or more of these metals, alloys of one or more of these metals and one or more metals selected from the group consisting of gold, silver, platinum, copper, manganese, titanium, cobalt, nickel, tungsten, and tin, graphite, graphite interlayer compounds, polyaniline, and derivatives thereof, and polythiophene and derivatives thereof. Examples of the alloy include a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

### (Active Layer)

The active layer included in the photoelectric conversion element according to the present embodiment, which is assumed to have a bulk heterojunction-type structure, includes a p-type semiconductor material and an n-type semiconductor material, and includes the compound according to the present embodiment as the n-type semiconductor material (details will be described later).

In the present embodiment, the thickness of the active layer is not particularly limited. The thickness of the active layer can be any suitable thickness in consideration of the balance between the suppression of dark current and the extraction of photocurrent. The thickness of the active layer is preferably 100 nm or more, more preferably 150 nm or more, and still more preferably 200 nm or more, particularly from the viewpoint of further reducing the dark current. In addition, the thickness of the active layer is preferably 10 um or less, more preferably 5 um or less, still more preferably 1 um or less.

In the present embodiment, the active layer is formed in accordance with a step including a treatment of heating at a heating temperature of 200°C or higher (details will be described later.).

In this regard, a p-type semiconductor material that can be suitably used in combination with the n-type semiconductor material that is the already-described compound according to the present embodiment will be described as a material for the active layer according to the present embodiment.

The p-type semiconductor material is preferably a polymer compound that has a predetermined polystyrene-equivalent weight average molecular weight.

In this regard, the polystyrene-equivalent weight average molecular weight means a weight average molecular weight calculated with the use of gel permeation chromatography (GPC) and a polystyrene standard sample.

The polystyrene-equivalent weight average molecular weight of the p-type semiconductor material is preferably 3000 or more and 500,000 or less particularly from the viewpoint of improving the solubility in a solvent.

In the present embodiment, the p-type semiconductor material is preferably a π-conjugated polymer compound (also referred to as a D-A-type conjugated polymer compound) including a donor constituent unit (also referred to as a D constituent unit) and an acceptor constituent unit (also referred to as an A constituent unit). It is to be noted that which is the donor constituent unit or the acceptor constituent unit can be relatively determined from the HOMO or LUMO energy level.

In this regard, the donor constituent unit is a constituent unit that is excessive in π electron, and the acceptor constituent unit is a constituent unit is deficient in π electron.

In the present embodiment, the constituent unit that can constitute the p-type semiconductor material encompasses a constituent unit that has a donor constituent unit and an acceptor constituent unit directly bonded, and further a constituent unit that has a donor constituent unit and an acceptor constituent unit bonded with an arbitrary suitable spacer (group or constituent unit) interposed therebetween.

Examples of the p-type semiconductor material as a polymer compound include polyvinylcarbazole and derivatives thereof, polysilane and derivatives thereof, polysiloxane derivatives that have an aromatic amine structure in a side chain or a main chain, polyaniline and derivatives thereof, polythiophene and derivatives thereof, polypyrrole and derivatives thereof, polyphenylenevinylene and derivatives thereof, polythienylenevinylene and derivatives thereof, and polyfluorene and derivatives thereof.

The p-type semiconductor material according to the present embodiment is preferably a polymer compound including a constituent unit represented by the following formula (VI). The constituent unit represented by the following formula (VI) is typically a donor constituent unit in the present embodiment.

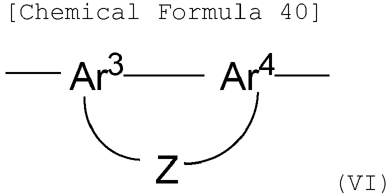

In the formula (VI), Ar³ and Ar⁴ represent trivalent aromatic heterocyclic groups optionally having a substituent, and Z represents a group represented by the following formulas (Z-1) to (Z-7).

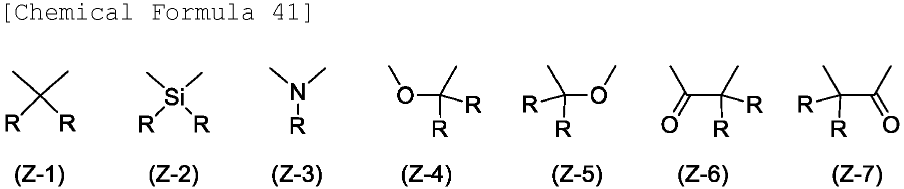

In the formulas (Z-1) to (Z-7),
R is as defined above.

In the case of having two groups R in each of the formulas (Z-1) to (Z-7), the two groups R may be identical to or different from each other.

The aromatic heterocyclic rings that can constitute Ar³ and Ar⁴ encompass a ring with an aromatic ring condensed to a heterocyclic ring, even if the heterocyclic ring itself constituting the ring exhibits no aromaticity, in addition to a monocyclic ring and a condensed ring in which the heterocyclic rings themselves exhibit aromaticity.

Each of the aromatic heterocyclic rings that can constitute Ar³ and Ar⁴ may be a monocyclic ring or a condensed ring. When the aromatic heterocyclic ring is a condensed ring, all of the rings constituting the condensed ring may be condensed rings with aromaticity, or only some thereof may be condensed rings with aromaticity. When these rings have multiple substituents, these substituents may be identical or different.

Specific examples of the aromatic carbocyclic rings that can constitute Ar³ and Ar⁴ include a benzene ring, a naphthalene ring, an anthracene ring, a tetracene ring, a pentacene ring, a pyrene ring, and a phenanthrene ring, and preferred are a benzene ring and a naphthalene ring, more preferred are a benzene ring and a naphthalene ring, and still more preferred is a benzene ring. These rings may have a substituent.

Specific examples of the aromatic heterocyclic rings include the ring structures of the compounds already described as the aromatic heterocyclic compound, and include an oxadiazole ring, a thiadiazole ring, a thiazole ring, an oxazole ring, a thiophene ring, a pyrrole ring, a phosphole ring, a furan ring, a pyridine ring, a pyrazine ring, a pyrimidine ring, a triazine ring, a pyridazine ring, a quinoline ring, an isoquinoline ring, a carbazole ring, and a dibenzophosphole ring, and a phenoxazine ring, a phenothiazine ring, a dibenzoborol ring, a dibenzosilole ring, and a benzopyran ring. These rings may have a substituent.

The constituent unit represented by the formula (VI) is preferably a constituent unit represented by the following formula (VII) or (VIII). In other words, in the present embodiment, the p-type semiconductor material is preferably a polymer compound including a constituent unit represented by the following formula (VII) or (VIII).

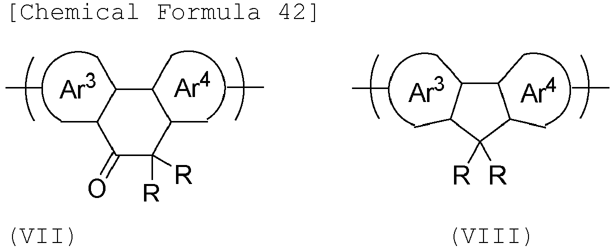

In the formulas (VII) and (VIII), Ar³, Ar⁴, and R are as defined above.

Examples of the preferred constituent units represented by the formulas (VI) and (VIII) include constituent units represented by the following formulas (VI-1) and (VI-2) and formulas (VIII-1) and (VIII-2).

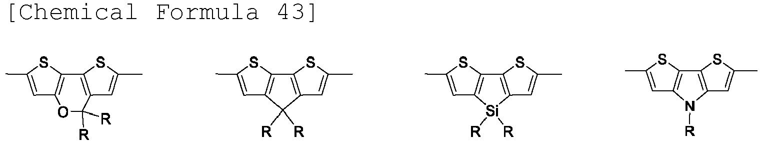

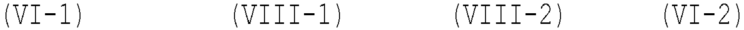

In the formulas (VI-1), (VI-2), (VIII-1), and (VIII-2),
R is as defined above.

In the case of having two groups R, the two groups R may be identical to or different.

Examples of a more specific preferred constituent unit represented by the formula (VI-1) include constituent units represented by the following formulas (VI-1-1) and (VI-1-2) .

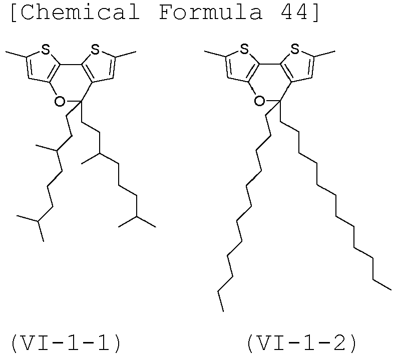

In addition, the constituent unit represented by the formula (VII) is preferably a constituent unit represented by the following formula (IX). In other words, in the present embodiment, the p-type semiconductor material may be a polymer compound including a constituent unit represented by the following formula (IX).

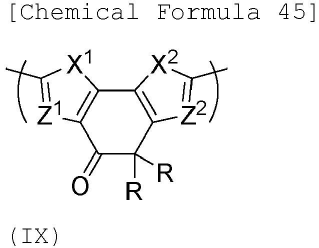

In the formula (IX),
X¹ and X² are each independently a sulfur atom or an oxygen atom,
Z¹ and Z² are each independently a group represented by =C(R)- or a nitrogen atom, and
R is as defined above.

Examples of the preferred constituent unit represented by the formula (IX) include constituent units represented by the following formulae (IX-1) to (IX-16).

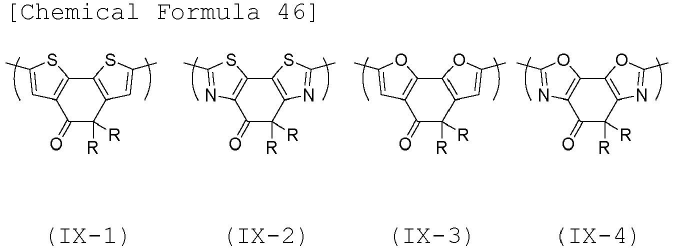

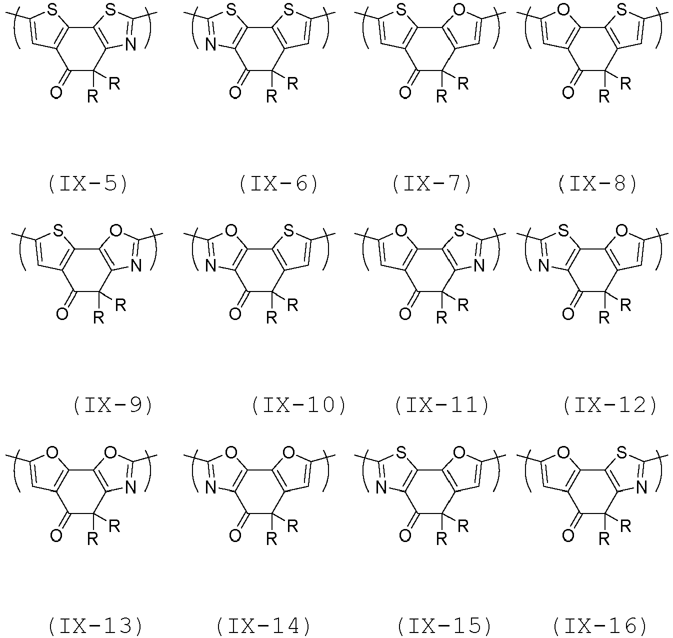

The constituent unit represented by the formula (IX) is preferably a constituent unit where X¹ and X² are sulfur atoms, and Z¹ and Z² are groups represented by =C(R)-.

In the present embodiment, the polymer compound to serve as the p-type semiconductor material preferably includes a constituent unit represented by the following formula (X). The constituent unit represented by the following formula (X) is typically an acceptor constituent unit in the present embodiment.

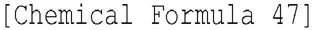

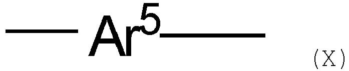

In the formula (X), Ar⁵ represents a divalent aromatic heterocyclic group.

The number of carbon atoms of the divalent aromatic heterocyclic group represented by Ar⁵ is typically 2 to 60, preferably 4 to 60, more preferably 4 to 20.

The divalent aromatic heterocyclic group represented by Ar⁵ optionally has a substituent. Examples of the substituent optionally included in the divalent aromatic heterocyclic group represented by Ar⁵ include a halogen atom, an alkyl group optionally having a substituent, an aryl group optionally having a substituent, an alkyloxy group optionally having a substituent, an aryloxy group optionally having a substituent, an alkylthio group optionally having a substituent, an arylthio group optionally having a substituent, a monovalent heterocyclic group optionally having a substituent, a substituted amino group optionally having a substituent, an acyl group optionally having a substituent, an imine residue optionally having a substituent, an amide group optionally having a substituent, an acid imide group optionally having a substituent, a substituted oxycarbonyl group optionally having a substituent, an alkenyl group optionally having a substituent, an alkynyl group optionally having a substituent, a cyano group, and a nitro group.

As the constituent unit represented by the formula (X), constituent units represented by the following formulas (X-1) to (X-10) are preferred.

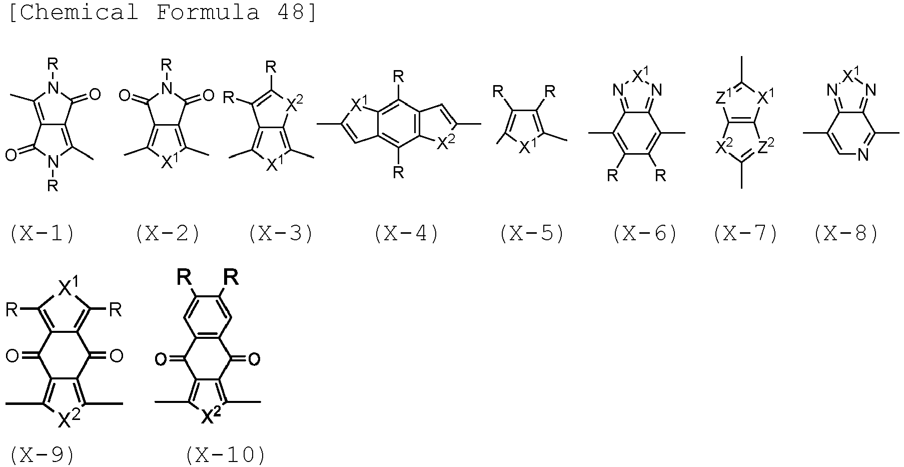

In the formulas (X-1) to (X-10),
X¹, X², Z¹, Z², and R are as defined above.

In the case of having two groups R, the two groups R may be identical to or different from each other.

From the viewpoint of availability of the raw material compounds, X¹ and X² in the formulas (X-1) to (X-10) are each preferably a sulfur atom.

It is to be noted that the constituent units represented by the formulas (X-1) to (X-10) can typically function as acceptor constituent units as mentioned above. The present invention is, however, not limited thereto, and in particular, the constituent units represented by the formulas (X-4), (X-5), and (X-7) can also function as donor constituent units.

In the present embodiment, the p-type semiconductor material is preferably a π-conjugated polymer compound including a constituent unit including a thiophene skeleton and including a π-conjugated system.

Specific examples of the divalent aromatic heterocyclic group represented by Ar⁵ include groups represented by the following formulas (101) to (191). These groups may further have a substituent.

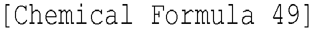

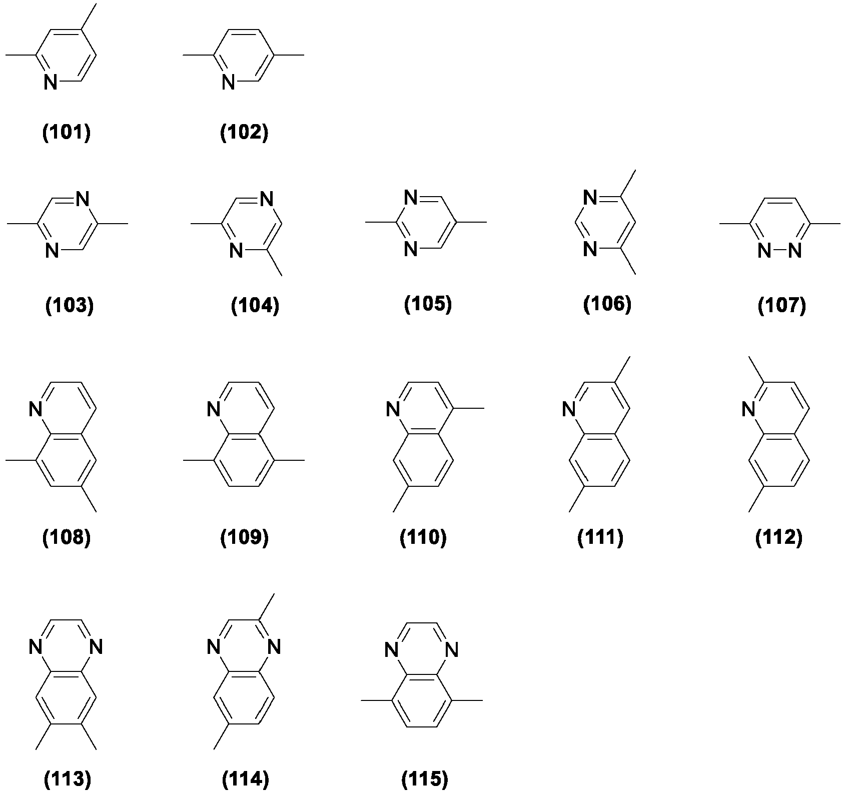

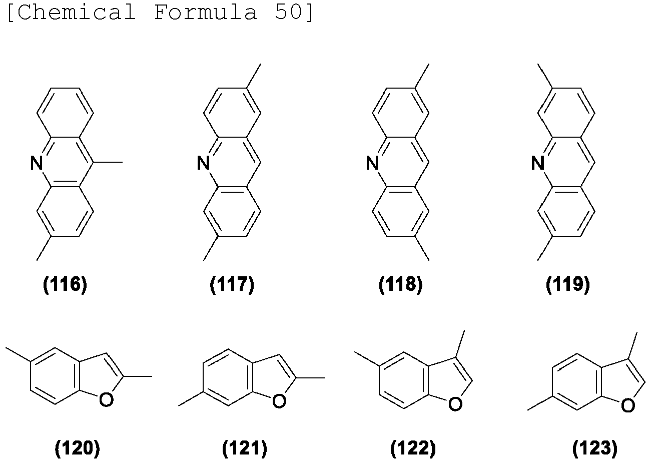

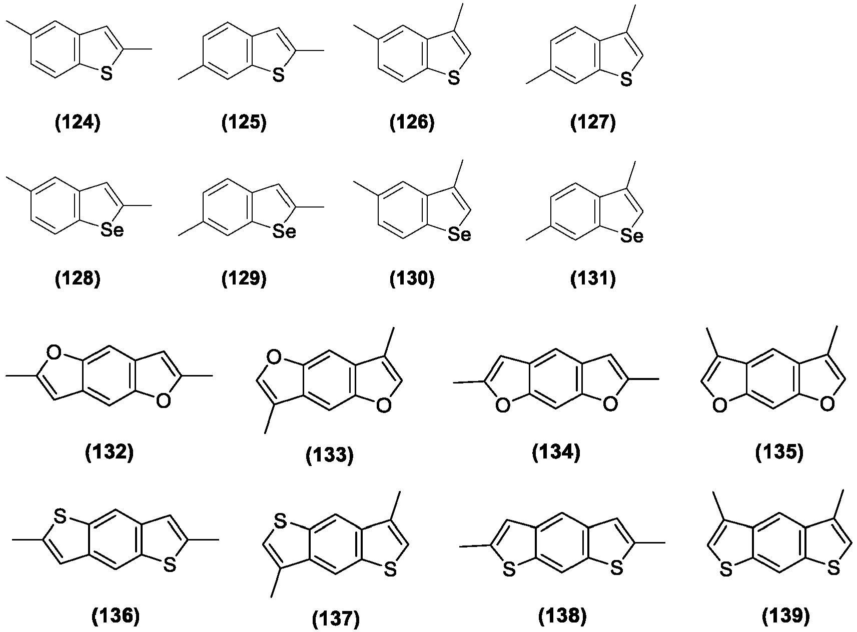

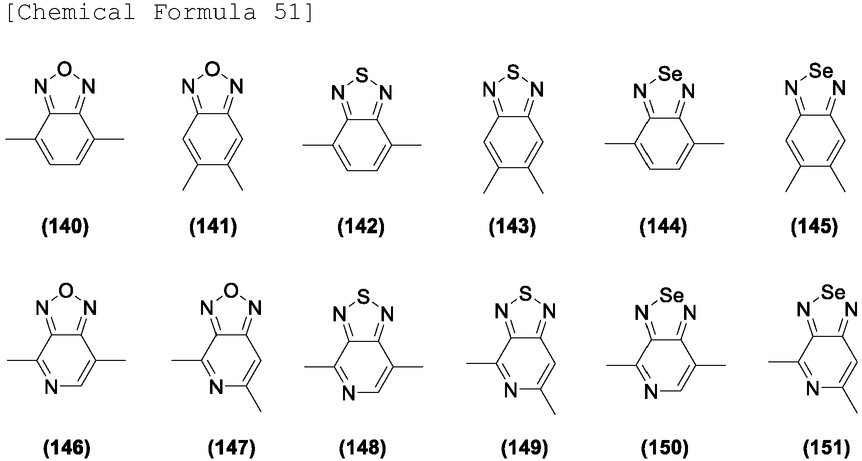

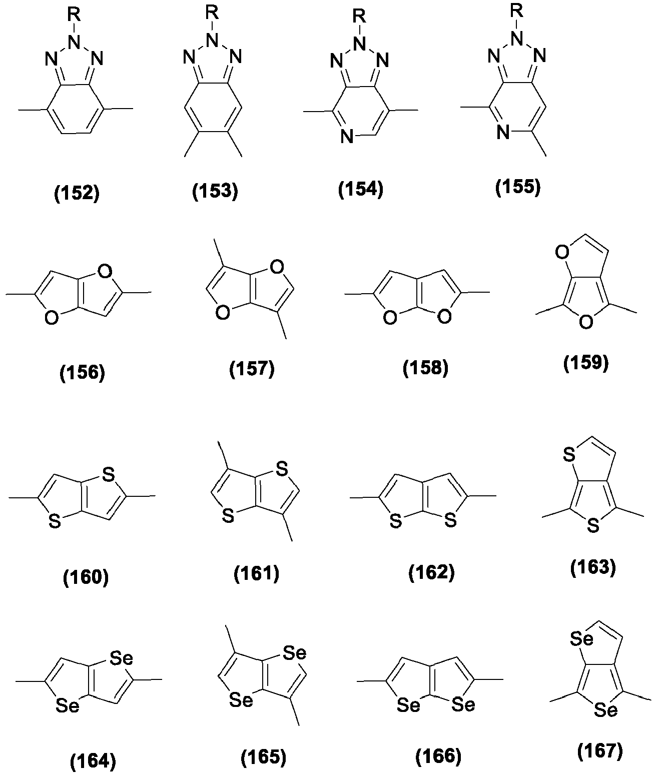

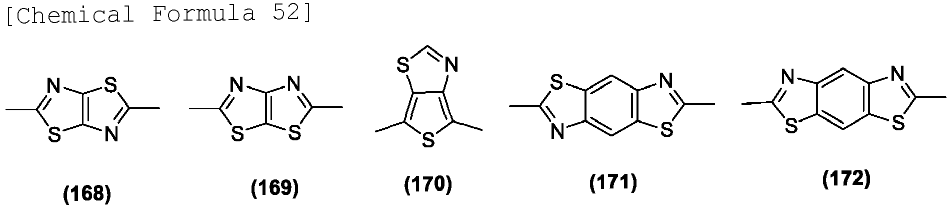

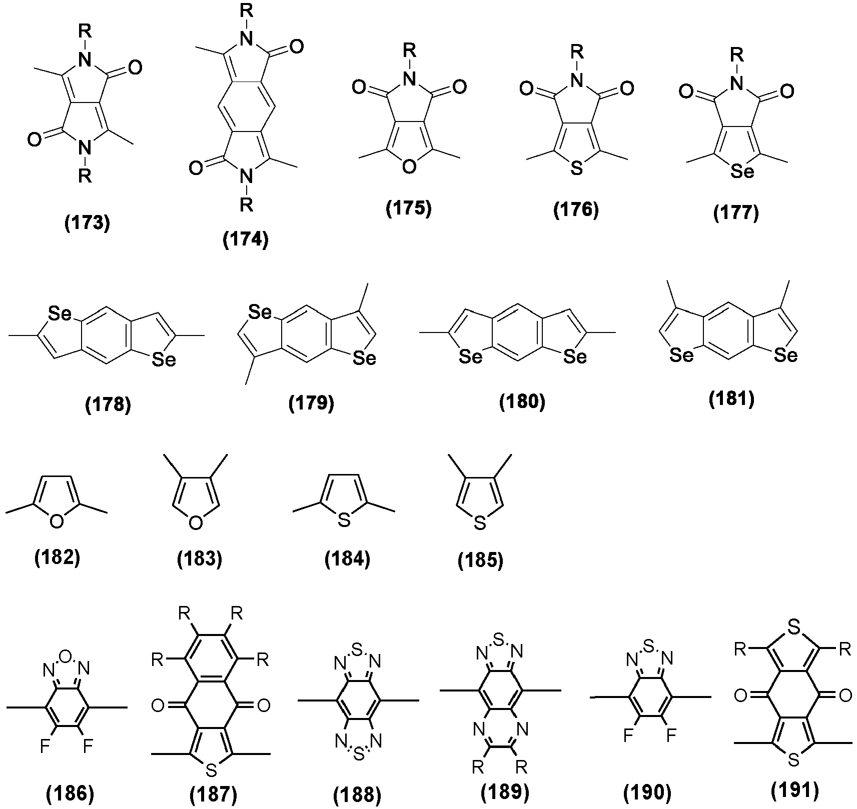

In the formulas (101) to (191), R has the same meaning as mentioned above. In the case of having multiple groups R, the multiple groups R may be identical to or different from each other.

The polymer compound to serve as the p-type semiconductor material according to the present embodiment is preferably a π-conjugated polymer compound including a constituent unit represented by the formula (VI) as a donor constituent unit and including a constituent unit represented by the formula (X) as an acceptor constituent unit.

The polymer compound to serve as the p-type semiconductor material may include two or more types of constituent units represented by the formula (VI), and may include two or more types of constituent units represented by the formula (X).

For example, from the viewpoint of improving the solubility in a solvent, the polymer compound to serve as the p-type semiconductor material according to the present embodiment may include a constituent unit represented by the following formula (XI).

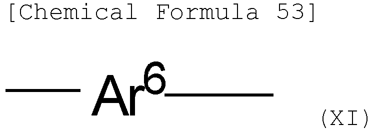

In the formula (XI), Ar⁶ represents an arylene group.

The arylene group represented by Ar⁶ means a remaining atomic group excluding two hydrogen atoms from an aromatic hydrocarbon optionally having a substituent. The aromatic hydrocarbon also encompasses a compound having a condensed ring, and a compound where two or more selected from the group consisting of independent benzene rings and condensed rings are bonded directly or with a divalent group such as a vinylene group interposed therebetween.

Examples of the substituent optionally included in the aromatic hydrocarbon include the same substituents as the substituents exemplified as substituents optionally included in the heterocyclic compound.

The number of carbon atoms of the arylene group represented by Ar⁶ is typically 6 to 60, preferably 6 to 20, excluding the number of carbon atoms of the substituent. The number of carbon atoms of the arylene group including the substituent is typically 6 to 100.

Examples of the arylene group represented by Ar⁶ include phenylene groups (for example, the following formulas 1 to 3), naphthalene-diyl groups (for example, the following formulas 4 to 13), anthracene-diyl groups (for example, the following formulas 14 to 19), biphenyl-diyl groups (for example, the following formulas 20 to 25), terphenyl-diyl groups (for example, the following formulas 26 to 28), condensed ring compound groups (for example, the following formulas 29 to 35), fluorene-diyl groups (for example, the following formulas 36 to 38), and benzofluorene-diyl groups (for example, the following formulas 39 to 46).

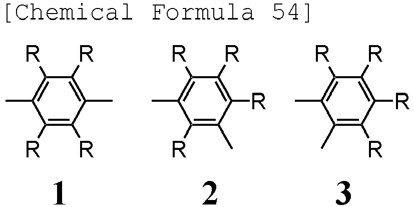

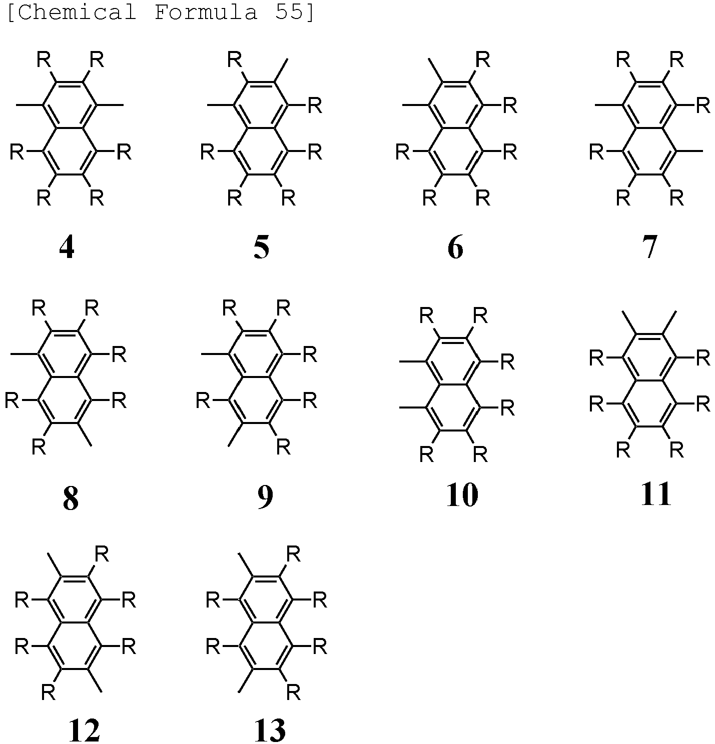

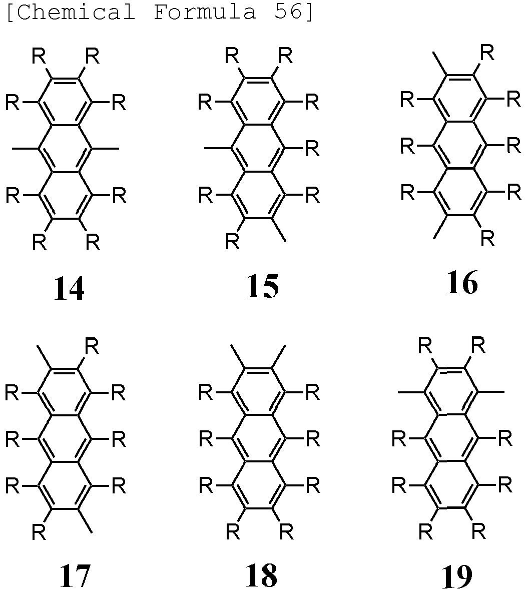

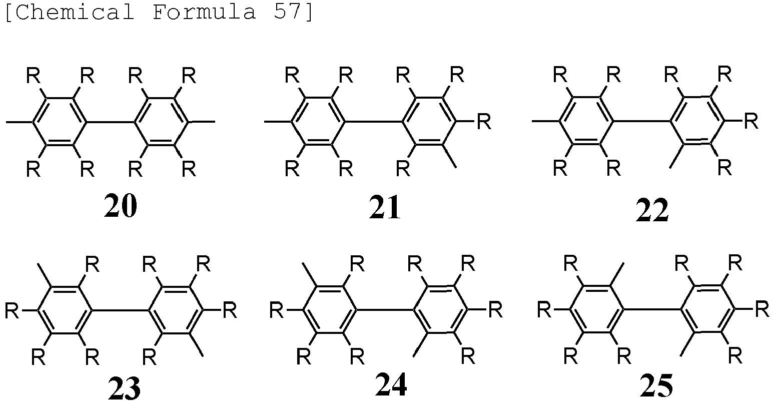

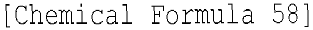

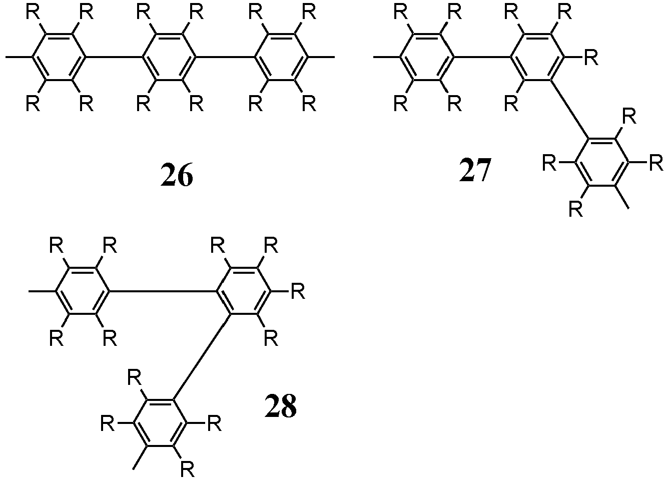

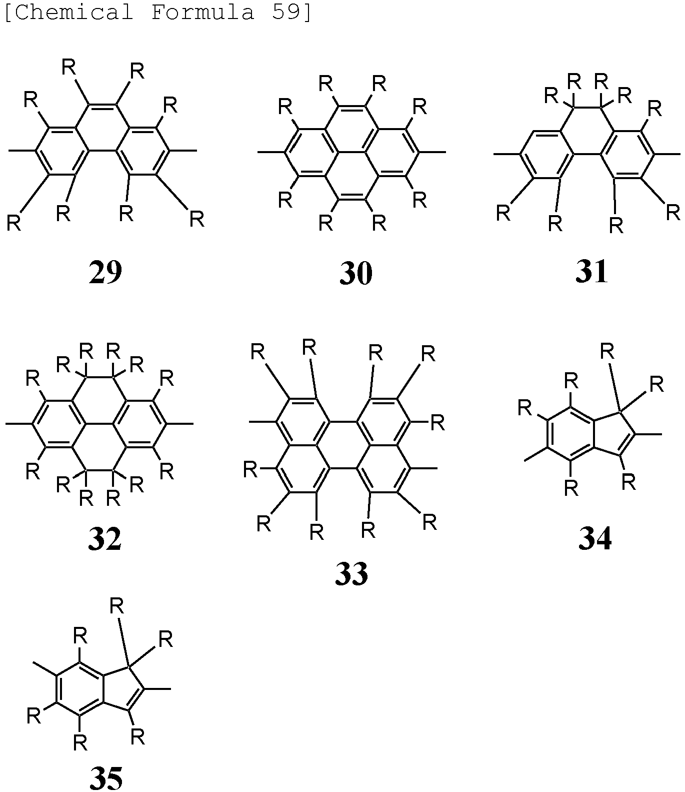

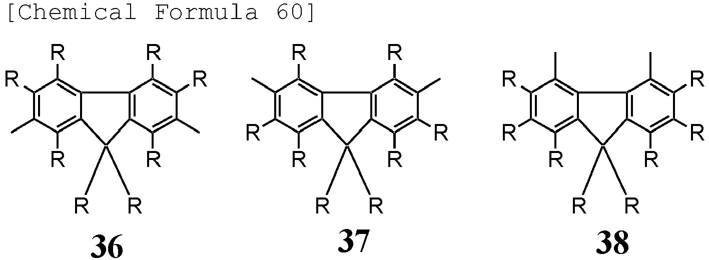

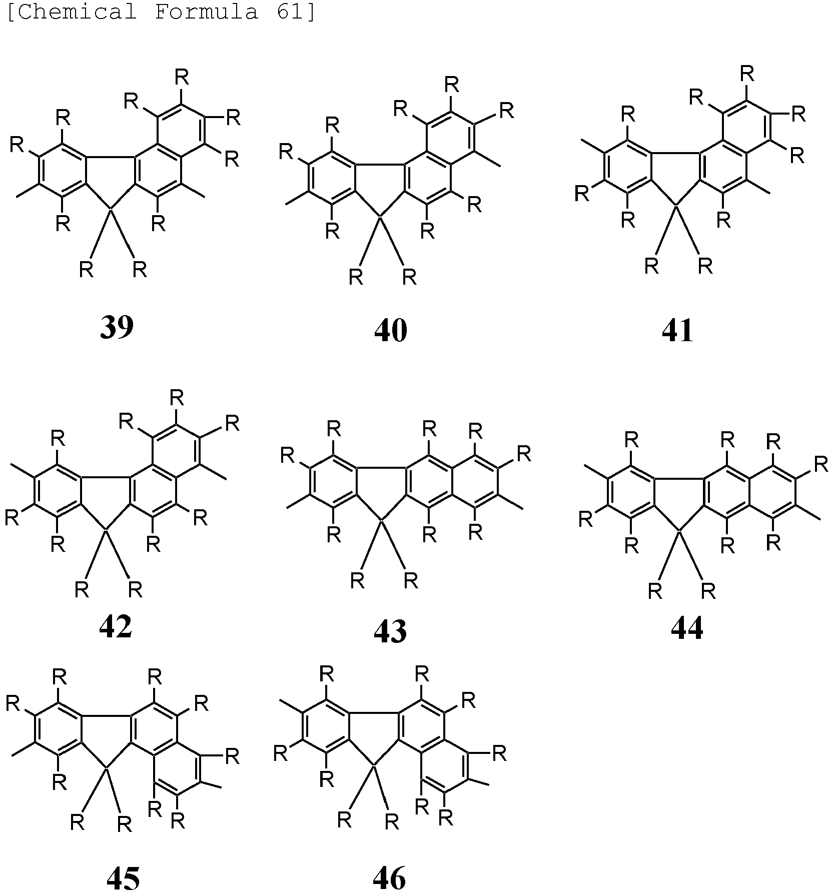

In the formulas, R is as defined above, the multiple groups R may be identical to or different from each other.

The constituent unit represented by the formula (XI) is preferably a constituent unit represented by the following formula (XII).

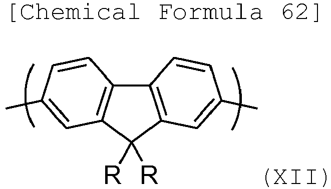

In formula (XII), R is as defined above. The two groups R may be identical to or different from each other.

The constituent unit constituting the polymer compound to serve as the p-type semiconductor material may be a constituent unit that has two or more constituent units combined and linked for each of two or more types of constituent units selected from the above-mentioned constituent units.

When the polymer compound to serve as the p-type semiconductor material includes the constituent unit represented by the formula (VI) and/or the constituent unit represented by the formula (X), the total amount of the constituent unit represented by the formula (VI) and the constituent unit represented by the formula (X) is typically 20 mol% to 100 mol% when the amount of all of the constituent units included in the polymer compound is determined be 100 mol%, and is preferably 40 mol% to 100 mol%, more preferably 50 mol% to 100 mol% because the charge transportability as the p-type semiconductor material can be improved.

Specific examples of the polymer compound to serve as the p-type semiconductor material according to the present embodiment include polymer compounds represented by the following formulas (P-1) to (P-17):

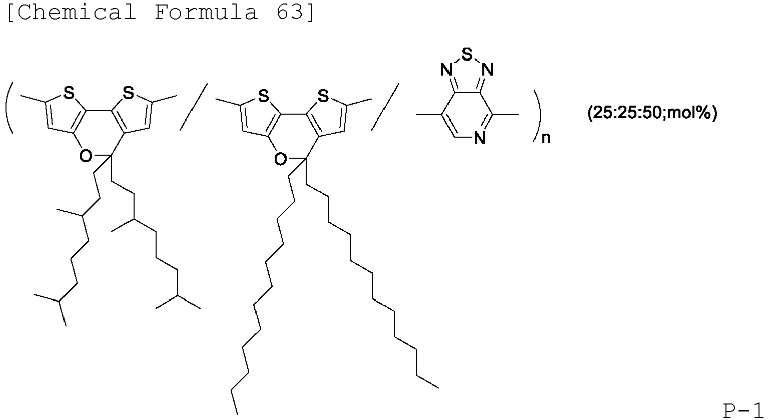

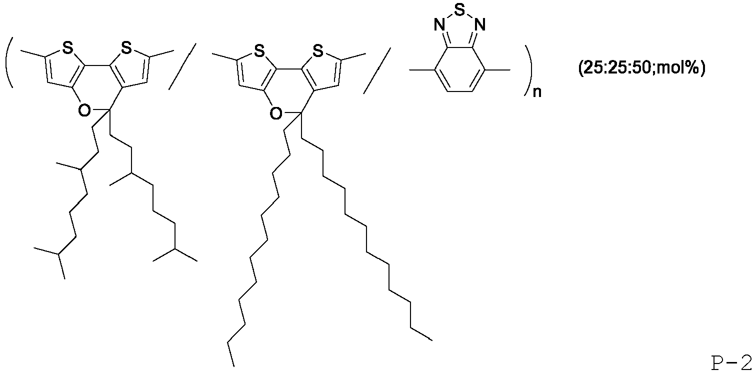

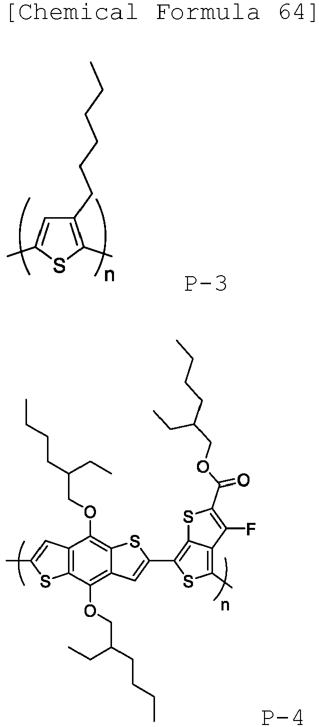

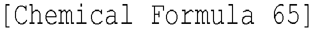

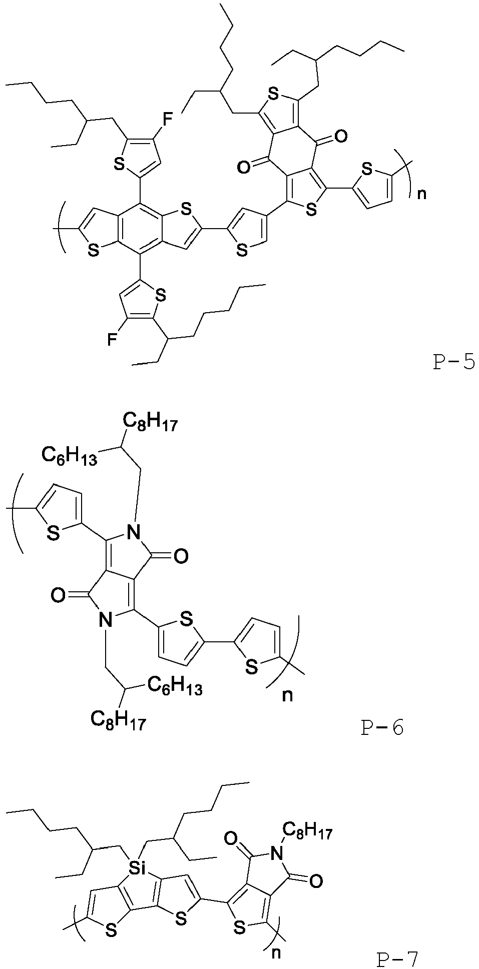

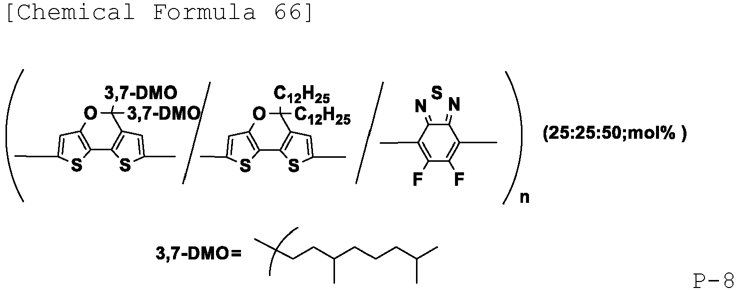

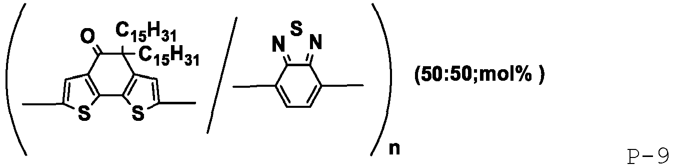

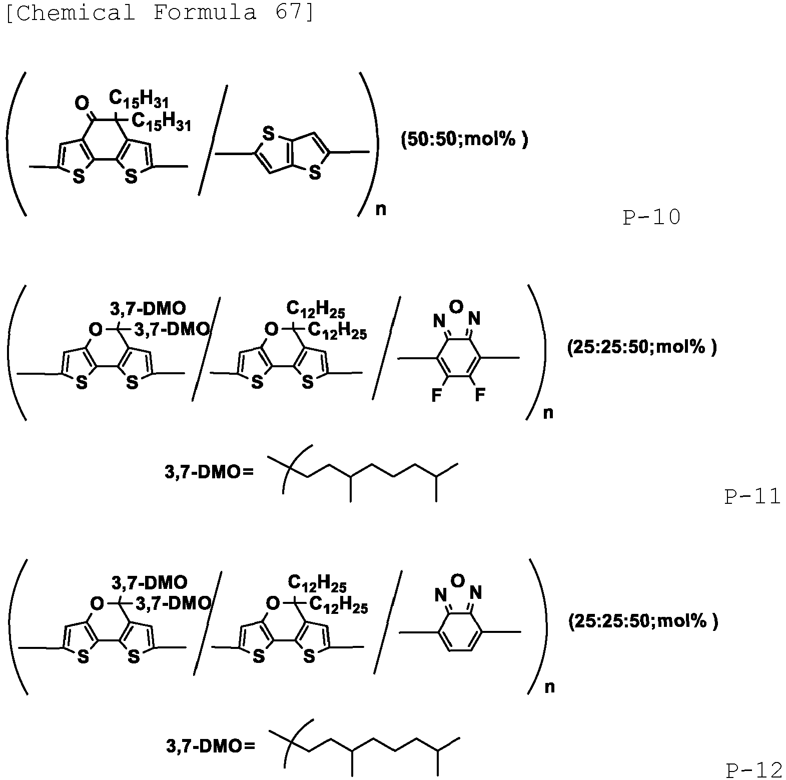

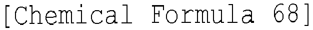

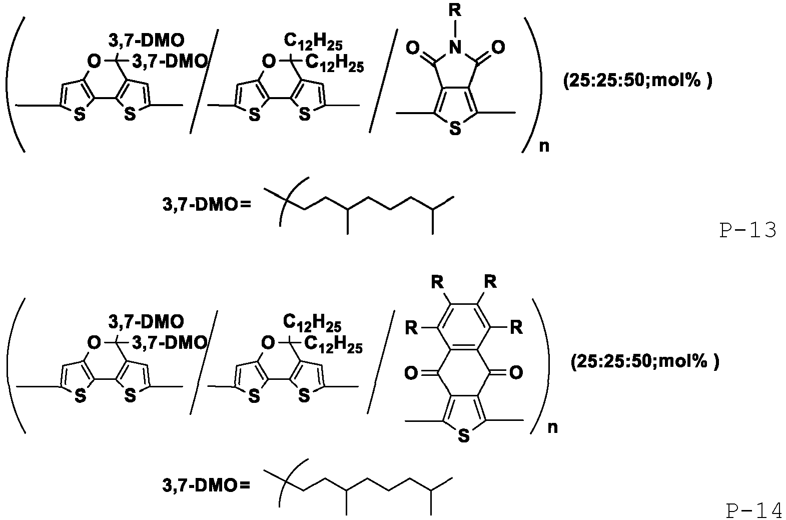

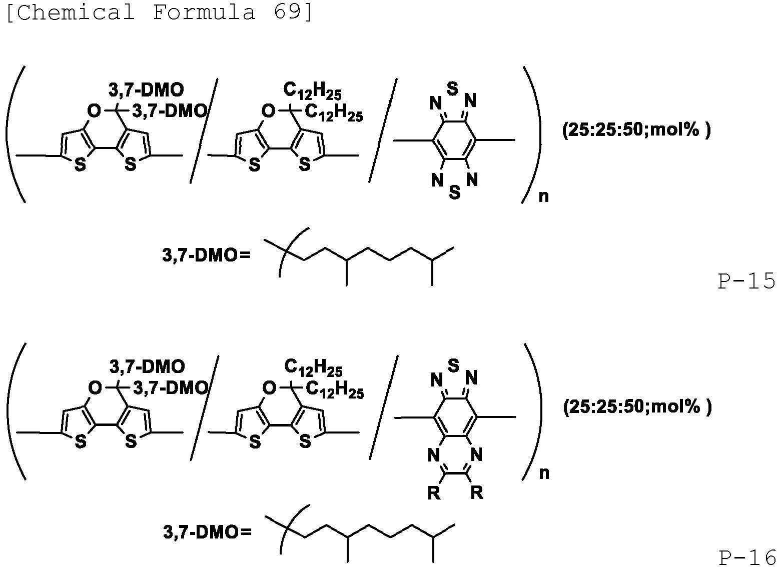

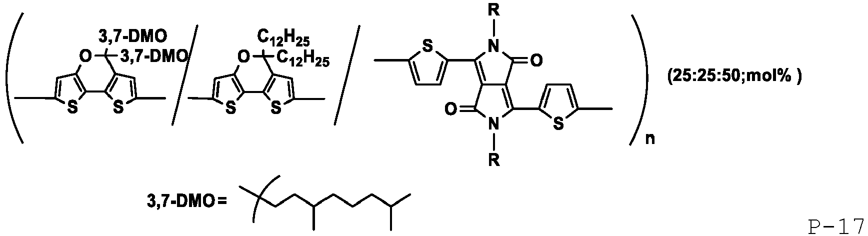

In the formulas, R is as defined above, the multiple groups R may be identical to or different from each other.

The use of the polymer compound exemplified above as the p-type semiconductor material can suppress a decrease in EQE with respect to a heating treatment in a step of manufacturing a photoelectric conversion element, a step of incorporating the photoelectric conversion element into a device to which the element is applied, or the like, or further improve the EQE, and improve the heat resistance of the photoelectric conversion element.

### (Intermediate Layer)

As illustrated in Fig. 1, the photoelectric conversion element according to the present embodiment preferably includes, for example, an intermediate layer (buffer layer) such as a charge transport layer (electron transport layer, hole transport layer, electron injection layer, hole injection layer) as a constituent element for improving characteristics such as a photoelectric conversion efficiency.

In addition, examples of the material for use in the intermediate layer include metals such as calcium, inorganic oxide semiconductors such as a molybdenum oxide and a zinc oxide, and a mixture of PEDOT (poly(3,4-ethylenedioxythiophene)) and PSS (poly(4-styrenesulfonate)) (PEDOT : PSS).

As shown in Fig. 1, the photoelectric conversion element preferably includes a hole transport layer between the anode and the active layer. The hole transport layer has the function of transporting holes from the active layer to the electrode.

The hole transport layer provided in contact with the anode may be referred to particularly as a hole injection layer. The hole transport layer (hole injection layer) provided in contact with the anode has the function of promoting the injection of holes into the anode. The hole transport layer (hole injection layer) may be in contact with the active layer.

The hole transporting layer contains a hole transporting material. Examples of the hole transporting material include polythiophene and derivatives thereof, aromatic amine compounds, polymer compounds including a constituent unit having an aromatic amine residue, CuSCN, CuI, NiO, a tungsten oxide (WO₃), and a molybdenum oxide (MoO₃).

The intermediate layer can be formed by any suitable forming method known conventionally. The intermediate layer can be formed by a vacuum deposition method or a coating method that is similar to the method for forming the active layer.

The photoelectric conversion element according to the present embodiment preferably has a configuration with an electron transport layer for the intermediate layer, where the substrate (support substrate), the anode, the hole transport layer, the active layer, the electron transport layer, and the cathode are stacked in this order so as to be in contact with each other.

As illustrated in Fig. 1, the photoelectric conversion element according to the present embodiment preferably includes an electron transport layer as the intermediate layer between the cathode and the active layer. The electron transport layer has the function of transporting electrons from the active layer to the cathode. The electron transport layer may be in contact with the cathode. The electron transport layer may be in contact with the active layer.

The electron transport layer provided in contact with the cathode may be referred to particularly as an electron injection layer. The electron transport layer (electron injection layer) provided in contact with the cathode has the function of promoting the injection of electrons generated in the active layer into the cathode.

The electron transport layer contains an electron transporting material. Examples of the electron transporting material include polyalkyleneimine and derivatives thereof, polymer compounds including a fluorene structure, metals such as calcium, and metal oxides.

Examples of the polyalkyleneimine and derivatives thereof include polymers obtained by a conventional method of polymerizing one, or two or more of alkyleneimines having 2 to 8 carbon atoms, particularly alkyleneimines having 2 to 4 carbon atoms, such as an ethyleneimine, a propyleneimine, a butyleneimine, a dimethylethyleneimine, a pentyleneimine, a hexyleneimine, a heptyleneimine, and an octyleneimine, and the polymers chemically modified by reacting the polymers with various compounds. As the polyalkyleneimine and derivatives thereof, a polyethyleneimine (PEI) and an ethoxylated polyethyleneimine (PEIE) are preferred.

Examples of the polymers compound including a fluorene structure include poly[(9,9-bis(3'-(N,N-dimethylamino)propyl)-2,7-fluorene)-ortho-2,7-(9,9'-dioctylfluorene)] (PFN) and PFN-P2.

Examples of the metal oxide include a zinc oxide, a gallium-doped zinc oxide, an aluminum-doped zinc oxide, a titanium oxide, and a niobium oxide. As the metal oxides, metal oxides containing zinc are preferred, and a zinc oxide is particularly preferred.

Examples of other electron transporting materials include poly(4-vinylphenol) and perylene diimide.

### (Sealing Member)

The photoelectric conversion element according to the present embodiment preferably further includes a sealing member, for providing a sealed body sealed with the sealing member.

Any suitable member known conventionally can be used for the sealing member. Examples of the sealing member include a combination of a glass substrate as a substrate (sealing substrate) and a sealing material (adhesive) such as a UV curable resin.

The sealing member may be a sealing layer that has a layer structure of one or more layers. Examples of the layer constituting the sealing layer include a gas barrier layer and a gas barrier film.

The sealing layer is preferably formed from a material that has the property of blocking moisture (water vapor barrier property) or the property of blocking oxygen (oxygen barrier property). Examples of suitable materials as a material for the sealing layer include organic materials such as a polyethylene trifluoride, a polytrifluoroethylene chloride (PCTFE), a polyimide, a polycarbonate, a polyethylene terephthalate, an alicyclic polyolefin, and an ethylene-vinyl alcohol copolymer, and inorganic materials such as a silicon oxide, a silicon nitride, an aluminum oxide, and diamond-like carbon.

The sealing member is typically made of a material that can withstand a heating treatment performed in incorporating the sealing member into a device to which the photoelectric conversion element is applied, for example, according to the following application example.

### (Application of Photoelectric Conversion Element)

Examples of applications of the photoelectric conversion element according to the present embodiment include a photodetection element and a solar cell.

More specifically, the photoelectric conversion element according to the present embodiment, with a voltage (reverse bias voltage) applied between the electrodes, is capable of causing a photocurrent to flow with light irradiation from the transparent or translucent electrode side, thereby allowing for operating as a photodetection element (photosensor). In addition, a plurality of photodetection elements can also be integrated for use as an image sensor. As described above, the photoelectric conversion element according to the present embodiment can be suitably used particularly as a photodetection element.

In addition, the photoelectric conversion element according to the present embodiment is capable of generating photovoltaic power between the electrodes with light irradiation, thereby allowing for operating as a solar cell. A plurality of photoelectric conversion elements can also be integrated for a solar cell module.

### (Application Example of Photoelectric Conversion Element)

The photoelectric conversion element according to the present embodiment can be suitably applied as a photodetection element to a detection unit included in various electronic devices such as a workstation, a personal computer, a portable information terminal, an access control system, a digital camera, and a medical device.

The photoelectric conversion element according to the present embodiment can be suitably applied to, for example, an image detection unit for solid-state imaging devices such as an X-ray imaging device and a CMOS image sensor (for example, an image sensor such as an X-ray sensor), a detection unit of a biometric information authentication device that detects a predetermined feature of a part of a living body, such as a fingerprint detection unit, a face detection unit, a vein detection unit, and an iris detection unit (for example, near infrared sensor), a detection unit of an optical biosensor such as a pulse oximeter, and the like included in the above-mentioned exemplary electronic devices.

The photoelectric conversion element according to the present embodiment can be also suitably applied as an image detection unit for a solid-state imaging device to a Time-of-Flight (TOF) type distance measuring device (TOF-type distance measuring device).

In the TOF-type distance measuring device, a distance is measured by causing a photoelectric conversion element to receive reflected light through reflection of radiation light from a light source at a measurement target. Specifically, the distance to the measurement target is determined by detecting the time-of-flight from reflection of irradiation light emitted from the light source at the measurement target to return as reflected light. The TOF type has a direct TOF method and an indirect TOF method. The difference between the time of light irradiation from a light source and the time of receiving reflected light at the photoelectric conversion element is directly measured in the direct TOF method, whereas the distance is measured by converting a change in the charge accumulation amount depending on the time-of-flight into a time change in the indirect TOF method. The distance measuring principle for obtaining the time-of-flight with the charge accumulation for use in the indirect TOF method has a continuous wave (particularly sinusoidal wave) modulation method and a pulse modulation method for determining the time-of-flight from phases of light emitted from a light source and reflected light reflected at a measurement target.

Among detection units to which the photoelectric conversion element according to the present embodiment can be suitably applied, configuration examples of an image detection unit for a solid-state imaging device and an image detection unit for an X-ray imaging device, a fingerprint detection unit and a vein detection unit for a biometric authentication device (for example, a fingerprint authentication device and a vein authentication device), and an image detection unit of a TOF-type distance measuring device (indirect TOF system) will be described below with reference to the drawings.

### (Image Detection Unit for Solid-state Imaging Device)

Fig. 2 is a diagram schematically illustrating a configuration example of an image detection unit for a solid-state imaging device.

The image detection unit 1 includes a CMOS transistor substrate 20, an interlayer insulating film 30 provided so as to cover the CMOS transistor substrate 20, a photoelectric conversion element 10 according to an embodiment of the present invention, provided on the interlayer insulating film 30, an interlayer wiring part 32 provided so as to penetrate through the interlayer insulating film 30, for electrically connecting the CMOS transistor substrate 20 and the photoelectric conversion element 10, a sealing layer 40 provided so as to cover the photoelectric conversion element 10, and a color filter 50 provided on the sealing layer 40.

The CMOS transistor substrate 20 has, in an aspect in accordance with a design, any suitable configuration known conventionally.

The CMOS transistor substrate 20 includes a transistor, a capacitor, and the like formed within the thickness of the substrate, and includes functional elements such as a CMOS transistor circuit (MOS transistor circuit) for achieving various functions.

Examples of the functional elements include a floating diffusion, a reset transistor, an output transistor, and a selection transistor.

With such functional elements, wirings, and the like, a signal reading circuit and the like are built in the CMOS transistor substrate 20.

The interlayer insulating film 30 can be made of any suitable insulating material known conventionally, such as a silicon oxide or an insulating resin. The interlayer wiring part 32 can be made of any suitable conductive material (wiring material) known conventionally, such as copper or tungsten. The interlayer wiring part 32 may be, for example, an in-hole wiring formed simultaneously with the formation of the wiring layer, or a buried plug formed separately from the wiring layer.

The sealing layer 40 can be made of any suitable material known conventionally, on the condition that harmful substances such as oxygen and water, which may possibly functionally deteriorate the photoelectric conversion element 10, can be prevented or kept from permeating. The sealing layer 40 may have the same configuration as the already-described sealing member 17.

As the color filter 50, for example, a primary color filter made of any suitable material known conventionally and corresponding to the design of the image detection unit 1 can be used. In addition, as the color filter 50, a complementary color filter can be also used, which can be reduced in thickness as compared with the primary color filter. As the complementary color filter, a color filter can be used, which has, for example, three types of (yellow, cyan, magenta), three types of (yellow, cyan, transparent), three types of (yellow, transparent, magenta), and three types of (transparent, cyan, magenta) combined. These color filters can be arbitrarily suitably arranged to correspond to the designs of the photoelectric conversion element 10 and CMOS transistor substrate 20 on the condition that color image data can be generated.

The light received by the photoelectric conversion element 10 through the color filter 50 is converted by the photoelectric conversion element 10 into an electric signal corresponding to the amount of received light, and is output through the electrode to the outside of the photoelectric conversion element 10 as a received light signal, that is, an electric signal corresponding to an imaging target.

Next, the received light signal output from the photoelectric conversion element 10 is input via the interlayer wiring part 32 to the CMOS transistor substrate 20, is read by a signal reading circuit built in the CMOS transistor substrate 20, and is subjected to signal processing by any additional suitable functional unit (not illustrated) known conventionally, thereby generating image information based on the imaging target.

### (Fingerprint Detection Unit)

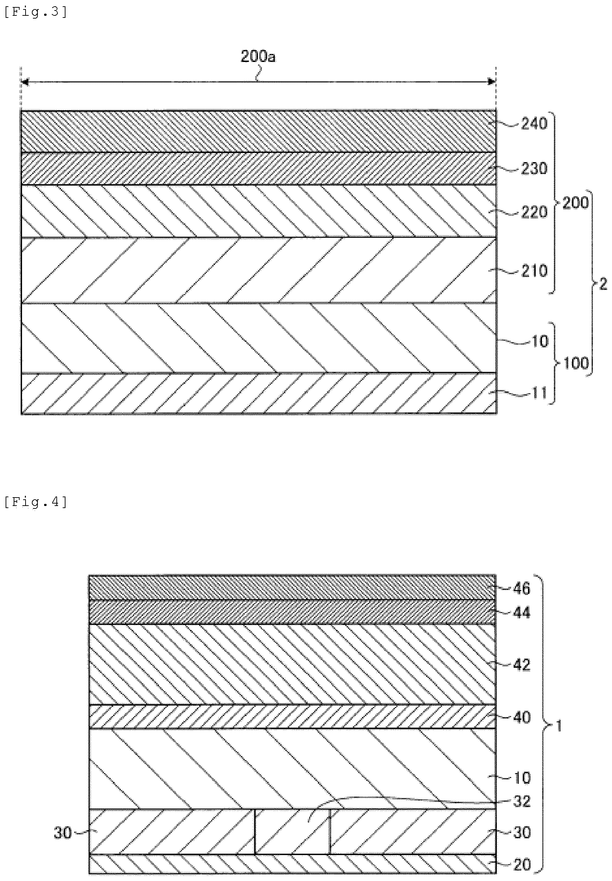

Fig. 3 is a diagram schematically illustrating a configuration example of a fingerprint detection unit integrally configured in a display device.

The display device 2 of a portable information terminal includes a fingerprint detection unit 100 including the photoelectric conversion element 10 according to the embodiment of the present invention as a main constituent element, and a display panel unit 200 provided on the fingerprint detection unit 100 for displaying a predetermined image.

In this configuration example, the fingerprint detection unit 100 is provided in an area that coincides with a display area 200a of the display panel unit 200. In other words, the display panel unit 200 is integrally stacked above the fingerprint detection unit 100.

In the case of performing the fingerprint detection only in a partial area of the display area 200a, the fingerprint detection unit 100 may be provided to correspond to only the partial area.

The fingerprint detection unit 100 includes the photoelectric conversion element 10 according to the embodiment of the present invention as a functional unit that has an essential function. The fingerprint detection unit 100 can include any suitable member known conventionally, such as a protection film (protection film), a support substrate, a sealing substrate, a sealing member, a barrier film, a bandpass filter, or an infrared cut film (not illustrated) in accordance with an aspect corresponding to such a design that can achieve desired characteristics. For the fingerprint detection unit 100, the configuration of the already described image detection unit can be also employed.

The photoelectric conversion element 10 can be included in accordance with any aspect in the display area 200a. For example, a plurality of photoelectric conversion elements 10 may be arranged in a matrix.

The photoelectric conversion element 10 is, as described already, provided on the support substrate 11, and the support substrate 11 is provided with an electrode (anode or cathode) in, for example, a matrix.

The light received by the photoelectric conversion element 10 is converted by the photoelectric conversion element 10 into an electric signal corresponding to the amount of received light, and is output via the electrode to the outside of the photoelectric conversion element 10 as a received light signal, that is, an electric signal corresponding to the imaged fingerprint.

In this configuration example, the display panel unit 200 is configured as an organic electroluminescence display panel (organic EL display panel) including a touch sensor panel. The display panel unit 200 may be configured by, for example, instead of the organic EL display panel, a display panel that has any suitable configuration known conventionally, such as a liquid crystal display panel including a light source such as a backlight.

The display panel unit 200 is provided on the already described fingerprint detection unit 100. The display panel unit 200 includes an organic electroluminescence element (organic EL element) 220 as a functional unit that has an essential function. The display panel unit 200 may further include any suitable member known conventionally, such as a substrate (support substrate 210 or sealing substrate 240), which may be any suitable glass substrate known conventionally, a sealing member, a barrier film, a polarizing plate such as a circularly polarizing plate, and the touch sensor panel 230 in accordance with an aspect corresponding to desired characteristics.

In the configuration example described above, the organic EL element 220 is used as a light source for pixels in the display area 200a, and is also used as a light source for imaging a fingerprint in the fingerprint detection unit 100.

In this regard, the operation of the fingerprint detection unit 100 will be briefly described.

At the time of executing fingerprint authentication, the fingerprint detection unit 100 detects a fingerprint with the use of light emitted from the organic EL element 220 of the display panel unit 200. Specifically, the light emitted from the organic EL element 220 is transmitted through the constituent elements between the organic EL element 220 and the photoelectric conversion element 10 of the fingerprint detection unit 100, and then reflected by the skin (finger surface) of a fingertip of a finger placed so as to be in contact with the surface of the display panel unit 200 in the display area 200a. At least a part of the light reflected by the finger surface is transmitted through the constituent elements therebetween, then received by the photoelectric conversion element 10, and converted into an electric signal corresponding to the amount of light received by the photoelectric conversion element 10. Then, image information on the fingerprint of the finger surface is made from the converted electric signal.

The portable information terminal including the display device 2 compares the obtained image information with previously recorded fingerprint data for fingerprint authentication to perform fingerprint authentication in accordance with any suitable step known conventionally.

### (Image Detection Unit for X-ray Imaging Device)

Fig. 4 is a diagram schematically illustrating a configuration example of an image detection unit for an X-ray imaging device.

The image detection unit 1 for an X-ray imaging device includes a CMOS transistor substrate 20, an interlayer insulating film 30 provided so as to cover the CMOS transistor substrate 20, a photoelectric conversion element 10 according to an embodiment of the present invention, provided on the interlayer insulating film 30, an interlayer wiring part 32 provided so as to penetrate through the interlayer insulating film 30 for electrically connecting the CMOS transistor substrate 20 and the photoelectric conversion element 10, a sealing layer 40 provided so as to cover the photoelectric conversion element 10, a scintillator 42 provided on the sealing layer 40, a reflective layer 44 provided so as to cover the scintillator 42, and a protective layer 46 provided so as to cover the reflective layer 44.

The CMOS transistor substrate 20 has, in an aspect in accordance with a design, any suitable configuration known conventionally.

The CMOS transistor substrate 20 includes a transistor, a capacitor, and the like formed within the thickness of the substrate, and includes functional elements such as a CMOS transistor circuit (MOS transistor circuit) for achieving various functions.

Examples of the functional elements include a floating diffusion, a reset transistor, an output transistor, and a selection transistor.

With such functional elements, wirings, and the like, a signal reading circuit and the like are built in the CMOS transistor substrate 20.

The interlayer insulating film 30 can be made of any suitable insulating material known conventionally, such as a silicon oxide or an insulating resin. The interlayer wiring part 32 can be made of any suitable conductive material (wiring material) known conventionally, such as copper or tungsten. The interlayer wiring part 32 may be, for example, an in-hole wiring formed simultaneously with the formation of the wiring layer, or a buried plug formed separately from the wiring layer.

The sealing layer 40 can be made of any suitable material known conventionally, on the condition that harmful substances such as oxygen and water, which may possibly functionally deteriorate the photoelectric conversion element 10, can be prevented or kept from permeating. The sealing layer 40 may have the same configuration as the already-described sealing member 17.

The scintillator 42 can be made of any suitable conventionally known material corresponding to the design of the image detection unit 1 for an X-ray imaging device. Examples of suitable materials for the scintillator 42 include inorganic crystals of inorganic materials such as CsI (cesium iodide), NaI (sodium iodide), ZnS (zinc sulfide), GOS (gadolinium oxysulfide), and GSO (gadolinium silicate); organic crystals of organic materials such as anthracene, naphthalene, and stilbene; organic liquids obtained by dissolving organic materials such as diphenyl oxazole (PPO) and terphenyl (TP) in organic solvents such as toluene, xylene, and dioxane; gases such as xenon and helium; and plastics, which can be used.

The constituent elements mentioned above can be arbitrarily suitably arranged to correspond to the designs of the photoelectric conversion element 10 and CMOS transistor substrate 20 on the condition that the scintillator 42 can convert incident X-ray into light with wavelengths mainly in the visible region to generate image data.

The reflective layer 44 reflects the light converted by the scintillator 42. The reflective layer 44 is capable of reducing the loss of the converted light and increasing the detection sensitivity. In addition, the reflective layer 44 is also capable of blocking light that is directly incident from the outside.

The protective layer 46 can be made of any suitable material known conventionally, on the condition that harmful substances such as oxygen and water, which may possibly functionally deteriorate the scintillator 42, can be prevented or kept from permeating.

In this regard, the operation of the image detection unit 1 for an X-ray imaging device, which has the configuration mentioned above, will be briefly described.

When radiation energy such as X-rays and γ-rays is incident on the scintillator 42, the scintillator 42 absorbs the radiation energy and converts the radiation energy into light (fluorescence) with wavelengths from ultraviolet to infrared regions mainly in the visible region. Then, the light converted by the scintillator 42 is received by the photoelectric conversion element 10.

The light received by the photoelectric conversion element 10 via the scintillator 42 as described above is converted by the photoelectric conversion element 10 into an electric signal corresponding to the amount of received light, and is output via the electrode to the outside of the photoelectric conversion element 10 as a received light signal, that is, an electric signal corresponding to the imaging target. The radiation energy (X-ray) to be detected may be incident from any of the side close to the scintillator 42 and the side close to the photoelectric conversion element 10.

Next, the received light signal output from the photoelectric conversion element 10 is input via the interlayer wiring part 32 to the CMOS transistor substrate 20, is read by a signal reading circuit built in the CMOS transistor substrate 20, and is subjected to signal processing by any additional suitable functional unit (not illustrated) known conventionally, thereby generating image information based on the imaging target.

### (Vein Detection Unit)

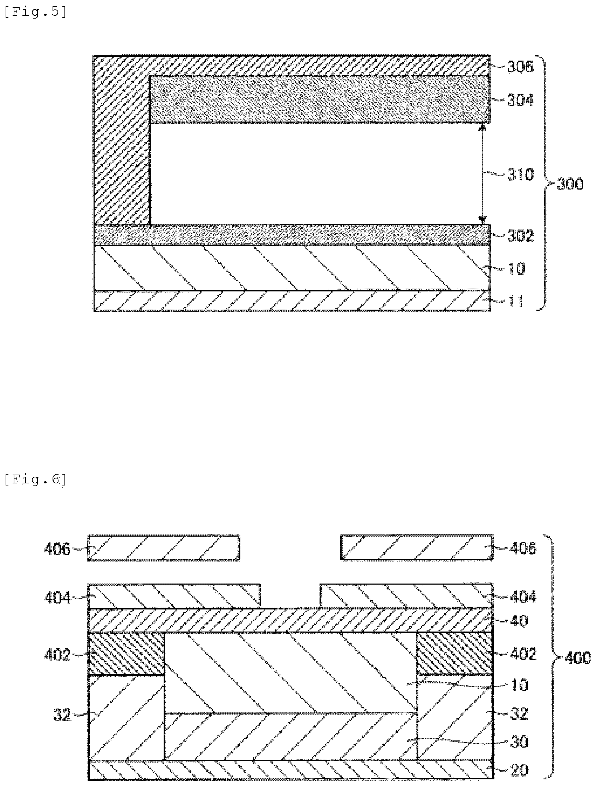

Fig. 5 is a diagram schematically illustrating a configuration example of a vein detection unit for a vein authentication device.

The vein detection unit 300 for a vein authentication device includes a cover part 306 that defines an insertion part 310 into which a finger (e.g., fingertips, fingers, and palms of one or more fingers) as a measurement target is inserted at the time of measurement, a light source part 304 provided in the cover part 306 for irradiating the measurement target with light, a photoelectric conversion element 10 that receives the light emitted from the light source part 304 through the measurement target, a support substrate 11 that supports the photoelectric conversion element 10, and a glass substrate 302 disposed to face the support substrate 11 with the photoelectric conversion element 10 interposed therebetween, and separated from the cover part 306 at a predetermined distance to define the insertion part 306 together with the cover part 306.

While this configuration example shows a transmission-type shooting system in which the light source part 304 is integrated with the cover part 306 so as to be separated in use from the photoelectric conversion element 10 with the measurement target interposed therebetween, the light source part 304 is not necessarily required to be located on the side close to the cover part 306.

On the condition that the measurement target can be efficiently irradiated with light from the light source part 304, for example, a reflection-type shooting system may be employed in which the measurement target is irradiated from the side close to the photoelectric conversion element 10.

The vein detection unit 300 includes the photoelectric conversion element 10 according to the embodiment of the present invention as a functional unit that has an essential function. The vein detection unit 300 may include any suitable member known conventionally, such as a protection film (protection film), a sealing member, a barrier film, a bandpass filter, a near-infrared transmission filter, a visible light cut film, and a finger placing guide, not illustrated, in accordance with an aspect corresponding to such a design that can achieve desired characteristics. For the vein detection unit 300, the configuration of the already described image detection unit 1 can be also employed.

The photoelectric conversion element 10 can be included in accordance with any aspect. For example, a plurality of photoelectric conversion elements 10 may be arranged in a matrix.

The photoelectric conversion element 10 is, as described already, provided on the support substrate 11, and the support substrate 11 is provided with an electrode (anode or cathode) in, for example, a matrix.

The light received by the photoelectric conversion element 10 is converted by the photoelectric conversion element 10 into an electric signal corresponding to the amount of received light, and is output via the electrode to the outside of the photoelectric conversion element 10 as a received light signal, that is, an electric signal corresponding to the imaged vein.

At the time of vein detection (in use), the measurement target may be in contact with or out of contact with the glass substrate 302 on the side closed to the photoelectric conversion element 10.

In this regard, the operation of the vein detection unit 300 will be briefly described.

At the time of vein detection, the vein detection unit 300 detects a vein pattern of a measurement target with the use of light emitted from the light source part 304. Specifically, the light emitted from the light source part 304 is transmitted through the measurement target, and then converted into an electric signal corresponding to the amount of light received by the photoelectric conversion element 10. Then, image information on the vein pattern of the measurement target is made from the converted electric signal.

The vein authentication device compares the obtained image information with previously recorded vein data for vein authentication to perform vein authentication in accordance with any suitable step known conventionally.

### (Image Detection Unit for TOF Type Distance Measuring Device)

Fig. 6 is a diagram schematically illustrating a configuration example of an image detection unit for an indirect TOF-type distance measuring device.

The image detection unit 400 for a TOF-type distance measuring device includes a CMOS transistor substrate 20, an interlayer insulating film 30 provided so as to cover the CMOS transistor substrate 20, a photoelectric conversion element 10 according to an embodiment of the present invention, provided on the interlayer insulating film 30, two floating diffusion layers 402 disposed to be separated from each other so as to sandwich the photoelectric conversion element 10, an insulating layer 40 provided so as to cover the photoelectric conversion element 10 and the floating diffusion layers 402, and two photogates 404 provided on the insulating layer 40 and disposed to be separated from each other. A part of the insulating layer 40 is exposed from the gap between the two photogates 404 separated from each other, and the remaining region thereof is shielded by a light-shielding part 406. The CMOS transistor substrate 20 and the floating diffusion layers 402 are electrically connected by an interlayer wiring part 32 provided so as to penetrate through the interlayer insulating film 30.

In this configuration example, the insulating layer 40 may have any suitable configuration known conventionally, such as a field oxide film made of a silicon oxide.

The photogates 404 can be made of any suitable material known conventionally, such as polysilicon.

The image detection unit 400 for a TOF-type distance measuring device includes the photoelectric conversion element 10 according to the embodiment of the present invention as a functional unit that has an essential function. The image detection unit 400 for a TOF-type distance measuring device can include any suitable member known conventionally, such as a protection film (protection film), a support substrate, a sealing substrate, a sealing member, a barrier film, a bandpass filter, or an infrared cut film (not illustrated) in accordance with an aspect corresponding to such a design that can achieve desired characteristics.

In this regard, the operation of the image detection unit 400 for a TOF-type distance measuring device will be briefly described.

Light is emitted from a light source, the light from the light source is reflected by a measurement target, and the reflected light is received by the photoelectric conversion element 10. With the two photogates 404 provided between the photoelectric conversion element 10 and the floating diffusion layers 402, alternately applying pulses to the photogates 404 causes signal charges generated by the photoelectric conversion element 10 to be transferred to one of the two floating diffusion layers 402, and the charges are accumulated in the floating diffusion layers 402. When light pulses arrive so as to spread equally with respect to the timing of opening the two photogates 404, the two floating diffusion layers 402 become equal in accumulation of charge. When a light pulse arrives at one of the photogates 404 later with respect to the timing at which a light pulse arrives at the other photogate 404, the two floating diffusion layers 402 become different in accumulation of charge.

The difference in accumulation of charge between the floating diffusion layers 402 depends on the delay time of the light pulse. The distance L to the measurement target has a relationship of L = (1/2)ctd with the round-trip time td of light and the velocity c of light, thus allowing the distance to the measurement target to be determined, if the delay time can be estimated from the difference in accumulation of charge between the two floating diffusion layers 402.

The amount of light received by the photoelectric conversion element 10 is converted into an electric signal as a difference in accumulation of change between the two floating diffusion layers 402, and is output to the outside of the photoelectric conversion element 10 as a received light signal, that is, an electric signal corresponding to the measurement target.

Next, the received light signal output from the floating diffusion layers 402 is input via the interlayer wiring part 32 to the CMOS transistor substrate 20, is read by a signal reading circuit built in the CMOS transistor substrate 20, and is subjected to signal processing by any additional suitable functional unit (not illustrated) known conventionally, thereby generating distance information based on the measurement target.

In the process of incorporation into the device according to the application example to which the photoelectric conversion element according to the present embodiment is applied, for example, a heating treatment may be performed, such as a reflow process for mounting on a wiring substrate or the like. For example, in manufacturing an image sensor, a process including a treatment of heating the photoelectric conversion element at a heating temperature of 200°C or higher for approximately 50 minutes may be performed.

For the photoelectric conversion element according to the present embodiment, the already described compound (non-fullerene compound as an n-type semiconductor material) according to the present embodiment and the already described p-type semiconductor material are used as materials for the active layer. Thus, even when a treatment of heating at a heating temperature of 200°C or higher is performed in the step of forming the active layer (details will be described later), in the step of manufacturing the photoelectric conversion element after the formation of the active layer, or in the step of incorporating the manufactured photoelectric conversion element into the image sensor or the biometric authentication device, a decrease in EQE can suppressed, or the EQE can be further improved, and heat resistance can be effectively improved.

Specifically, the EQE is preferably 0.80 or more, more preferably 0.85 or more, still more preferably 1.0 or more as a value (hereinafter, referred to as "EQEₕₑₐₜ/EQE_{100°C}") obtained from normalization by dividing the EQE value in the photoelectric conversion element in which the heating temperature in a post-baking step is changed to a higher temperature, by the value of the EQE as a reference in the photoelectric conversion element for which the heating temperature is 100°C in a pre-baking step, in a step of forming the active layer in a method for manufacturing the photoelectric conversion element.

### 2. Method for Manufacturing Photoelectric Conversion Element

The method for manufacturing the photoelectric conversion element according to the present embodiment is not particularly limited. The photoelectric conversion element according to the present embodiment can be manufactured by combining suitable forming methods with materials selected for forming the constituent elements.

The method for manufacturing the photoelectric conversion element according to the present embodiment can include a step including a treatment of heating at a heating temperature of 200°C or higher. More specifically, the active layer is formed in accordance with a step including a treatment of heating at a heating temperature of 200°C or higher, and/or the method can include a step of heating at a heating temperature of 200°C or higher after the step of forming the active layer.

A method for manufacturing a photoelectric conversion element that has a configuration of a substrate (support substrate), an anode, a hole transport layer, an active layer, an electron transport layer, and a cathode in contact with each other in this order will be described below as an embodiment of the present invention.

### (Step of Preparing Substrate)

In this step, for example, a support substrate provided with an anode is prepared. In addition, a substrate provided with a conductive thin film formed from the already described electrode material is obtained from the market, and if necessary, the conductive thin film is subjected to patterning to form an anode, thereby allowing a support substrate provided with the anode to be prepared.

In the method for manufacturing the photoelectric conversion element according to the present embodiment, the method for forming the anode in the case of forming the anode on the support substrate is not particularly limited. The anode can be formed on the configuration with the anode to be formed (e.g., support substrate, active layer, hole transport layer) by any suitable method known conventionally, such as a vacuum deposition method, a sputtering method, an on plating method, a plating method, or a coating method.

### (Step of Forming Hole Transport Layer)

The method for manufacturing a photoelectric conversion element may include a step of forming the hole transport layer (hole injection layer) provided between the active layer and the anode.

The method for forming the hole transport layer is not particularly limited. From the viewpoint of further simplifying the step of forming the hole transport layer, it is preferable to form the hole transport layer by any suitable coating method known conventionally. The hole transport layer can be formed, for example, by a coating method with the use of a coating liquid including a material for the already described hole transport layer and a solvent, or a vacuum deposition method.

### (Step of Forming Active Layer)

In the method for manufacturing the photoelectric conversion element according to the present embodiment, the active layer is formed on the hole transport layer. The active layer, which is a main constituent element, can be formed in accordance with any suitable forming step known conventionally. In the present embodiment, the active layer is preferably produced by a coating method with the use of an ink (coating liquid).

The step (i) and step (ii) included in the step of forming the active layer, which is a main constituent element for the photoelectric conversion element according to the present invention, will be described below.

### Step (i)

As a method for applying the ink to the coating target, any suitable coating method can be used. As the coating method, a slit coating method, a knife coating method, a spin coating method, a micro-gravure coating method, a gravure coating method, a bar coating method, an inkjet printing method, a nozzle coating method, or a capillary coating method is preferred, a slit coating method, a spin coating method, a capillary coating method, or a bar coating method is more preferred, and a slit coating method or a spin coating method is still more preferred.

The ink for use in the method for manufacturing the photoelectric conversion element according to the present embodiment includes: a composition including a p-type semiconductor material and an n-type semiconductor material, and including the already described compound according to the present embodiment as the n-type semiconductor material; and a solvent.

The ink for the formation of the active layer according to the present embodiment will be described. It is to be noted that the ink for the formation of the active layer according to the present embodiment is an ink for the formation of a bulk heterojunction-type active layer. Accordingly, the ink for the formation of the active layer includes a composition including the already described compound according to the present embodiment as the already described p-type semiconductor material and n-type semiconductor material. The ink for the formation of the active layer according to the present embodiment includes the composition and one type or two or more types of solvents.

The ink for the formation of the active layer according to the present invention, including the p-type semiconductor material and "the compound according to the present embodiment", can suppress a decrease in EQE with respect to a heating treatment in a step of manufacturing a photoelectric conversion element, a step of incorporating the photoelectric conversion element into a device to which the element is applied, or the like, or further improve the EQE, and improve the heat resistance.

The ink for the formation of the active layer according to the present embodiment is not particularly limited on the condition that the active layer can be formed. As the solvent, for example, a mixed solvent can be used, which is obtained by combining a first solvent and a second solvent described later. Specifically, when the ink for the formation of the active layer includes two or more types of solvents, the ink preferably includes a main solvent (first solvent) as a main component and another additive solvent (second solvent) that is added for improved solubility or the like. The first solvent only, however, may be used.

The first solvent and second solvent that can be suitably used for the ink for the formation of the active layer according to the present embodiment, and the combination thereof will be described below.

### (1) First Solvent

The first solvent is preferably a solvent that is capable of dissolving the p-type semiconductor material. The first solvent according to the present embodiment is an aromatic hydrocarbon.

Examples of the aromatic hydrocarbon as the first solvent include a toluene, a xylene (e.g., an o-xylene, a m-xylene, a p-xylene), an o-dichlorobenzene, a trimethylbenzene (e.g., a mesitylene, a 1,2,4-trimethylbenzene (pseudocumene)), a butylbenzene (e.g., an n-butylbenzene, a sec-butylbenzene, a tert-butylbenzene), a methylnaphthalene (e.g., a 1-methylnaphthalene), a tetralin, and an indane.

The first solvent may be composed of one aromatic hydrocarbon, or composed of two or more aromatic hydrocarbons. The first solvent is preferably composed of one aromatic hydrocarbon.

The first solvent is preferably one or more selected from the group consisting of a toluene, an o-xylene, a m-xylene, a p-xylene, a mesitylene, an o-dichlorobenzene, a 1,2,4-trimethylbenzene, an n-butylbenzene, a sec-butylbenzene, a tert-butylbenzene, a methylnaphthalene, a tetralin, and an indane, and more preferably a toluene, an o-xylene, a m-xylene, a p-xylene, an o-dichlorobenzene, a mesitylene, a 1,2,4-trimethylbenzene, an n-butylbenzene, a sec-butylbenzene, a tert-butylbenzene, a methylnaphthalene, a tetralin, or an indane.

### (2) Second Solvent

The second solvent is a solvent selected from the viewpoint of facilitating the implementation of the manufacturing process and further improving the characteristics of the photoelectric conversion element. Examples of the second solvent include ketone solvents such as an acetone, a methyl ethyl ketone, a cyclohexanone, an acetophenone, and a propiophenone, and ester solvents such as an ethyl acetate, a butyl acetate, a phenyl acetate, an ethyl cellosolve acetate, a methyl benzoate, a butyl benzoate, and a benzyl benzoate.

For the second solvent, for example, an acetophenone, a propiophenone, or a butyl benzoate is preferably used from the viewpoint of further reducing the dark current.

### (3) Combination of First Solvent and Second Solvent

Examples of the preferred combination of the first solvent and second solvent include combinations of: a tetralin and an ethyl benzoate; a tetralin and a propyl benzoate; and a tetralin and a butyl benzoate, more preferably a combination of a tetralin and a butyl benzoate.

### (4) Ratio by Weight between First Solvent and Second Solvent

The ratio by weight of the first solvent as the main solvent to the second solvent as the additive solvent (first solvent : second solvent) preferably falls within the range of 85 : 15 to 99 : 1 from the viewpoint of further improving the solubility of the p-type semiconductor material and n-type semiconductor material.

### (5) Any Other Solvent

The solvent may include any other solvent other than the first solvent and the second solvent. When the total weight of all of the solvents included in the ink is determined to be 100% by weight, the content of any other solvent is preferably 5% by weight or less, more preferably 3% by weight or less, still more preferably 1% by weight or less. As any other solvent, a solvent that is higher in boiling point than the second solvent is preferred.

### (6) Optional Components

In addition to the first solvent, the second solvent, the p-type semiconductor material, and the n-type semiconductor material, the ink may contain optional components such as a surfactant, an ultraviolet absorber, an antioxidant, a sensitizer for sensitizing the function of charge generation with absorbed light, and a light stabilizer for increasing stability from ultraviolet light, as long as the object and advantageous effects of the present invention are not impaired.

### (7) Concentrations of p-type Semiconductor Material and n-type Semiconductor Material

The concentrations of the p-type semiconductor material and n-type semiconductor material in the ink (composition) can be any suitable concentration to the extent that the object of the present invention is not impaired, also in consideration of the solubility in the solvent and the like.

The ratio by weight (polymer/non-fullerene compound) of the "p-type semiconductor material" to the "n-type semiconductor material" in the ink (composition) typically falls within in the range of 1/0.1 to 1/10, preferably within the range of 1/0.5 to 1/2, more preferably 1/1.5.

The total concentration of the "p-type semiconductor material" and "n-type semiconductor material" in the ink is typically 0.01% by weight or more, more preferably 0.02% by weight or more, still more preferably 0.25% by weight or more. In addition, the total concentration of the "p-type semiconductor material" and "n-type semiconductor material" in the ink is typically 20% by weight or less, preferably 10% by weight or less, more preferably 7.50% by weight or less.

The concentration of the "p-type semiconductor material" in the ink is typically 0.01% by weight or more, more preferably 0.02% by weight or more, still more preferably 0.10% by weight or more. In addition, the concentration of the "p-type semiconductor material" in the ink is typically 10% by weight or less, more preferably 5.00% by weight or less, still more preferably 3.00% by weight or less.

The concentration of the "n-type semiconductor material" in the ink is typically 0.01% by weight or more, more preferably 0.02% by weight or more, still more preferably 0.15% by weight or more. In addition, the concentration of the "n-type semiconductor material" in the ink is typically 10% by weight or less, more preferably 5% by weight or less, still more preferably 4.50% by weight or less.

### (8) Ink Preparation

The ink can be prepared by a known method. For example, the ink can be prepared by a method of mixing the first solvent, or the first solvent and the second solvent to prepare a mixed solvent, and adding the p-type semiconductor material and the n-type semiconductor material to the obtained mixed solvent, a method of adding the p-type semiconductor material to the first solvent, adding the n-type semiconductor material to the second solvent, and then mixing the first solvent and second solvent with the respective materials added thereto, or the like.

The first solvent and the second solvent may be mixed with the p-type semiconductor material and the n-type semiconductor material, with the first and second solvents and p-type and n-type semiconductor materials heated to a temperature that is equal to or lower than the boiling point of the solvent.

After mixing the first solvent and the second solvent with the p-type semiconductor material and the n-type semiconductor material, the obtained mixture may be filtered with the use of a filter, and the obtained filtrate may be used as. As the filter, for example, a filter formed from a fluororesin such as a polytetrafluoroethylene (PTFE) can be used.

The ink for the formation of the active layer is applied to a coating target selected depending on the photoelectric conversion element and the manufacturing method therefor. The ink for the formation of the active layer can be applied to a functional layer included in the photoelectric conversion element, where the active layer can be present, in the process for manufacturing the photoelectric conversion element. Thus, the target of coating with the ink for the formation of the active layer varies depending on the layer configuration of the photoelectric conversion element to be manufactured and the order of layer formation. For example, when the photoelectric conversion element has a layer configuration of: a substrate; an anode; a hole transport layer; an active layer; an electron transport layer; and a cathode stacked, with the layer listed on the further left side formed earlier, the target of coating with the ink for the formation of the active layer is the hole transport layer. In addition, for example, when the photoelectric conversion element has a layer configuration of: a substrate; a cathode, an electron transport layer; an active layer; a hole transport layer; and an anode stacked, with the layer listed on the further left side formed earlier, the target of coating with the ink for the formation of the active layer is the electron transport layer.

### Step (ii)

As a method for removing the solvent from the coating film of the ink, that is, a method for removing the solvent from the coating film to solidify the coating film, any suitable method can be used. Examples of the method for removing the solvent include a method of directly heating with the use of a hot plate under an inert gas atmosphere such as a nitrogen gas atmosphere, a drying method such as a hot air drying method, a method of drying by infrared heating, a method of drying by flash lamp annealing, and a reduced pressure drying method.

In the method for manufacturing the photoelectric conversion element according to the present embodiment, the step (ii) is a step for volatilizing and then removing the solvent, which is also referred to as a pre-baking step (first heating treatment step).

The conditions for performing the pre-baking step and the post-baking step, that is, the conditions such as the heating temperature and the heating treatment time can be set to provide any suitable condition in consideration of the composition of the ink used, the boiling point of the solvent, and the like.

In the method for manufacturing the photoelectric conversion element according to the present embodiment, specifically, the pre-baking step and the post-baking step can be performed with the use of a hot plate under a nitrogen gas atmosphere, for example.

The heating temperature in the pre-baking step is typically approximately 100°C. In the method for manufacturing the photoelectric conversion element according to the present embodiment, however, the heating temperature in the pre-baking step and/or the post-baking step can be further increased as a result of including, as materials for the active layer, the already described p-type semiconductor material and the already described compound according to the present embodiment as the n-type semiconductor material. Specifically, the heating temperature in the pre-baking step and/or the post-baking step can be preferably 200°C or higher, and further 220°C or higher. The upper limit of the heating temperature is preferably 280°C or lower, more preferably 250°C or lower.

The total heating treatment time in the pre-baking step and the post-baking step can be, for example, 1 hour.

The heating temperature in the pre-baking step and the heating temperature in the post-baking step may be the same temperature or different temperatures.

The heating treatment time can be, for example, 10 minutes or longer. The upper limit of the heating treatment time is not particularly limited, but can be, for example, 4 hours in consideration of takt time and the like.

The thickness of the active layer can be made any suitable desired thickness by appropriately adjusting the solid content concentration in the coating liquid and the conditions of the step (i) and/or the step (ii).

The step of forming the active layer may include other steps besides the step (i) and the step (ii) on the condition that the object and advantageous effects of the present invention are not impaired.

The method for manufacturing the photoelectric conversion element according to the present embodiment may be a method for manufacturing a photoelectric conversion element including multiple active layers, or may be a method in which the step (i) and the step (ii) are repeated multiple times.

### (Step of Forming Electron Transport Layer)

The method for manufacturing the photoelectric conversion element according to the present embodiment includes a step of forming the electron transport layer (electron injection layer) provided on the active layer.

The method for forming the electron transport layer is not particularly limited. From the viewpoint of further simplifying the step of forming the electron transport layer, it is preferable to form the electron transport layer by any suitable vacuum deposition method known conventionally.

### (Step of Forming Cathode)

The method for forming the cathode is not particularly limited. The cathode can be formed on the electron transport layer, for example, from the electrode material exemplified above by any suitable method known conventionally, such as a coating method, a vacuum deposition method, a sputtering method, an ion plating method, or a plating method. Through the foregoing steps, the photoelectric conversion element according to the present embodiment is manufactured.

### (Step of Forming Sealed Body)

According to the present embodiment, any suitable sealing material (adhesive) and substrate (sealing substrate) known conventionally are used for the formation of the sealed body. Specifically, a sealing material such as a UV curable resin, for example, is applied onto the support substrate so as to surround the periphery of the manufactured photoelectric conversion element, the support substrate is then bonded with the sealing material without any gap, and the photoelectric conversion element is then sealed in the gap between the support substrate and the sealing substrate with the use of a method that is suitable for the selected sealing material, such as irradiation with UV light, thereby allowing the sealed body of the photoelectric conversion element to be obtained.

### 3. Method for Manufacturing Image Sensor or Biometric Authentication Device

In particular, the photodetection element, which serves as the photoelectric conversion element according to the present embodiment, can function, with the element incorporated in the image sensor or the biometric authentication device (fingerprint authentication device and vein authentication device) as mentioned above.

Such an image sensor or a biometric authentication device can be manufactured by a manufacturing method including a step including a process of heating the photoelectric conversion element (the sealed body of the photoelectric conversion element) at a heating temperature of 200°C or higher.

Specifically, for performing the step of incorporating the photoelectric conversion element into the image sensor or the biometric authentication device, for example, a reflow step or the like that is performed in mounting on a wiring substrate is performed, thereby allowing for performing a process of heating the photoelectric conversion element at a heating temperature of 200°C or higher, further 220°C or higher. The photoelectric conversion element according to the present embodiment, however, because of the use of the already described n-type semiconductor material as a material for the active layer, can suppress a decrease in the EQE of the incorporated photoelectric conversion element or further improve the EQE thereof, furthermore suppress an increase in dark current or further reduce the dark current, and effectively improve the heat resistance, thus making it possible to improve characteristics such as a detection accuracy in the manufactured image sensor or biometric authentication device.

The heating treatment time can be, for example, 10 minutes or longer. The upper limit of the heating treatment time is not particularly limited, but can be, for example, 4 hours in consideration of takt time and the like.

### [Examples]

Examples will be described below for describing the present invention in more detail. The present invention is not to be considered limited to the examples described below.

In the present examples, the polymer compounds shown in Table 1 (Table 1-1 and Table 1-2) below were used as p-type semiconductor materials (electron-donating compounds), and the compounds shown in Table 2, Table 3 (Table 3-1, Table 3-2, Table 3-3, and Table 3-4) and Table 4 below were used as n-type semiconductor materials (electron-accepting compounds).

### [Table 1-1]

**(Table 1-1)**

| Polymer Compound | Chemical Structure |
|---|---|
| P-1 | 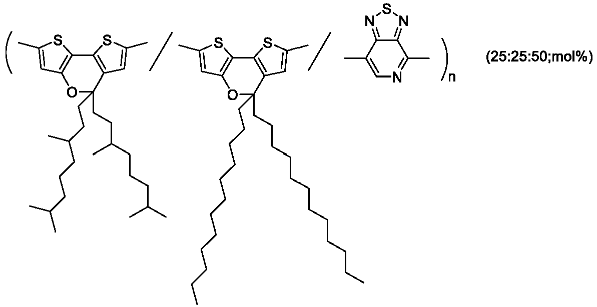 |
| P-2 | 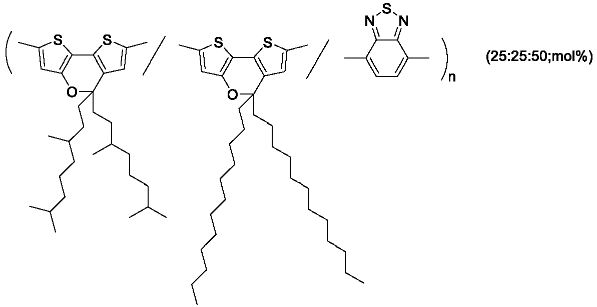 |
| P-3 | 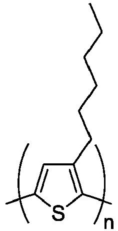 |

### [Table 1-2]

**(Table 1-2)**

| Polymer Compound | Chemical Structure |
|---|---|
| P-4 | 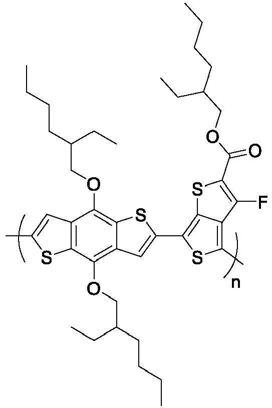 |
| P-5 | 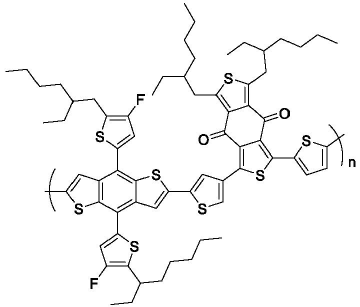 |
| P-6 | 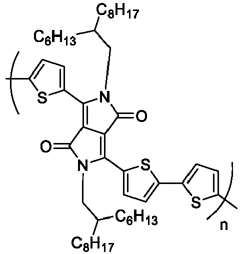 |
| P-7 | 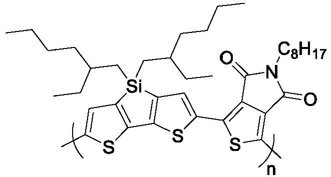 |

### [Table 2]

**(Table 2)**

| Compound | Chemical Structure |
|---|---|
| N-1 | 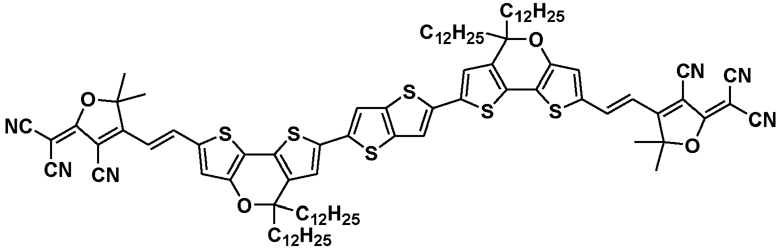 |
| N-2 | 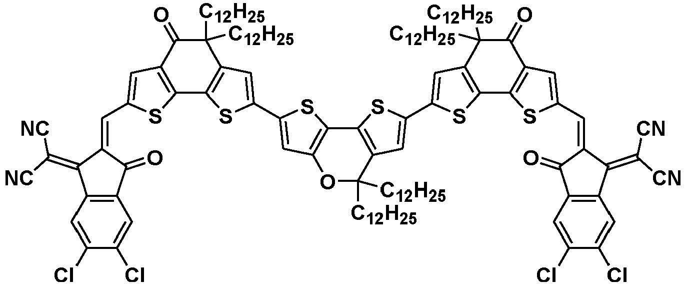 |
| N-3 | 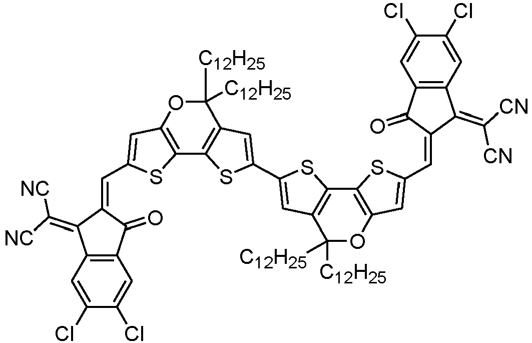 |
| N-4 | 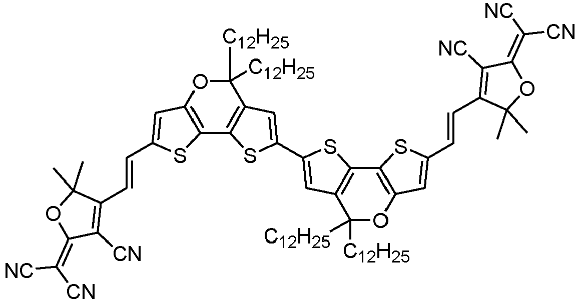 |

### [Table 3-1]

**(Table 3-1)**

| Compound | Chemical Structure |
|---|---|
| N-5 | 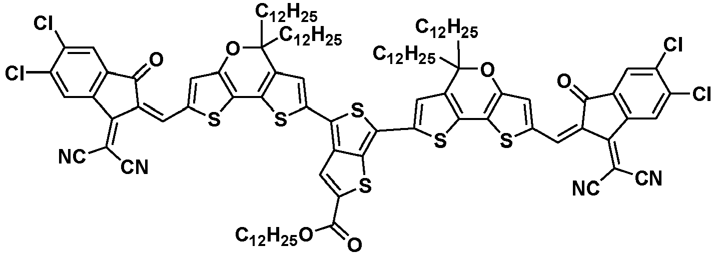 |
| N-6 | 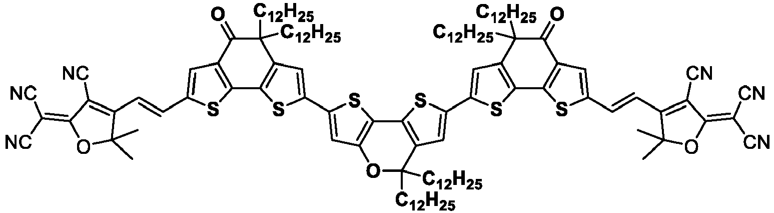 |
| N-7 | 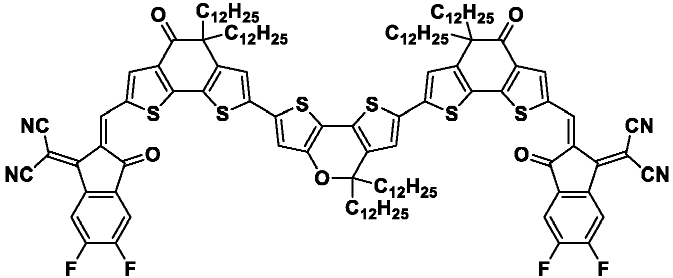 |

### [Table 3-2]

**(Table 3-2)**

| Compound | Chemical Structure |
|---|---|
| N-8 | 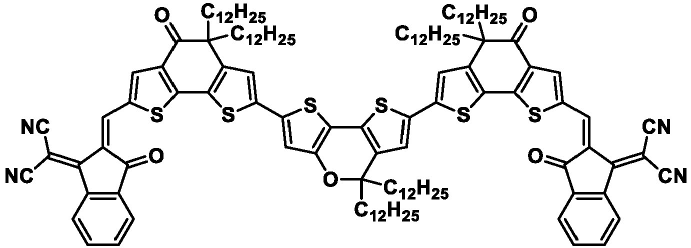 |
| N-9 | 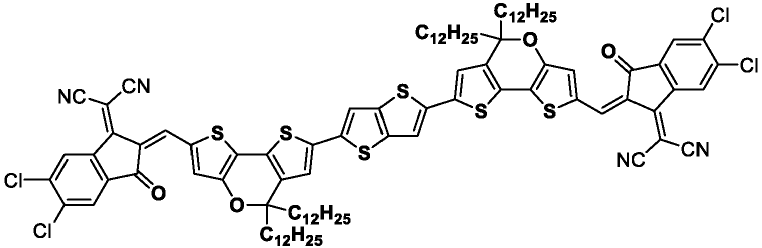 |
| N-10 | 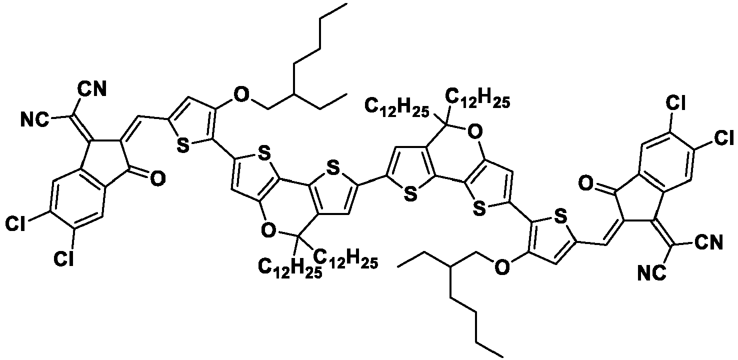 |

### [Table 3-3]

**(Table 3-3)**

| Compound | Chemical Structure |
|---|---|
| N-11 | 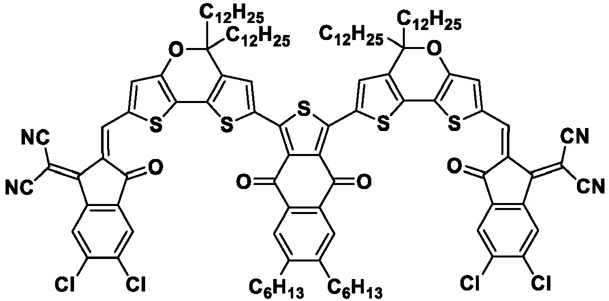 |
| N-12 | 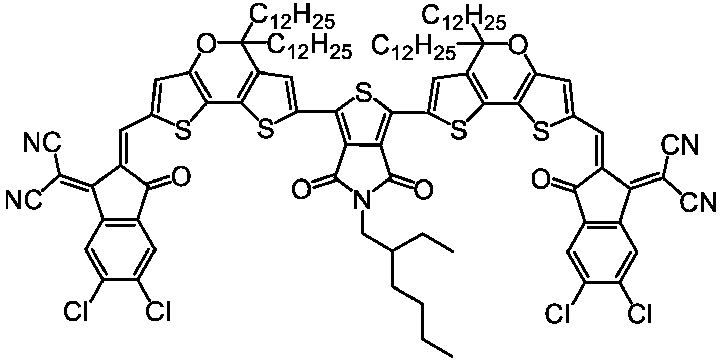 |
| N-13 | 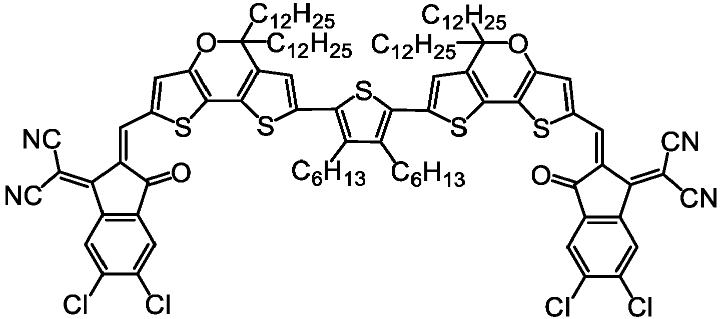 |

### [Table 3-4]

**(Table 3-4)**

| Compound | Chemical Structure |
|---|---|
| N-14 | 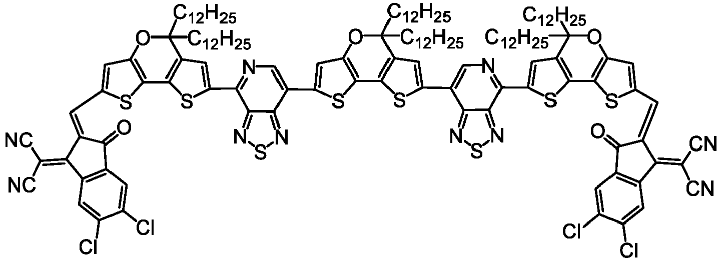 |
| N-15 | 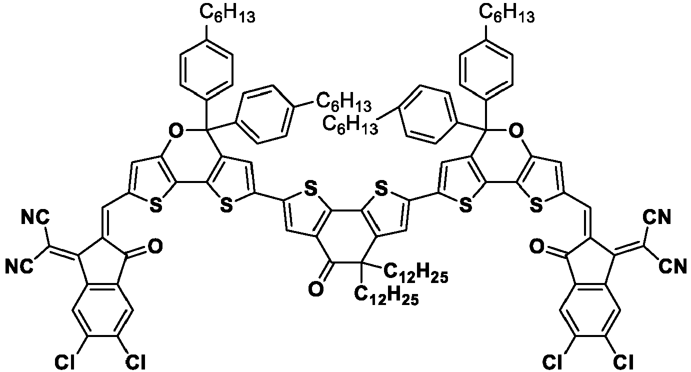 |
| N-16 | 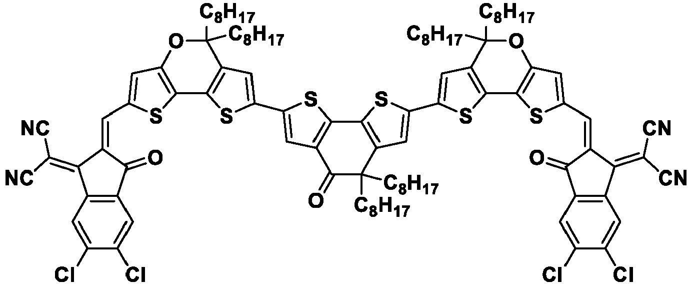 |

### [Table 4]

**(Table 4)**

| Compound | Chemical Structure |
|---|---|
| N-17 | 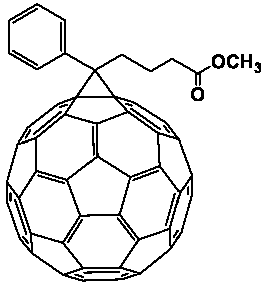 |
| N-18 | 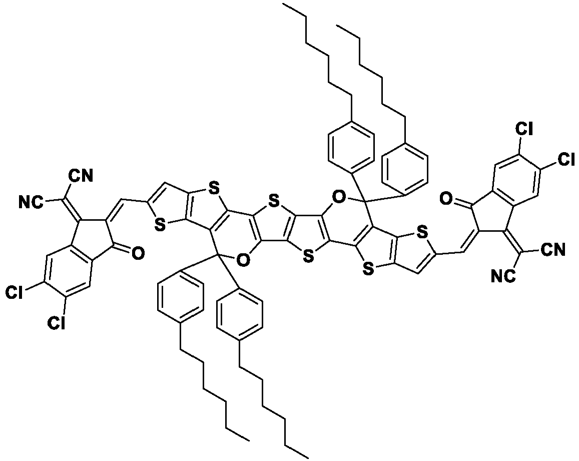 |

The polymer compound P-1 and the polymer compound P-2, which are p-type semiconductor materials, were synthesized with reference to the method described in WO 2011/052709 A, and then used.

For the p-type semiconductor material P-3, P3HT (trade name: manufactured by Sigma-Aldrich Co. LLC) was obtained from the market, and then used.

For the p-type semiconductor material P-4, PTB7 (trade name, manufactured by 1-material Inc.) was obtained from the market, and then used.

For the p-type semiconductor material P-5, PBDB-T-2F (trade name, manufactured by 1-material Inc.) was obtained from the market, and then used.

For the p-type semiconductor material P-6, PDPP3T (trade name: manufactured by Lumtec) was obtained from the market, and then used.

For the p-type semiconductor material P-7, PDTSTPD (trade name, manufactured by 1-material Inc.) was obtained from the market, and then used.

The compounds N-1 to N-16, which are n-type semiconductor materials, were synthesized as in synthesis examples described later, and then used.

For the compound N-17, which is an n-type semiconductor material, E100 (trade name: manufactured by Frontier Carbon Co., Ltd.) was obtained from the market, and then used.

For the compound N-18, which is an n-type semiconductor material, COi8DFIC (trade name, manufactured by 1-material Inc.) was obtained from the market, and then used.

### <Synthesis Example 1> (Synthesis of Compound 3)

A compound 1 and a compound 2 represented by the following formula were used to synthesize a compound 3 represented by the following formula.

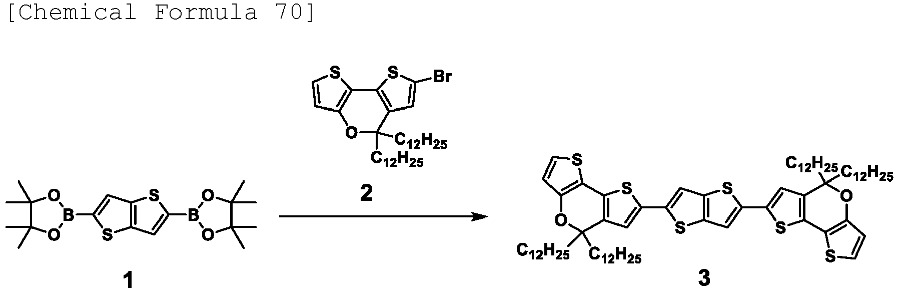

In a 500 mL four-necked flask whose internal atmosphere was replaced with a nitrogen gas, 7.61 g (19.4 mmol) of the compound 1, 24.8 g (40.7 mmol) of the compound 2 synthesized by the method described in paragraph [0335] of WO 2011/052709 A, and 200 mL of a tetrahydronaphthalene were put, and subjected to degassing by bubbling with an argon gas for 30 minutes.

Then, in the four-necked flask, 0.88 g (0.97 mmol, 5 mol%) of tris(dibenzylideneacetone) dipalladium (0), 1.18 g (3.88 mmol, 20 mol%) of a tri-tert-butylphosphonium tetrafluoroborate, and 60 mL of a tetrahydronaphthalene were put, and stirred for 5 minutes.

Next, 65 mL of a 3.0 M aqueous potassium phosphate solution was added to the four-necked flask, and the reaction liquid was stirred on heating for 3 hours in an oil bath at a set temperature of 50°C.

After cooling the reaction liquid, 100 mL of water and 100 mL of hexane were added to the four-necked flask, and the organic layer was separated and washed with water three times and with a saturated aqueous sodium chloride solution one time.

The obtained solution was dried with the use of an anhydrous sodium sulfate, and then filtered, and the solvent was distilled away under reduced pressure. The obtained crude product was purified with a silica gel column to obtain 21.1 g (17.6 mmol, 90.8% in yield) of the compound 3 as an orange solid.

The obtained compound 3 was analyzed for an NMR spectrum. Here is the result.
[¹H NMR (CDCl₃)]

δ 7.23 (s, 2H), 7.00 (d, J_{HH}=4.8Hz, 2H), 6.78 (s, 2H), 6.68 (d, J_{HH}=4.8Hz, 2H), 1.88 (m, 8H), 1.26 (br, 80H), 0.87 (t, 12H) .

### <Synthesis Example 2> (Synthesis of Compound 4)

From the compound 3 represented by the following formula, a compound 4 represented by the following formula was synthesized.

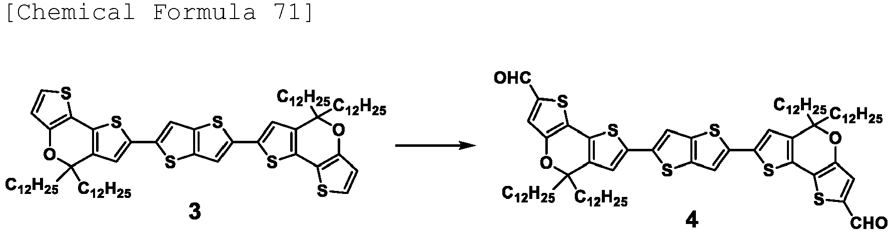

In a 100 mL three-necked flask whose internal atmosphere was replaced with a nitrogen gas, 3.59 g (3.00 mmol) of the compound 3 and 30 mL of a dichloromethane were put, and stirred at room temperature for 10 minutes to dissolve the compound 3.

Next, in the three-necked flask, 1.17 g (9.00 mmol) of a (chloromethylene)dimethyliminium chloride was put, and stirred on heating for 2 hours in an oil bath at a set temperature of 45°C.

Then, after cooling the reaction liquid, 20 mL of water was added thereto, and the organic layer was separated and washed two times with a saturated aqueous sodium chloride solution. The obtained solution was dried with the use of an anhydrous sodium sulfate, and then subjected to filtration, and the solvent was distilled away under reduced pressure.

The obtained crude product was purified with a silica gel column to obtain 3.52 g (2.81 mmol, 93.6% in yield) of the compound 4 as a dark red solid.

The obtained compound 4 was analyzed for an NMR spectrum. Here is the result.

### [¹H NMR (CDCl₃)]

δ 9.77 (s, 2H), 7.32 (s, 2H), 7.27 (s, 2H), 6.83 (s, 2H), 1.90 (m, 8H), 1.23 (br, 80H), 0.86 (t, 12H).

### <Example 1> (Synthesis of Compound N-1)

The compound 4 and compound 5 represented by the following formula were used to synthesize a compound 6 (compound N-1) represented by the following formula.

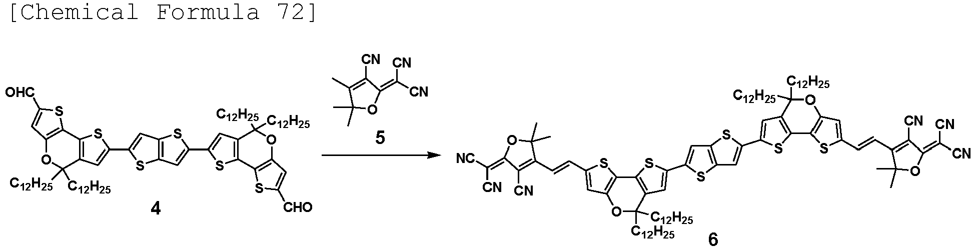

In a 100 mL three-necked flask whose internal atmosphere was replaced with a nitrogen gas, 1.25 g (1.00 mmol) of the compound 4, 0.797 g (4.00 mmol) of the compound 5 synthesized by the method described in Synthetic Communications, 1995, 25 (19), 3045, 40 mL of a chloroform, and 2.02 g (20.0 mmol) of a pyridine were put, and stirred on heating for 16 hours in an oil bath at 65°C.

The reaction liquid was cooled, water was further added thereto, and the organic layer was then washed with the use of an aqueous ammonium chloride solution and a saturated aqueous sodium chloride solution. The obtained organic layer was dried with a magnesium sulfate, and then subjected to filtration, and the solvent was distilled away under reduced pressure.

The obtained crude product was purified with a silica gel column to obtain 654 mg (0.405 mmol, 40.5% in yield) of the compound 6 (compound N-1) as a dark green solid.

The obtained compound N-1 was analyzed for an NMR spectrum. Here is the result.
[¹H NMR (CDCl₃)]

δ 7.67 (d, J_{HH}=15.6Hz, 2H), 7.36 (s, 2H), 7.03 (s, 2H), 6.86 (s, 2H), 6.61 (d, J_{HH}=15.6Hz, 2H), 1.90 (br, 8H), 1.75 (s, 12H), 1.26 (br, 80H), 0.86 (t, 12H).

### <Synthesis Example 3> (Synthesis of Compound 8)

From a compound 7 represented by the following formula, a compound 8 represented by the following formula was synthesized.

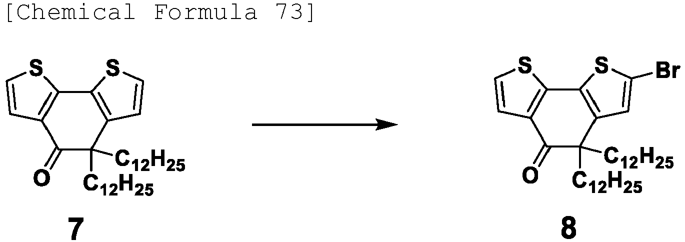

In a 1 L four-necked flask whose internal atmosphere was replaced with a nitrogen gas, 13.30 g (24.50 mmol) of the compound 7 synthesized by the method described in paragraph [0203] of JP-A-2014-31364 and 490 mL of a tetrahydrofuran were put, and cooled down to 0°C.

Next, to the four-necked flask, 4.361 g (24.50 mmol) of an N-bromosuccinimide was added, and stirred at 0°C for 4 hours.

Then, the temperature of the obtained solution was increased up to ordinary temperature, and the solution was reacted by further stirring for 3 hours at ordinary temperature, and a 3% aqueous sodium sulfite solution was then further added to stop the reaction.

The obtained reaction liquid was extracted with hexane, and then washed with water and a saturated aqueous sodium chloride solution.

Next, the obtained organic layer was dried with a magnesium sulfate, and then subjected to filtration, and the solvent was further distilled away under reduced pressure.

The obtained crude product was purified with a silica gel column to obtain 10.1 g (16.2 mmol, 66.3% in yield) of the compound 8 as a light yellow oil.

The obtained compound 8 was analyzed for an NMR spectrum. Here is the result.
[¹H NMR (CDCl₃)]

δ 7.44 (d, J_{HH}=5.6Hz, 1H), 7.07 (d, J_{HH}=5.6Hz, 1H), 6.95 (s, 1H), 2.16 (m, 2H), 1.72 (m, 2H), 1.21 (br, 40H), 0.86 (t, 6H).

### <Synthesis Example 4> (Synthesis of Compound 10)

The compound 8 and compound 9 represented by the following formula were used to synthesize a compound 10 represented by the following formula.

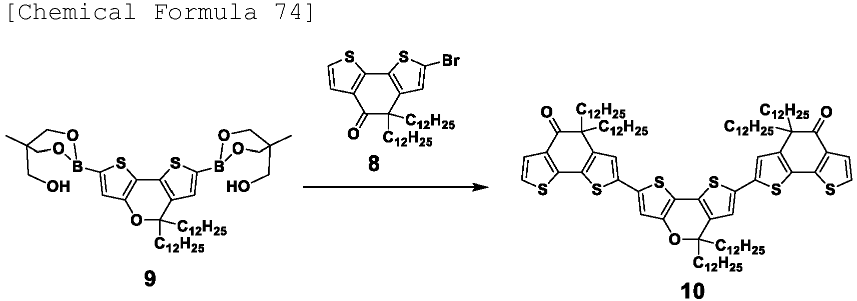

In a 300 mL four-necked flask whose internal atmosphere was replaced with a nitrogen gas, 5.23 g (6.65 mmol) of the compound 9 synthesized by the method described in paragraphs [0139] to [0141] of WO 2011/052709 A, 9.51 g (15.3 mmol) of the compound 8, and 120 mL of a tetrahydronaphthalene were put, and subjected to degassing by bubbling with an argon gas for 30 minutes.

Next, in the four-necked flask, 0.304 g (0.333 mmol, 5 mol%) of tris(dibenzylideneacetone) dipalladium (0), 0.405 g (1.33 mmol, 20 mol%) of a tri-tert-butylphosphonium tetrafluoroborate, and 13 mL of a tetrahydronaphthalene were put, and stirred for 5 minutes.

Then, 22 mL of a 3.0 M aqueous potassium phosphate solution was further added to the four-necked flask, and the reaction liquid was stirred on heating for 1 hour in an oil bath at a set temperature of 75°C.

After cooling the reaction liquid, 100 mL of water and 100 mL of hexane were added, and the reaction liquid was separated and washed with water three times and with a saturated aqueous sodium chloride solution one time.

The obtained solution was dried with an anhydrous sodium sulfate, and then subjected to filtration, and the solvent was distilled away under reduced pressure.

The obtained crude product was purified with a silica gel column to obtain 9.83 g (6.09 mmol, 91.7% in yield) of the compound 10 as a dark red oil.

The obtained compound 10 was analyzed for an NMR spectrum. Here is the result.

### [¹H NMR (CDCl₃)]

δ 7.46 (d, J_{HH}=5.2Hz, 1H), 7.45 (d, J_{HH}=5.2Hz, 1H), 7.07 (d, J_{HH}=5.2Hz, 1H), 7.06 (d, J_{HH}=5.2Hz, 1H), 7.02 (s, 1H), 6.98 (s, 1H), 6.86 (s, 1H), 6.84 (s, 1H), 2.19 (m, 4H), 1.91 (m, 4H), 1.73 (m, 4H), 1.18 (br, 120H), 0.86 (t, 18H) .

### <Synthesis Example 5> (Synthesis of Compound 11)

From the compound 10 represented by the following formula, a compound 11 represented by the following formula was synthesized.

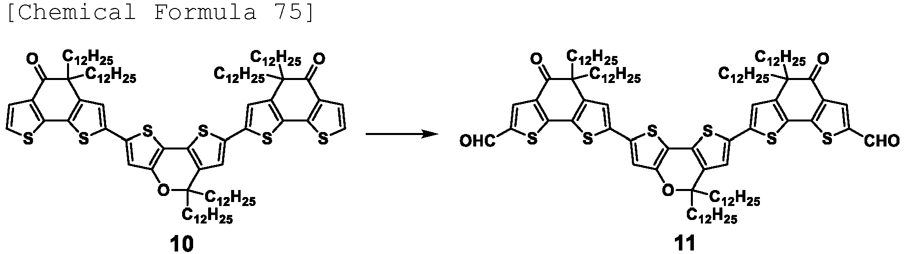

In a 300 mL four-necked flask whose internal atmosphere was replaced with a nitrogen gas, 9.68 g (6.00 mmol) of the compound 10 and 120 mL of a chloroform were put, and stirred at ordinary temperature for 10 minutes to dissolve the compound 10.

Next, to the four-necked flask, 5.38 g (42.0 mmol) of a (chloromethylene)dimethyliminium chloride was put, and stirred on heating for 20 hours in total in an oil bath at a set temperature of 65°C.

After cooling the reaction liquid, 100 mL of water was added thereto, and the reaction liquid was separated and washed two times with a saturated aqueous sodium chloride solution.

The obtained solution was dried with an anhydrous sodium sulfate, and then filtered, and the solvent was further distilled away under reduced pressure.

The obtained crude product was purified with a silica gel column to obtain 5.90 g (3.53 mmol, 58.9% in yield) of the compound 11 as a purple oil.

The obtained compound 11 was analyzed for an NMR spectrum. Here is the result.
[¹H NMR (CDCl₃)]

δ 9.91 (s, 2H), 8.08 (s, 1H), 8.07 (s, 1H), 7.06 (s, 1H), 7.02 (s, 1H), 6.93 (s, 1H), 6.91 (s, 1H), 2.22 (m, 4H), 1.92 (m, 4H), 1.75 (m, 4H), 1.21 (br, 120H), 0.85 (t, 18H) .

### <Example 2> (Synthesis of Compound N-2)

The compound 11 and compound 12 represented by the following formula were used to synthesize a compound 13 (compound N-2) represented by the following formula.

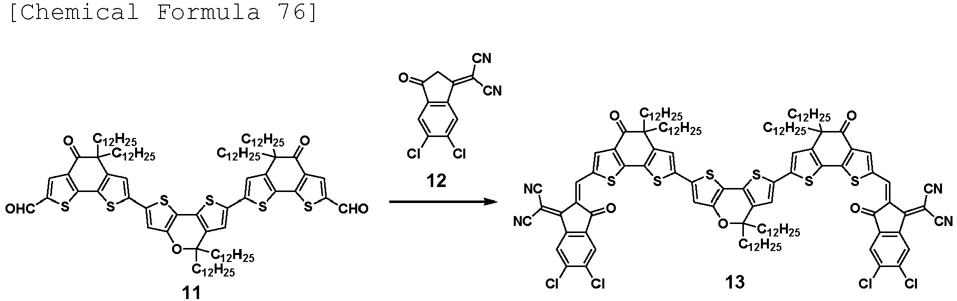

In a 200 mL four-necked flask whose internal atmosphere was replaced with a nitrogen gas, 2.25 g (1.35 mmol) of the compound 11, 0.888 g (3.38 mmol) of the compound 12 synthesized by the method described in Adv. Mater. 2017, 29, and 1703080., 68 mL of a chloroform, and 1.07 g (13.5 mmol) of pyridine were put, and stirred on heating for 2 hours in an oil bath at 65°C.

The obtained solution was cooled down to ordinary temperature, and water was added to the solution to stop the reaction. The obtained solution was extracted with a chloroform, and then washed two times with water and one time with a saturated aqueous sodium chloride solution.

Next, the obtained organic layer was dried with a magnesium sulfate, and subjected to filtration, and the solvent was further distilled away under reduced pressure.

The obtained crude product was purified with a silica gel column to obtain 1.91 g (0.886 mmol, 65.6% in yield) of the compound 13 (compound N-2) as a dark blue-green solid.

The obtained compound N-2 was analyzed for an NMR spectrum. Here is the result.
[¹H NMR (CDCl₃)]

δ 8.90 (s, 1H), 8.89 (s, 1H), 8.81 (s, 1H), 8.17 (s, 1H), 7.99 (s, 1H), 7.98 (s, 1H), 7.11 (s, 1H), 7.07 (s, 1H), 6.98 (s, 1H), 6.98 (s, 1H), 2.25 (m, 4H), 1.94 (m, 4H), 1.77 (m, 4H), 1.24 (br, 120H), 0.86 (m, 18H).

### <Synthesis Example 6> (Synthesis of Compound 14)

From the compound 2 represented by the following formula, a compound 14 represented by the following formula was synthesized.

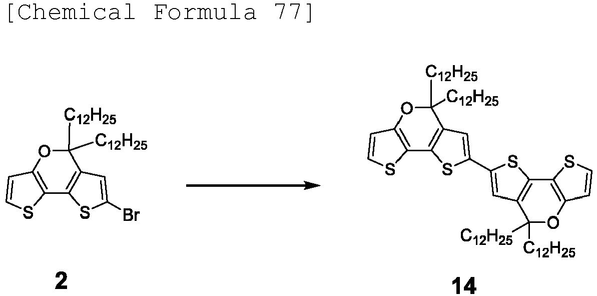

In a 200 mL four-necked flask whose internal atmosphere was replaced with a nitrogen gas, 8.2 g (13.4 mmol) of the compound 2 and 67 mL of a tetrahydrofuran were put, and cooled to 0°C in an ice bath.

Next, into the four-necked flask, 6.7 mL of a tetrahydrofuran solution (2.0 M) of iPrMgCl was slowly delivered by drops, and stirred for 1 hour while keeping the temperature at 0°C.

Subsequently, in another vessel, 8.2 g (13.4 mmol) of the compound 2 diluted with 67 mL of a tetrahydrofuran and a solution of 363.2 mg (0.67 mmol) of a [1,3-bis(diphenylphosphino)propane] nickel (II) dichloride were slowly delivered by drops at 0°C into the obtained reaction liquid, and the temperature of the reaction solution was then increased up to ordinary temperature. Thereafter, after stirring for 2 hours at ordinary temperature, the reaction was stopped with the use of a 0.5 M hydrochloric acid.

The obtained solution was extracted with a chloroform, and then washed two times with water and one time with a saturated aqueous sodium chloride solution.

The obtained organic layer was dried with a magnesium sulfate, and subjected to filtration, and the solvent was distilled away under reduced pressure.

The obtained crude product was purified with a silica gel column to obtain 2.14 g (4.04 mmol, 15% in yield) of the compound 14 as an orange solid.

The obtained compound 14 was analyzed for an NMR spectrum. Here is the result.
[¹H NMR (CDCl₃)]

δ 6.98 (d, J_{HH} = 5.6Hz, 2H), 6.69 (s, 2H), 6.67 (d, J_{HH} = 5.6Hz, 2H), 1.88-1.84 (m, 8H), 1.30-1.19 (m, 80H), 0.87 (t, 12H) .

### <Synthesis Example 7> (Synthesis of Compound 15)

From the compound 14 represented by the following formula, a compound 15 represented by the following formula was synthesized.

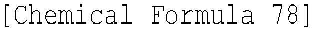

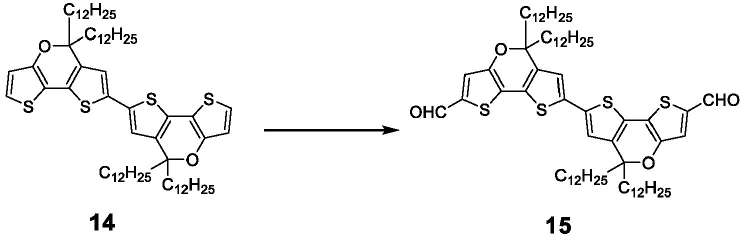

In a 100 mL four-necked flask whose internal atmosphere was replaced with a nitrogen gas, 2.0 g (1.89 mmol) of the compound 14 and 19 mL of a dichloromethane were put to dissolve the compound 14.

Next, to the four-necked flask, 0.72 g (5.66 mmol) of a (chloromethylene)dimethyliminium chloride was put, and stirred on heating for 3 hours in an oil bath at a set temperature of 40°C.

After cooling the reaction liquid, 20 mL of water was added thereto, and the reaction liquid was separated and washed two times with a saturated aqueous sodium chloride solution.

The obtained organic layer was dried with a magnesium sulfate, and then subjected to filtration, and the solvent was further distilled away under reduced pressure.

The obtained crude product was purified with a silica gel column to obtain 1.83 g (1.64 mmol, 87% in yield) of the compound 15 as a purple solid.

The obtained compound 15 was analyzed for an NMR spectrum. Here is the result.
[¹H NMR (THF-d₈)]

δ 9.73 (s, 2H), 7.37 (s, 2H), 7.10 (s, 2H), 1.98-1.86 (m, 8H), 1.44-1.23 (m, 80H), 0.84 (t, 12H).

### <Example 3> (Synthesis of Compound N-3)

The compound 12 and compound 15 represented by the following formula were used to synthesize a compound 16 represented by the following formula.

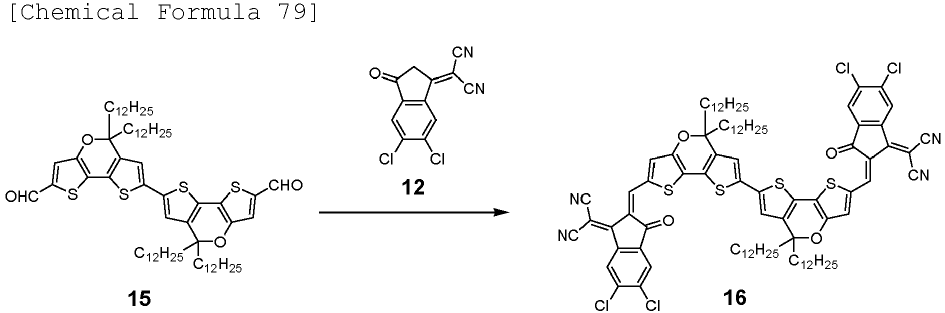

In a 100 mL four-necked flask whose internal atmosphere was replaced with a nitrogen gas, 0.6 g (0.54 mmol) of the compound 15, 0.35 g (1.34 mmol) of the compound 12, 8 mL of a chloroform, and 0.02 g (0.27 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was further distilled away under reduced pressure.

The obtained crude product was purified with a silica gel column to obtain 0.69 mg (0.47 mmol, 87% in yield) of the compound 16 (compound N-3) as a dark blue-green solid.

The obtained compound N-3 was analyzed for an NMR spectrum. Here is the result.
[¹H NMR (CDCl₃)]

δ 8.74 (s, 4H), 7.91 (s, 2H), 7.39 (s, 2H), 6.92 (s, 2H) 1.99-1.88 (m, 8H), 1.43-1.23 (m, 80H), 0.85 (t, 12H).

### <Example 4> (Synthesis of Compound N-4)

The compound 5 and compound 15 represented by the following formula were used to synthesize a compound 17 (compound N-4) represented by the following formula.

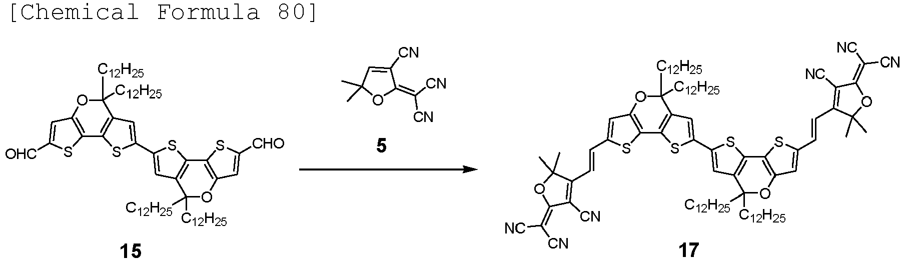

In a 100 mL three-necked flask whose internal atmosphere was replaced with a nitrogen gas, 0.6 g (0.54 mmol) of the compound 15, 0.43 g (2.15 mmol) of the compound 5, 8 mL of a chloroform, and 0.85 g (10.8 mmol) of a pyridine were put, and stirred on heating for 7 hours in an oil bath at 65°C.

The obtained reaction liquid was cooled, water was further added thereto, and the organic layer was then washed with an aqueous ammonium chloride solution and a saturated aqueous sodium chloride solution.

Next, the organic layer was dried with a magnesium sulfate, and subjected to filtration, and the solvent was further distilled away under reduced pressure.

The obtained crude product was purified with a silica gel column to obtain 243 mg (0.18 mmol, 34% in yield) of the compound 17 (compound N-4) as a dark blue solid.

The obtained compound N-4 was analyzed for an NMR spectrum. Here is the result.
[¹H NMR (CDCl₃)]

δ 7.63 (d, J_{HH} = 16Hz, 2H), 7.03 (s, 2H), 6.83 (s, 2H), 6.63 (d, J_{HH} = 16Hz, 2H), 1.95-1.85 (m, 8H), 1.75 (s, 12H), 1.40-1.23 (m, 80H), 0.86 (t, 12H).

### <Synthesis Example 8> (Synthesis of Compound 19)

From the compound 18 represented by the following formula, a compound 19 represented by the following formula was synthesized.

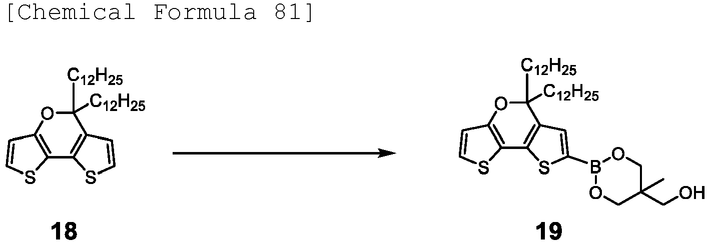

Into a 100 mL three-necked flask whose internal atmosphere was replaced with a nitrogen gas, 2.85 g (5.36 mmol) of the compound 18 synthesized by the method described in paragraph [0271] of WO 2011/052709 A, and 27 mL of a dehydrated tetrahydrofuran were added, and the obtained solution was cooled down to -70°C, and then with 3.3 mL of an n-butyllithium solution (1.64 mol/L, hexane solution) added thereto, stirred for 2 hours.

Next, with the reaction liquid kept at -70°C, 0.83 g (8.04 mmol) of a trimethoxyborane was added thereto, and stirred for 2 hours.

Then, a 10 w% acetic acid aqueous solution (30 mL) was put into the obtained reaction liquid, and a separation operation was performed with the use of an ethyl acetate to extract the organic layer. The obtained organic layer was, with toluene (20 mL) and 1.29 g (10.72 mmol) of a 2-hydroxymethylene-2-methyl-1,3-propanediol added thereto, subjected to a dehydration operation with a Dean-Stark tube used, for 30 minutes. Furthermore, the solvent was removed with a rotary evaporator, thereby providing, as a green oil, 3.53 g of a crude product for the compound 19.

### <Synthesis Example 9> (Synthesis of Compound 21)

The compound 19 and compound 20 represented by the following formula were used to synthesize a compound 21 represented by the following formula.

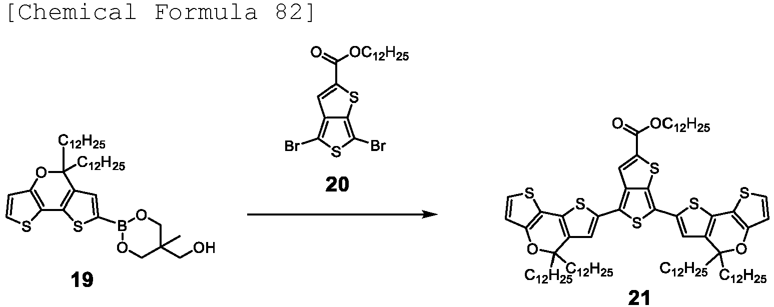

In a 100 mL four-necked flask whose internal atmosphere was replaced with a nitrogen gas, 3.53 g (5.36 mmol) of the compound 19, which was the unpurified crude product obtained in Synthesis Example 8, 1.2 g (2.23 mmol) of the compound 20 purchased from Luminescence Technology Corp., and 22 mL of a tetrahydrofuran were put, and subjected to degassing by bubbling with an argon gas for 30 minutes.

To the obtained reaction liquid, 0.164 g (0.179 mmol, 8 mol%) of tris(dibenzylideneacetone) dipalladium (0) and 0.109 g (0.357 mmol, 16 mol%) of a tri-tert-butylphosphonium tetrafluoroborate were put, and stirred for 5 minutes.

Next, 22 mL of a 3.0 M aqueous potassium phosphate solution was further added to the reaction liquid, and the obtained reaction liquid was stirred on heating for 1 hour in an oil bath at a set temperature of 75°C.

Then, after cooling the reaction liquid, 100 mL of water and 100 mL of hexane were added thereto, and the reaction liquid was separated and washed with water three times and with a saturated aqueous sodium chloride solution one time.

The obtained solution was dried with an anhydrous sodium sulfate, and then filtered, and the solvent was distilled away under reduced pressure. The obtained crude product was purified with a silica gel column to obtain 2.11 g (1.49 mmol, 67% in yield) of the compound 21 as a dark red oil.

The obtained compound 21 was analyzed for an NMR spectrum. Here is the result.
[¹H NMR (CDCl₃)]

δ 7.94 (s, 1H), 7.03 (dd, J_{HH} = 9.2Hz, 5.2Hz, 2H), 6.85 (s, 1H), 6.81 (s, 1H), 6.68 (dd, J_{HH} = 5.2Hz, 1.6Hz, 2H), 4.37 (t, J_{HH} = 6.8Hz, 2H), 1.95-1.78 (m, 10H), 1.44-1.22 (m, 100H), 0.86 (t, 15H).

### <Synthesis Example 10> (Synthesis of Compound 22)

From the compound 21 represented by the following formula, a compound 22 represented by the following formula was synthesized.

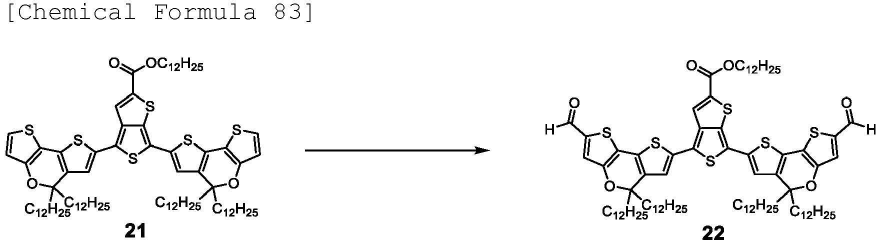

In a 100 mL four-necked flask whose internal atmosphere was replaced with a nitrogen gas, 1.5 g (1.06 mmol) of the compound 21 and 11 mL of a dichloromethane were added to dissolve the compound 21.

Next, in the obtained reaction liquid, 0.41 g (3.19 mmol) of a (chloromethylene)dimethyliminium chloride was put, and heated and stirred for 3 hours in an oil bath at a set temperature of 40°C.

Then, after cooling the obtained reaction liquid, 20 mL of water was added thereto, and the reaction liquid was separated and washed two times with a saturated aqueous sodium chloride solution.

The crude product obtained by drying the obtained solution with a magnesium sulfate, then filtering the dried solution, and distilling away the solvent under reduced pressure, and the resulting was purified with a silica gel column to obtain 1.44 g (0.98 mmol, 92% in yield) of the compound 22 as a purple solid.

The obtained compound 22 was analyzed for an NMR spectrum. Here is the result.
[¹H NMR (CDCl₃)]

δ 9.78 (s, 1H), 9.77 (s, 1H), 7.95 (s, 1H), 7.29 (s, 1H), 7.28 (s, 1H), 6.90 (s, 1H), 6.86 (s, 1H), 4.39 (t, J_{HH} = 7.2Hz, 2H), 1.97-1.79 (m, 10H), 1.46-1.22 (m, 100H), 0.86 (t, 15H).

### <Example 5> (Synthesis of Compound N-5)

The compound 22 and compound 12 represented by the following formula were used to synthesize a compound 23 (compound N-5) represented by the following formula.

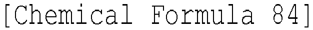

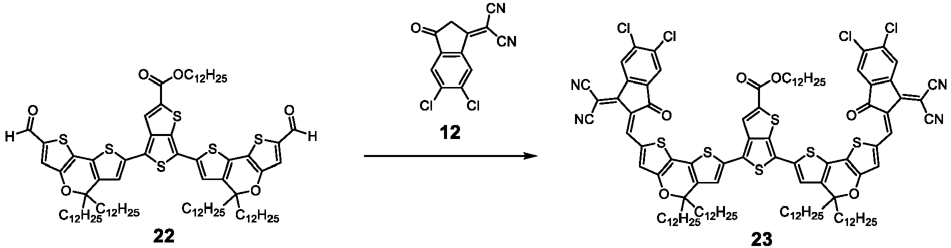

In a 100 mL four-necked flask whose internal atmosphere was replaced with a nitrogen gas, 1.4 g (0.98 mmol) of the compound 22, 0.78 g (2.95 mmol) of the compound 12, 20 mL of a chloroform, and 0.04 g (0.49 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was distilled away under reduced pressure. The obtained crude product was purified with a silica gel column to obtain 1.5 g (0.77 mmol, 95% in yield) of the compound 23 (compound N-5) as a black solid.

The obtained compound N-5 was analyzed for an NMR spectrum. Here is the result.
[¹H NMR (CDCl₃)]

δ 8.57-8.51 (m, 4H), 7.81 (s, 1H), 7.77 (s, 1H), 7.73 (s, 1H), 7.29 (br, 2H), 6.96 (s, 1H), 6.88 (s, 1H), 4.50 (t, J_{HH} = 7.2Hz, 2H), 2.08-1.93 (m, 10H), 1.59-1.20 (m, 100H), 0.83 (t, 15H).

### <Example 6> (Synthesis of Compound N-6)

The compound 11 and compound 5 represented by the following formula were used to synthesize a compound 24 (compound N-6) represented by the following formula.

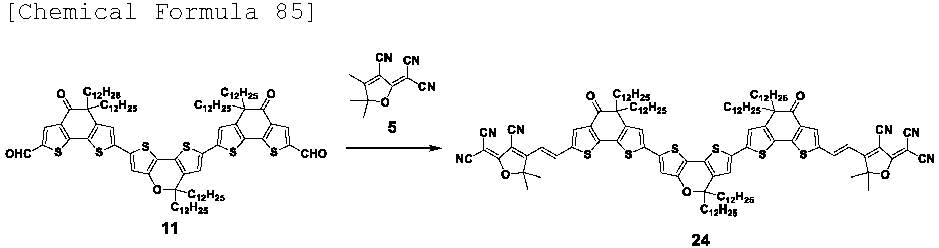

In a 200 mL four-necked flask whose internal atmosphere was replaced with a nitrogen gas, 1.67 g (1.00 mmol) of the compound 11, 0.797 g (4.00 mmol) of the compound 5, 50 mL of a chloroform, and 2.02 g (20.0 mmol) of a triethylamine were put, and stirred on heating for 8 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was distilled away under reduced pressure. The obtained crude product was purified with a silica gel column to obtain 1.52 g (0.749 mmol, 74.0% in yield) of the compound 24 (compound N-6) as a dark blue-green solid.

The obtained compound N-6 was analyzed for an NMR spectrum. Here is the result.
[¹H NMR (CDCl₃)]

δ 7.783 (s, 1H), 7.779 (s, 1H), 7.72 (d, J_{HH} = 7.2Hz, 1H), 7.68 (d, J_{HH} = 7.2Hz, 1H), 7.07 (s, 1H), 7.04 (s, 1H), 6.94 (d, J_{HH} = 7.2Hz, 2H), 7.07 (s, 1H), 6.71 (s, 1H), 6.69 (s, 1H), 2.20 (m, 4H), 1.94 (m, 4H), 1.79 (s, 12H)1.75 (m, 4H), 1.24 (br, 120H), 0.86 (m, 18H).

### <Example 7> (Synthesis of Compound N-7)

The compound 11 and compound 25 represented by the following formula were used to synthesize a compound 26 (compound N-7) represented by the following formula.

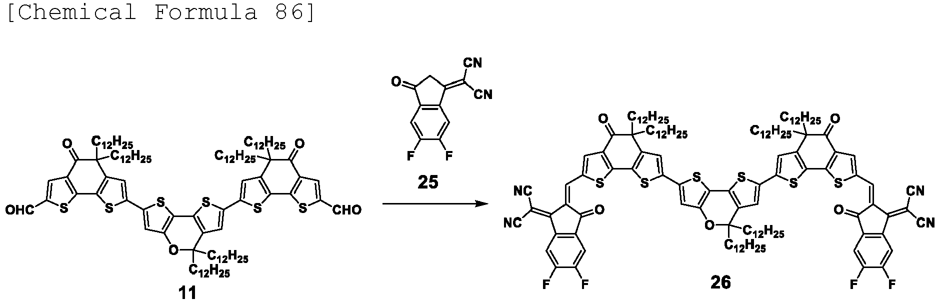

In a 100 mL four-necked flask whose internal atmosphere was replaced with a nitrogen gas, 1.00 g (0.600 mmol) of the compound 11, 0.345 g (1.50 mmol) of the compound 25 purchased from Angene International Limited, 30 mL of a chloroform, and 0.475 g (6.00 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was distilled away under reduced pressure. The obtained crude product was purified with a silica gel column to obtain 0.866 g (0.414 mmol, 68.9% in yield) of the compound 26 (compound N-7) as a dark blue-green solid.

The obtained compound N-7 was analyzed for an NMR spectrum. Here is the result.
[¹H NMR (CDCl₃)]

δ 8.865 (s, 1H), 8.863 (s, 1H), 8.57 (m, 2H), 8.16 (s, 2H), 7.72 (m, 2H), 7.10 (s, 1H), 7.07 (s, 1H), 6.971 (s, 1H), 6.967 (s, 1H), 2.25 (m, 4H), 1.96 (m, 4H), 1.77 (m, 4H), 1.24 (br, 120H), 0.86 (m, 18H).

### <Example 8> (Synthesis of Compound N-8)

The compound 11 and compound 27 represented by the following formula were used to synthesize a compound 28 (compound N-8) represented by the following formula.

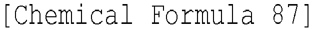

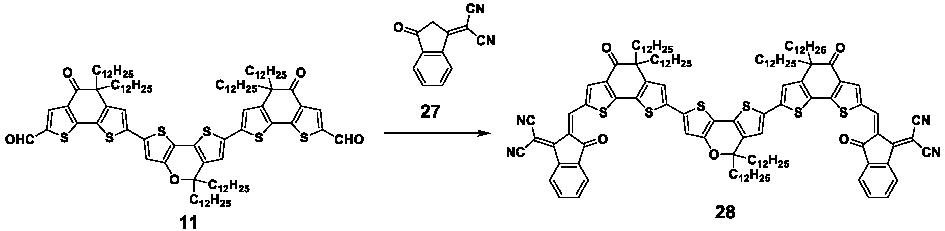

In a 100 mL four-necked flask whose internal atmosphere was replaced with a nitrogen gas, 0.951 g (0.570 mmol) of the compound 11, 0.332 g (1.71 mmol) of the compound 27 purchased from Tokyo Chemical Industry Co., Ltd., 30 mL of a chloroform, and 0.676 g (8.55 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution. The obtained organic layer was dried with a magnesium sulfate, and subjected to filtration, and the solvent was distilled away under reduced pressure.

The obtained crude product was purified with a silica gel column to obtain 0.975 g (0.482 mmol, 84.6% in yield) of the compound 28 (compound N-8) as a dark blue-green solid.

The obtained compound N-8 was analyzed for an NMR spectrum. Here is the result.
[¹H NMR (CDCl₃)]

δ 8.875 (s, 1H), 8.872 (s, 1H), 8.74 (s, 1H), 8.72 (s, 1H), 8.14 (s, 2H), 7.97 (m, 2H), 7.81 (m, 4H), 7.10 (s, 1H), 7.06 (s, 1H), 6.96 (s, 2H), 2.24 (m, 4H), 1.94 (m, 4H), 1.76 (m, 4H), 1.20 (br, 120H), 0.86 (m, 18H).

### <Example 9> (Synthesis of Compound N-9)

The compound 4 and compound 12 represented by the following formula were used to synthesize a compound 29 (compound N-9) represented by the following formula.

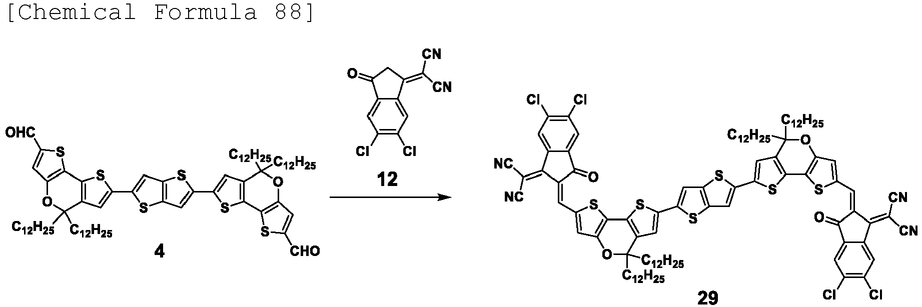

In a 100 mL four-necked flask whose internal atmosphere was replaced with a nitrogen gas, 1.63 g (1.30 mmol) of the compound 4, 0.855 g (3.25 mmol) of the compound 12, 26 mL of a chloroform, and 0.950 g (12.0 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was distilled away under reduced pressure.

The obtained crude product was purified with a silica gel column to obtain 1.33 g (0.745 mmol, 58.7% in yield) of the compound 29 (compound N-9) as a dark blue-green solid.

The obtained compound N-9 was analyzed for an NMR spectrum. Here is the result.
[¹H NMR (CDCl₃)]

δ 8.723 (s, 2H), 8.718 (s, 2H), 7.91 (s, 2H), 7.39 (s, 2H), 7.38 (s, 2H), 6.90 (s, 2H), 1.93 (m, 8H), 1.23 (br, 80H), 0.86 (t, 12H).

### <Synthesis Example 11> (Synthesis of Compound 30)

From the compound 14 represented by the following formula, a compound 30 represented by the following formula was synthesized.

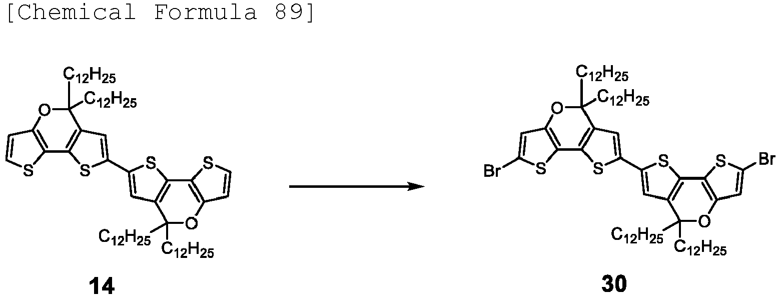

In a 100 mL four-necked flask whose internal atmosphere was replaced with a nitrogen gas, 2.0 g (1.88 mmol) of the compound 14 and 19 mL of a tetrahydrofuran were put, and cooled to 0°C in an ice bath. With 0.75 g (3.96 mmol) of N-bromosuccinimide purchased from Tokyo Chemical Industry Co., Ltd. put therein, the mixture was stirred as it was for 2 hours at ordinary temperature, and the reaction was then stopped with the use of a saturated aqueous sodium thiosulfate solution.

Next, the obtained solution was extracted with a chloroform, and then washed two times with water and one time with a saturated aqueous sodium chloride solution. The obtained organic layer was dried with a magnesium sulfate, and subjected to filtration, and the solvent was distilled away under reduced pressure.

Then, the obtained crude product was purified with a silica gel column to obtain 2.11 g (1.73 mmol, 92% in yield) of the compound 30 as a red solid.

### <Synthesis Example 12> (Synthesis of Compound 32)

From a compound 31 represented by the following formula, a compound 32 represented by the following formula was synthesized.

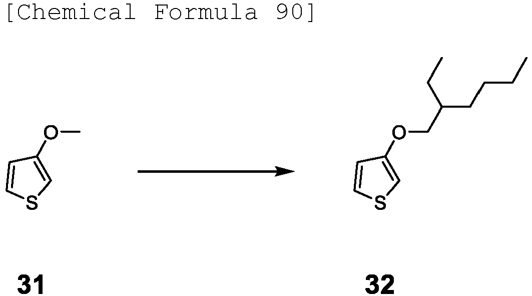

Into a 200 mL four-necked flask whose internal atmosphere was replaced with a nitrogen gas, 4.5 g (39.4 mmol) of the compound 31, 15.3 g (118.3 mmol) of a 2-ethylhexanol purchased from Tokyo Chemical Industry Co., Ltd., 0.68 g (3.94 mmol) of a p-toluenesulfonic acid, and 95 mL of a toluene were put, and stirred on heating for 2 hours in an oil bath at 100°C. The obtained solution was cooled down to ordinary temperature, and water was added to the solution to stop the reaction.

Next, the obtained solution was extracted with a chloroform, and then washed two times with water and one time with a saturated aqueous sodium chloride solution. The obtained organic layer was dried with a magnesium sulfate, and subjected to filtration, and the solvent was distilled away under reduced pressure.

Then, the obtained crude product was purified with a silica gel column to obtain 7.8 g (36.7 mmol, 93% in yield) of the compound 32 as a colorless oil.

### <Synthesis Example 13> (Synthesis of Compound 34)

The compound 32 and a compound 33 represented by the following formula were used to synthesize a compound 34 represented by the following formula.

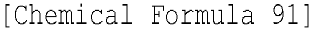

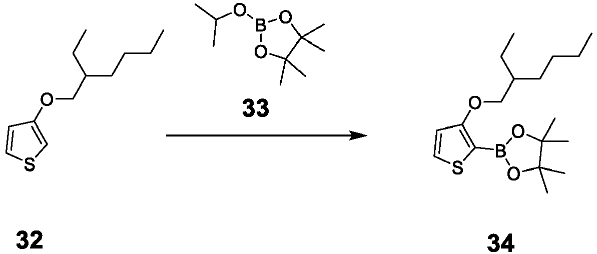

Into a 100 mL three-necked flask whose internal atmosphere was replaced with a nitrogen gas, 1.52 g (7.16 mmol) of the compound 32 and 30 mL of a dehydrated tetrahydrofuran were added, and the solution was cooled down to -70°C, and then with 3.3 mL of an n-butyllithium solution (2.6 mol/L, hexane solution) added thereto, stirred for 2 hours.

Next, with the reaction liquid kept at -70°C, 2.0 g (10.74 mmol) of the compound 33 purchased from Tokyo Chemical Industry Co., Ltd. was added thereto, and stirred for 2 hours.

Then, the temperature of the obtained reaction liquid was increased up to ordinary temperature, the reaction was then stopped with a saturated aqueous ammonium chloride solution, and a separation operation was performed with the use of an ethyl acetate to extract the organic layer.

The obtained organic layer was dried with a magnesium sulfate, and subjected to filtration, and the solvent was distilled away under reduced pressure to obtain 2.34 g of a crude product for the compound 34 as a colorless oil.

### <Synthesis Example 14> (Synthesis of Compound 35)

The compound 34 and compound 30 represented by the following formula were used to synthesize a compound 35 represented by the following formula.

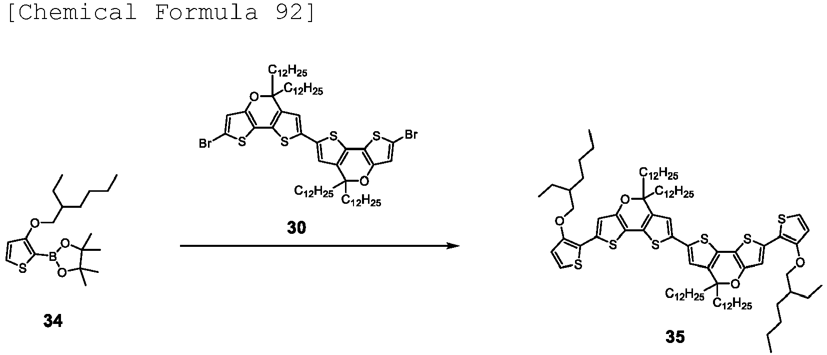

In a 100 mL four-necked flask whose internal atmosphere was replaced with a nitrogen gas, 1.27 g (2.63 mmol) of the compound 34, 1.45 g (1.19 mmol) of the compound 30, and 5 mL of a tetrahydrofuran were put, and subjected to degassing by bubbling with an argon gas for 30 minutes.

Next, to the obtained reaction liquid, 0.044 g (0.05 mmol, 8 mol%) of tris(dibenzylideneacetone) dipalladium (0) and 0.058 g (0.19 mmol, 16 mol%) of a tri-tert-butylphosphonium tetrafluoroborate were put, and stirred for 5 minutes.

Then, 5 mL of a 3.0 M aqueous potassium phosphate solution was further added to the reaction liquid, and the reaction liquid was stirred on heating for 1 hour in an oil bath at a set temperature of 65°C. After cooling the reaction liquid, 100 mL of water and 100 mL of hexane were added to the reaction liquid, and the reaction liquid was separated and washed with water three times and with a saturated aqueous sodium chloride solution one time. The obtained solution was dried with an anhydrous sodium sulfate, and then filtered, and the solvent was distilled away under reduced pressure.

The obtained crude product was purified with a silica gel column to obtain 1.51 g (1.02 mmol, 86% in yield) of the compound 35 as a dark red oil.

### <Synthesis Example 15> (Synthesis of Compound 36)

From the compound 35 represented by the following formula, a compound 36 represented by the following formula was synthesized.

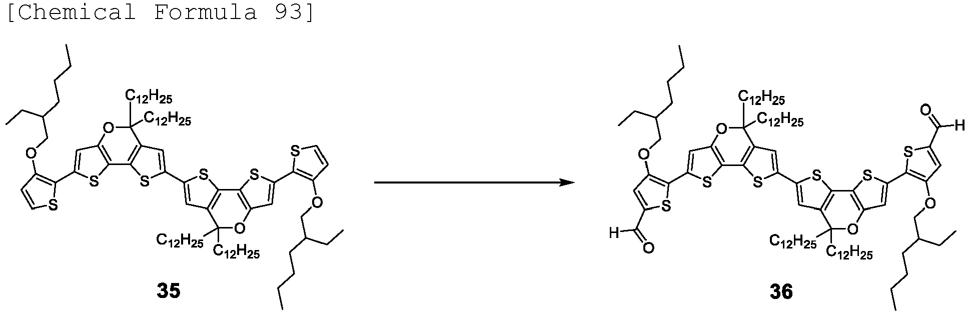

In a 100 mL four-necked flask whose internal atmosphere was replaced with a nitrogen gas, 0.43 g (0.29 mmol) of the compound 35 and 5 mL of a dichloromethane were added to dissolve the compound 35.

Next, in the obtained solution, 0.12 g (0.94 mmol) of a (chloromethylene)dimethyliminium chloride was put, and stirred on heating for 3 hours in an oil bath at a set temperature of 40°C.

Then, after cooling the obtained solution, 5 mL of water was added thereto, and the reaction liquid was separated and washed two times with a saturated aqueous sodium chloride solution. The obtained solution was dried with a magnesium sulfate, and then filtered, and the solvent was distilled away under reduced pressure.

The obtained crude product was purified with a silica gel column to obtain 0.35 g (0.23 mmol, 78.6% in yield) of the compound 36 as a purple solid.

### <Example 10> (Synthesis of Compound N-10)

The compound 36 and compound 12 represented by the following formula were used to synthesize a compound 37 (compound N-10) represented by the following formula.

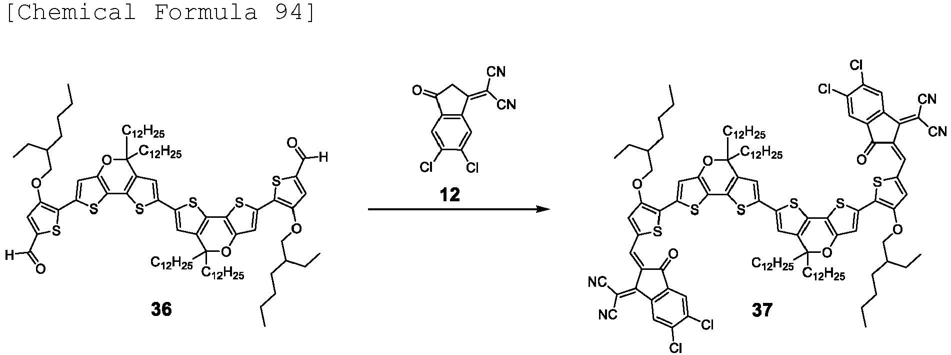

In a 100 mL four-necked flask whose internal atmosphere was replaced with a nitrogen gas, 0.35 g (0.23 mmol) of the compound 36, 0.32 g (1.23 mmol) of the compound 12, 10 mL of a chloroform, and 0.002 g (0.025 mmol) of a pyridine were put, and stirred on heating for 2 hours in an oil bath at 65°C.

Next, the obtained solution was cooled down to ordinary temperature, and water was added to the solution to stop the reaction. The obtained solution was extracted with a chloroform, and then washed two times with water and one time with a saturated aqueous sodium chloride solution.

Then, the obtained organic layer was dried with a magnesium sulfate, and subjected to filtration, and the solvent was distilled away under reduced pressure.

The obtained crude product was purified with a silica gel column to obtain 0.4 g (0.197 mmol, 85.9% in yield) of the compound 37 (compound N-10) as a black solid.

The obtained compound N-10 was analyzed for an NMR spectrum. Here is the result.
[¹H NMR (CDCl₃)]

δ 8.71 (s, 2H), 8.66 (s, 2H), 8.04 (s, 2H), 7.89 (s, 2H), 7.48 (br, 2H), 6.78 (s, 2H), 3.94 (d, 4H), 2.01-1.80 (m, 8H), 1.70-0.97 (m, 46H), 0.91-0.76 (m, 32H).

### <Synthesis Example 16> (Synthesis of Compound 39)

The compound 38 and compound 19 represented by the following formula were used to synthesize a compound 39 represented by the following formula.

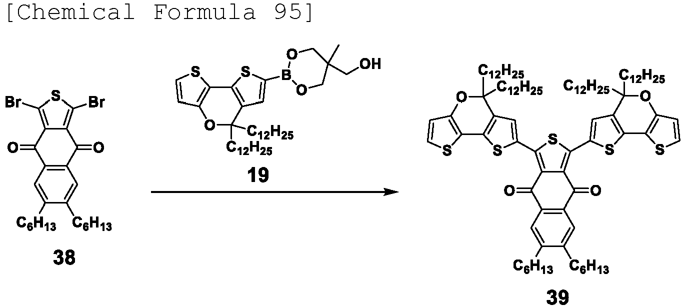

In a 200 mL four-necked flask whose internal atmosphere was replaced with a nitrogen gas, 1.08 g (2.00 mmol) of the compound 38 synthesized by the method described in JP-A-2013-213180, 3.36 g (4.60 mmol) of the compound 12, and 67 mL of tetrahydrofuran were put, and subjected to degassing by bubbling with a nitrogen gas for 30 minutes.

Then, to the four-necked flask, 0.0916 g (0.100 mmol, 5 mol%) of tris(dibenzylideneacetone) dipalladium (0) and 0.116 g (0.400 mmol, 20 mol%) of a tri-tert-butylphosphonium tetrafluoroborate were put, and stirred for 5 minutes.

Next, 6.7 mL of a 3.0 M aqueous potassium phosphate solution was added to the four-necked flask, and the reaction liquid was stirred on heating for 1 hour in an oil bath at a set temperature of 76°C.

After cooling the reaction liquid, 100 mL of water and 100 mL of heptane were added to the four-necked flask, and the organic layer was separated and washed with water three times and with a saturated aqueous sodium chloride solution one time.

The obtained solution was dried with the use of an anhydrous magnesium sulfate, and then filtered, and the solvent was distilled away under reduced pressure. The obtained crude product was purified with a silica gel column to obtain 2.55 g (1.77 mmol, 88.5% in yield) of the compound 39 as a dark red-purple oil.

The obtained compound 39 was analyzed for an NMR spectrum. Here is the result.
[¹H NMR (CDCl₃)]

δ 8.13 (s, 2H), 7.51 (s, 2H), 7.11 (s, 1H), 7.09 (s, 1H), 6.70 (s, 1H), 6.69 (s, 1H), 2.76 (m, 4H), 1.95 (m, 8H), 1.69-1.62(m, 4H), 1.45-1.22 (br, 92H), 0.93-0.82 (m, 18H)

### <Synthesis Example 17> (Synthesis of Compound 40)

The compound 39 represented by the following formula was used to synthesize a compound 40 represented by the following formula.

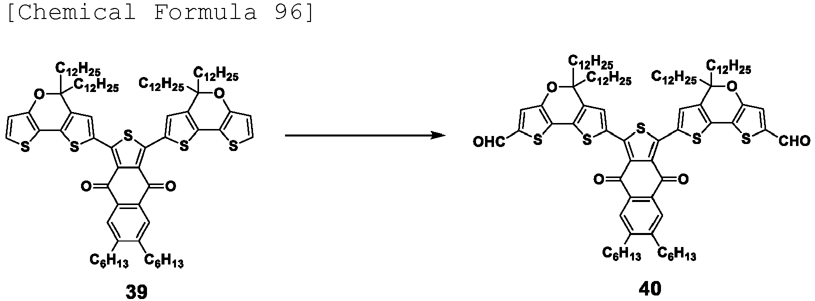

In a 100 mL four-necked flask whose internal atmosphere was replaced with a nitrogen gas, 2.52 g (1.75 mmol) of the compound 39 and 88 mL of dichloromethane were put, and stirred at ordinary temperature for 10 minutes to dissolve the compound 39.

Next, in the four-necked flask, 1.12 g (8.75 mmol) of a (chloromethylene)dimethyliminium chloride was put, and stirred on heating for 7.5 hours in an oil bath at a set temperature of 49°C.

Then, after cooling the reaction liquid, 18 mL of water was added thereto, and the organic layer was separated and washed two times with a saturated aqueous sodium bicarbonate solution. The obtained solution was dried with the use of an anhydrous magnesium sulfate, and then subjected to filtration, and the solvent was distilled away under reduced pressure.

The obtained crude product was purified with a silica gel column to obtain 2.54 g (1.70 mmol, 97.0% in yield) of the compound 40 as a dark red-purple oil.

The obtained compound 40 was analyzed for an NMR spectrum. Here is the result.
[¹H NMR (CDCl₃)]

δ 9.81 (s, 2H), 8.11 (s, 2H), 7.53 (s, 2H), 7.30 (s, 2H), 2.77(t, J_{HH}=8.0Hz,4H), 1.99-1.94 (m, 8H), 1.72-1.62 (m, 4H), 1.45-1.21 (br, 92H), 0.91(t, J_{HH}=7.0Hz,6H), 0.84 (t, J_{HH}=6.8Hz,12H) .

### <Example 11> (Synthesis of Compound N-11)

The compound 40 and compound 12 represented by the following formula were used to synthesize a compound 41 (compound N-11) represented by the following formula.

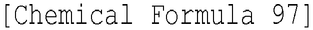

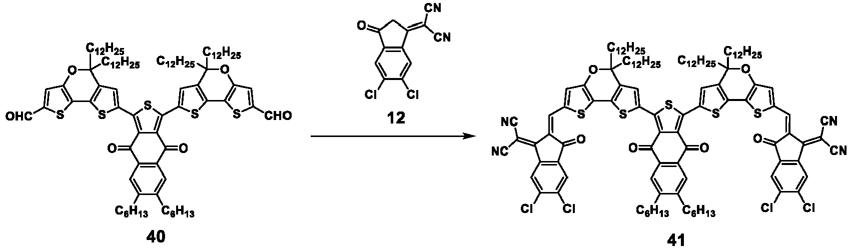

In a 100 mL four-necked flask whose internal atmosphere was replaced with a nitrogen gas, 0.748 g (0.500 mmol) of the compound 40, 0.395 g (1.50 mmol) of the compound 12, 25 mL of a chloroform, and 0.396 g (5.00 mmol) of a pyridine were put, and stirred on heating for 1 hour in an oil bath at 68°C.

Then, the obtained solution was cooled down to ordinary temperature, and added to 150 mL of a methanol. The obtained solution was filtered to obtain a crude product as a precipitate.

The obtained crude product was purified with a silica gel column to obtain 0.882 g (0.444 mmol, 88.8% in yield) of the compound 41 (compound N-11) as a black solid.

The obtained compound N-11 was analyzed for an NMR spectrum. Here is the result.
[¹H NMR (CDCl₃)]

δ 8.63 (s, 2H), 8.60 (s, 2H), 7.85 (s, 2H), 7.48 (s, 2H), 7.24 (s, 2H), 2.80(t, J_{HH}=7.9Hz,4H), 1.98 (br, 8H), 1.79-1.69 (m, 4H), 1.56-1.21 (br, 92H), 0.97(t, J_{HH}=6.9Hz,6H), 0.83(t, J_{HH}= 6.9Hz,12H) .

### <Synthesis Example 18> (Synthesis of Compound 43)

The compound 19 and a compound 42 represented by the following formula were used to synthesize a compound 43 represented by the following formula.

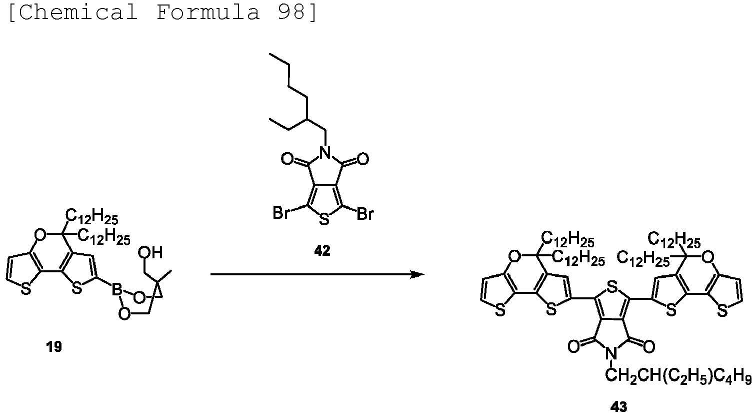

In a 100 mL three-necked flask, 1.00 g (2.36 mmol) of the compound 42 (manufactured by Tokyo Chemical Industry Co., Ltd.), 4.14 g (4.96 mmol) of the compound 19, and 23.6 mL of THF were put, and subjected to degassing by bubbling with argon gas for 30 minutes.

Next, 0.087 g (0.09 mmol, 4 mol%) of tris(dibenzylideneacetone)dipalladium (0), 0.056 g (0.19 mmol, 8 mol%) of tri-tert-butylphosphonium tetrafluoroborate, and 23.6 mL of a 3.0 M aqueous potassium phosphate solution were added thereto, and the temperature was increased to 40°C.

The obtained reaction liquid was cooled, and then diluted with a toluene, and the organic layer was washed with water two times.

The obtained solution was dried with a magnesium sulfate, and then subjected to filtration, and the solvent was distilled away under reduced pressure to obtain a crude product.

The obtained crude product was purified with a silica gel column to obtain 1.87 g (59.8% in yield, 1.41 mmol) of the compound 43 as a black solid.

The obtained compound 43 was analyzed for an NMR spectrum. Here is the result.
[¹H NMR (CDCl₃)]

δ 7.60 (s, 2H), 7.05 (d, J_{HH}=5.6Hz, 2H), 6.66 (d, J_{HH}=5.6Hz, 2H), 3.56 (t, 2H), 1.92-1.88 (m, 9H), 1.38-1.21 (m, 88H), 0.95-0.83 (m, 18H).

### <Synthesis Example 19> (Synthesis of Compound 44)

The compound 43 represented by the following formula was used to synthesize a compound 44 represented by the following formula.

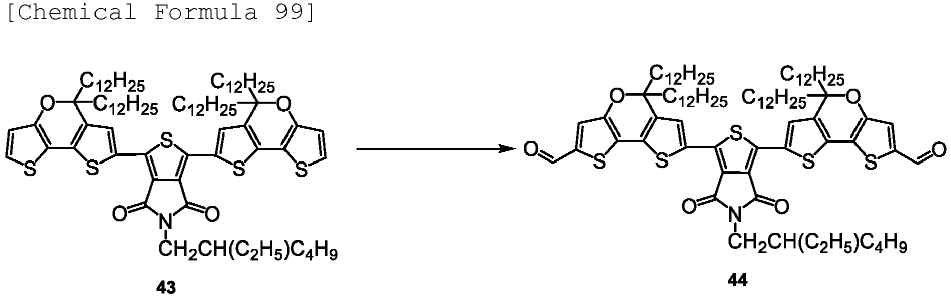

In a 100 mL three-necked flask whose internal atmosphere was replaced with a nitrogen gas, 1.49 g (1.13 mmol) of the compound 43, 56.3 mL of a dehydrated chloroform, and 0.552 g (4.32 mmol) of a (chloromethylene)dimethyliminium chloride were put, and at the internal temperature increased to 61°C, stirred for 16 hours.

Next, after cooling the obtained reaction liquid, water was further added thereto, and the organic layer was separated and washed two times with a saturated aqueous sodium bicarbonate solution.

The obtained solution was dried with the use of an anhydrous magnesium sulfate, and then subjected to filtration, and the solvent was distilled away under reduced pressure.

The obtained crude product was purified with a silica gel column to obtain 1.31 g (84.4% in yield, 0.95 mmol) of the compound 44 as a black oil.

The obtained compound 44 was analyzed for an NMR spectrum. Here is the result.
[¹H NMR (CDCl₃)]

δ 9.73 (s, 2H), 7.77 (s, 2H), 7.28 (s, 2H), 3.56 (t, 2H), 1.94-1.90 (m, 9H), 1.52-1.09 (m, 88H), 0.87-0.73 (m, 18H) .

### <Example 12> (Synthesis of Compound N-12)

The compound 44 and compound 12 represented by the following formula were used to synthesize a compound 45 (compound N-12) represented by the following formula.

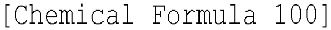

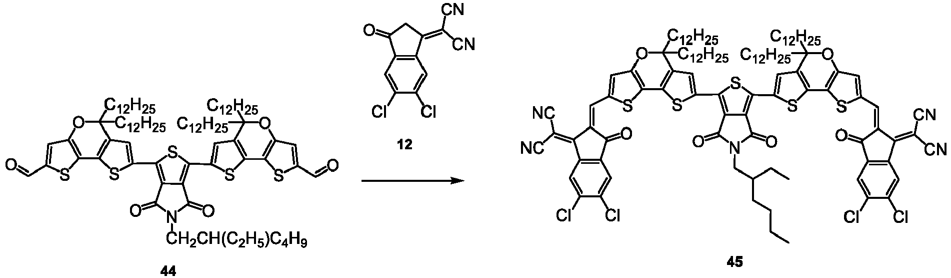

In a 50 mL two-necked flask, 0.500 g (0.363 mmol) of the compound 44, 18.0 g of a dehydrated chloroform, 0.286 g (1.088 mmol) of the compound 12, and 0.287 g (3.63 mmol) of a pyridine were put, and the temperature was increased to 60°C in an oil bath.

After stirring for 3 hours, the reaction liquid was allowed to cool to ordinary temperature, and separated and washed with 10 g of water three times, the organic layer was then dried with a magnesium sulfate, subjected to filtration, and the solvent was distilled away under reduced pressure to obtain a crude product.

The obtained crude product was purified with a recycled preparative gas chromatography (GPC) to obtain 0.350 g (52% in yield, 0.187 mmol) of the compound 45 (N-12) as a black solid.

The obtained compound N-12 was analyzed for an NMR spectrum. Here is the result.
[¹H NMR (CDCl₃)]

δ 8.71 (m, 4H), 7.91 (s, 2H), 7.71 (s, 2H), 7.31 (br, 2H), 3.60 (t, 2H), 2.11-1.77 (m, 9H), 1.50-1.06 (m, 88H), 0.98-0.82 (m, 18H).

### <Synthesis Example 20> (Synthesis of Compound 47)

The compound 19 and a compound 46 represented by the following formula were used to synthesize a compound 47 represented by the following formula.

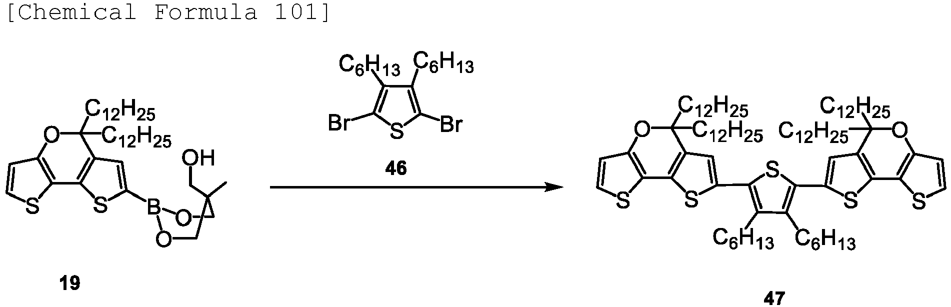

In a 100 mL three-necked flask, 8.34 g (10.00 mmol) of the compound 19, 1.71 g (4.17 mmol) of the compound 46 (manufactured by Tokyo Chemical Industry Co., Ltd.), and 41.7 mL of a tetrahydrofuran were put, and subjected to degassing by bubbling with an argon gas for 30 minutes.

Next, after adding 0.305 g (0.33 mmol, 4 mmol%) of tris(dibenzylideneacetone) dipalladium (0), 0.203 g (0.67 mmol, 8 mmol%) of tri-tert-butylphosphonium tetrafluoroborate, and 41.7 mL of a 3.0 M aqueous potassium phosphate solution thereto, the temperature was increased to 60°C.

Then, the reaction liquid was stirred for 2 hours, and then cooled to ordinary temperature.

Next, the organic layer was extracted, diluted with a hexane, washed two times with water, then dried with a magnesium sulfate, filtered, and then entirely concentrated with a rotary evaporator.

The product was further purified with a silica gel column to obtain 5.56 g (yield) of the compound 47 as an orange oil.

The obtained compound 47 was analyzed for an NMR spectrum. Here is the result.
[¹H NMR (CDCl₃)]

δ 6.98 (d, J_{HH}=6.4Hz, 2H), 6.71 (s, 2H), 6.67 (d, J_{HH}=6.4Hz, 2H), 2.68 (t, 4H), 1.86 (m, 8H), 1.57 (m, 4H), 1.22 (m, 92H), 0.88 (m, 18H).

### <Synthesis Example 21> (Synthesis of Compound 48)

The compound 47 represented by the following formula was used to synthesize a compound 48 represented by the following formula.

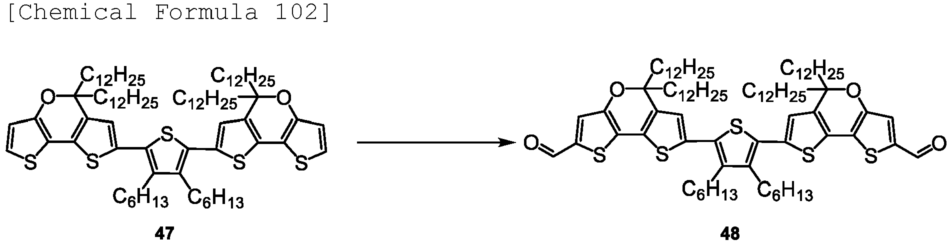

In a 200 mL three-necked flask whose internal atmosphere was replaced with a nitrogen gas, 1.61 g (1.23 mmol) of the compound 47, 61.4 mL of a dehydrated chloroform, and 0.472 g (3.69 mmol) of (chloromethylene)dimethyliminium chloride were charged, and stirred at an internal temperature of 60°C for 2 hours.

After cooling the reaction liquid, water was added thereto, and the organic layer was separated and washed two times with a saturated aqueous sodium bicarbonate solution.

The obtained solution was dried with the use of an anhydrous magnesium sulfate, and then subjected to filtration, and the solvent was distilled away under reduced pressure.

The obtained crude product was purified with a silica gel column to obtain 1.22 g (72.7% in yield, 0.89 mmol) of the compound 48 as a reddish black oil.

The obtained compound 48 was analyzed for an NMR spectrum. Here is the result.
[¹H NMR (CDCl₃)]

δ 9.76 (s, 2H), 7.27 (s, 2H), 6.75 (s, 2H), 2.70 (t, 4H), 1.89 (m, 8H), 1.56 (m, 4H), 1.22 (m, 92H), 0.85 (m, 18H) .

### <Example 13> (Synthesis of Compound N-13)

The compound 48 and compound 12 represented by the following formula were used to synthesize a compound 49 (compound N-13) represented by the following formula.

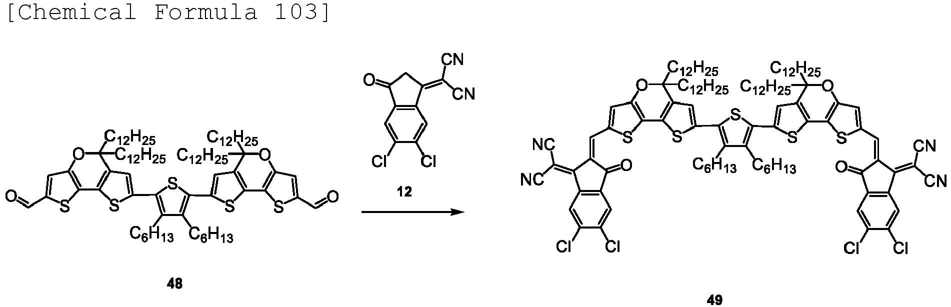

In a 50 mL two-necked flask, 0.80 g (0.586 mmol) of the compound 48, 29.0 g of a dehydrated chloroform, 0.462 g (1.76 mmol) of the compound 12, and 0.463 g (5.86 mmol) of a pyridine were put, and immersed in an oil bath heated to 60°C and then stirred for 2 hours.

After cooling, the reaction liquid was separated and washed with 87 g of water, and the organic layer was then dried with an anhydrous magnesium sulfate and subjected to filtration, and the solvent was distilled away under reduced pressure.

The obtained crude product was purified with a silica gel column to obtain 0.636 g (58% in yield, 0.342 mmol) of the compound 49 (N-13) as a black solid.

The obtained compound N-13 was analyzed for an NMR spectrum. Here is the result.
[¹H NMR (CDCl₃)]

δ 8.75 (s, 2H,), 8.73 (s, 2H), 7.89 (s, 2H), 7.39 (s, 2H), 6.86 (s, 2H), 2.77 (t, 4H), 1.92 (m, 8H), 1.60 (m, 4H), 1.21 (m, 92H), 0.93 (m, 6H), 0.85 (m, 12H).

### <Synthesis Example 22> (Synthesis of Compound 51)

The compound 19 and a compound 50 represented by the following formula were used to synthesize a compound 51 represented by the following formula.

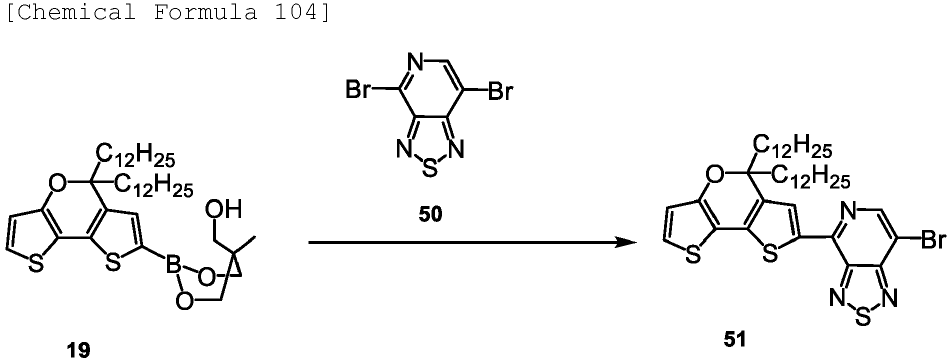

In a 10 mL four-necked flask, 1.2 g (4.05 mmol) of the compound 50 (manufactured by Tokyo Chemical Industry Co., Ltd.), 3.15 g (4.05 mmol) of the compound 19, and 40.5 mL of a tetrahydrofuran were put, and subjected to degassing by bubbling with a nitrogen gas for 30 minutes.

Next, after cooling to 0°C with an ice bath, 0.111 g (0.12 mmol) of tris(dibenzylideneacetone) dipalladium (0), 0.074 g (0.24 mmol) of tri-tert-butylphosphonium tetrafluoroborate, and 40.5 mL of a 3.0 M aqueous potassium phosphate solution were added in this order.

After stirring for 4 hours, the temperature was increased to ordinary temperature, and the reaction liquid was diluted with water and a chloroform, and separated and washed to obtain an organic layer.

Next, the obtained organic layer was washed one time with each of water and a saturated saline, dried with a magnesium sulfate, and filtered, and the solvent was then distilled away under reduced pressure.

The obtained crude product was purified with a silica gel column to obtain 2.43 g (80.5% in yield) of the compound 51 as a black solid.

The obtained compound 51 was analyzed for an NMR spectrum. Here is the result.
[¹H NMR (CDCl₃)]

δ 8.57 (s, 1H), 8.27 (s, 1H), 7.11 (d, J_{HH}=5.6Hz, 1H), 6.69 (d, J_{HH}=5.6Hz, 1H), 1.96 (m, 4H), 1.19-1.49 (m, 40H), 0.82-0.89 (m, 6H).

### <Synthesis Example 23> (Synthesis of Compound 53)

The compound 51 and compound 52 represented by the following formula were used to synthesize a compound 53 represented by the following formula.

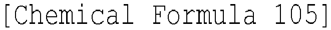

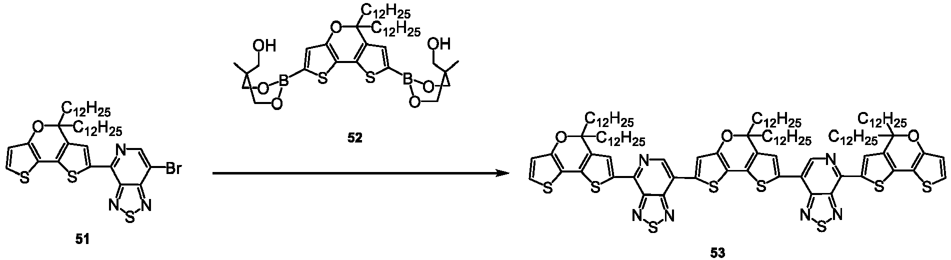

In a 100 mL four-necked flask, 2.29 g (2.84 mmol) of 51, 1.12 g (1.42 mmol) of the compound 52 synthesized by the method described in WO 2014/112656 A, and 14.2 mL of a tetrahydrofuran were put, and subjected to degassing by bubbling with a nitrogen gas for 30 minutes.

Next, 0.039 g (0.04 mmol) of tris(dibenzylideneacetone) dipalladium (0), 0.026 g (0.09 mmol) of tri-tert-butylphosphonium tetrafluoroborate, and 14.2 mL of a 3.0 M aqueous potassium phosphate solution were added in this order, and the mixture was immersed in an oil bath at 40°C to increase the temperature.

Then, the reaction liquid was stirred for 3 hours, and then cooled to ordinary temperature. The obtained reaction liquid was diluted with water and a chloroform, and separated and washed with the use of a separating funnel to collect an organic layer, and the organic layer was washed one time with an aqueous acetic acid and one time with a saturated saline, then dried with a magnesium sulfate, and filtered, and the solvent was then distilled away under reduced pressure.

The obtained crude product was dissolved in a hexane, an acetone was then added thereto, and the precipitated solid was filtered to obtain 2.14 g (81% in yield, 1.15 mmol) of the compound 53 as a blue-black solid.

The obtained compound 53 was analyzed for an NMR spectrum. Here is the result.
[¹H NMR (CDCl₃)]

δ 8.71 (d, J_{HH}=4.8Hz, 2H), 8.26 (d, J_{HH}=4.8Hz, 2H), 7.81 (s, 1H),7.73 (s, 1H), 7.10 (m, 2H), 6.69 (d, 2H), 1.94-2.04 (m, 12H), 1.20-1.51 (m, 120H), 0.81-0.86 (m, 18H) .

### <Synthesis Example 24> (Synthesis of Compound 54)

The compound 53 represented by the following formula was used to synthesize a compound 54 represented by the following formula.

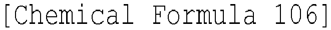

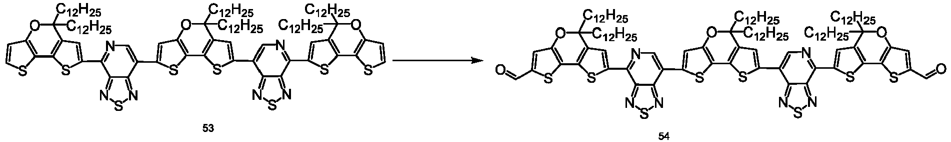

In a 50 mL two-necked flask whose internal atmosphere was replaced with an argon gas, 1.02 g (0.55 mmol) of the compound 53, 27.5 mL of a chloroform, and 0.211 g (1.65 mmol) of a (chloromethylene dimethyliminium chloride were put, and the temperature was increased to 60°C.

After cooling, water was added to the reaction liquid, and the organic layer was separated and washed two times with a saturated aqueous sodium bicarbonate solution. The obtained solution was dried with the use of an anhydrous magnesium sulfate, and then subjected to filtration, and the solvent was distilled away under reduced pressure.

The obtained crude product was purified with a silica gel column to obtain 0.979 g (92.9% in yield, 0.191 mmol) of the compound 54 as a black solid.

The obtained compound 54 was analyzed for an NMR spectrum. Here is the result.
[¹H NMR (CDCl₃)]

δ 9.7 (s, 1H), 9.75 (s, 1H), 8.66 (s, 2H), 8.25 (s, 1H), 8.23 (s, 1H), 7.83 (s, 1H), 7.72 (s, 1H), 7.28 (s, 1H), 7.26 (s, 1H), 1.99-2.07 (m, 12H), 1.18-1.51 (m, 120H), 0.79-0.86 (m, 18H).

### <Example 14> (Synthesis of Compound N-14)

The compound 54 and compound 12 represented by the following formula were used to synthesize a compound 55 (compound N-14) represented by the following formula.

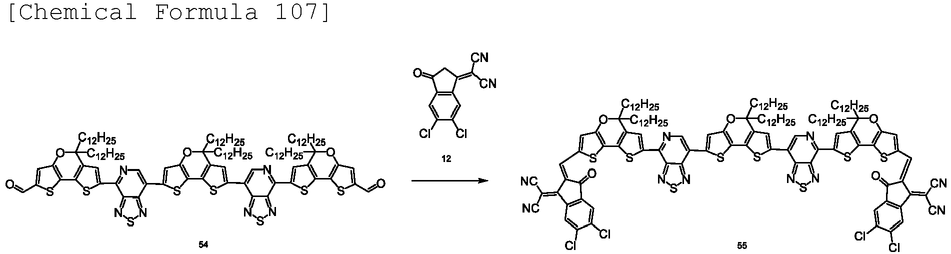

In a 50 mL two-necked flask whose internal atmosphere was replaced with a nitrogen gas, 0.622 g (0.322 mmol) of the compound 54, 16 g of a dehydrated chloroform, 0.257 g of the compound 12, and 0.257 g of a pyridine were put, and the mixture was immersed in an oil bath heated to 60°C to increase the temperature.

The reaction liquid was stirred for 4 hours, then cooled to ordinary temperature, diluted with 70 g of a chloroform, and purified with a silica gel column to obtain 0.675 g (86.3% in yield, 0.28 mmol) of the compound 55 (N-14) as a black solid.

The obtained compound N-14 was analyzed for an NMR spectrum. Here is the result.
[¹H NMR (O-DICHLOROBENZENE-D4)]

δ 8.46-8.66 (m, 9H), 8.03 (s, 1H), 7.83(s, 1H), 7.68 (s, 2H), 7.42 (m, 2H), 2.14-2.23 (m, 12H), 1.29-1.66 (m, 120H), 0.77 (m, 18H).

### <Synthesis Example 25> (Synthesis of Compound 56)

The compound 55 represented by the following formula was used to synthesize a compound 56 represented by the following formula.

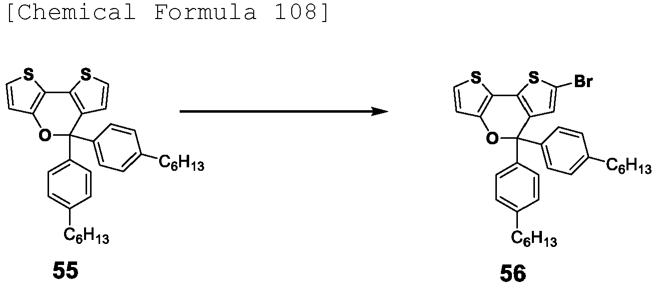

In a 300 mL four-necked flask whose internal atmosphere was replaced with a nitrogen gas, 4.00 g (7.77 mmol) of the compound 55 synthesized by the method described in the literature (Dyes and Pigments, 2015,112,145.) and 78 mL of a tetrahydrofuran were put, and cooled to -30°C.

Next, to the four-necked flask, 1.31 g (7.38 mmol) of an N-bromosuccinimide was added, and stirred at -30°C for 6 hours.

Then, the temperature of the obtained solution was increased up to ordinary temperature, and the solution was reacted by further stirring for 3 hours at ordinary temperature, and a 3% aqueous sodium sulfite solution was then further added to stop the reaction.

The obtained reaction liquid was extracted with a hexane, and then washed with water and a saturated aqueous sodium chloride solution to obtain an organic layer.

Next, the obtained organic layer was dried with a magnesium sulfate, and then subjected to filtration, and the solvent was further distilled away under reduced pressure.

The obtained crude product was purified with a silica gel column to obtain 4.16 g (7.01 mmol, 94.9% in yield) of the compound 56 as a light yellow oil.

The obtained compound 56 was analyzed for an NMR spectrum. Here is the result.
[¹H NMR (CDCl₃)]

δ 7.16 (d, J_{HH}=8.4Hz, 4H),7.10 (d, J_{HH}=8.4Hz, 4H), 6.98 (d, J_{HH}=4.8Hz, 1H),6.75 (d, J_{HH}=4.8Hz, 1H), 6.42 (s, 1H), 2.58 (t, 4H), 1.63-1.57(m, 4H), 1.36-1.27 (br, 12H), 0.87 (t, 6H) .

### <Synthesis Example 26> (Synthesis of Compound 57)

The compound 56 represented by the following formula was used to synthesize a compound 57 represented by the following formula.

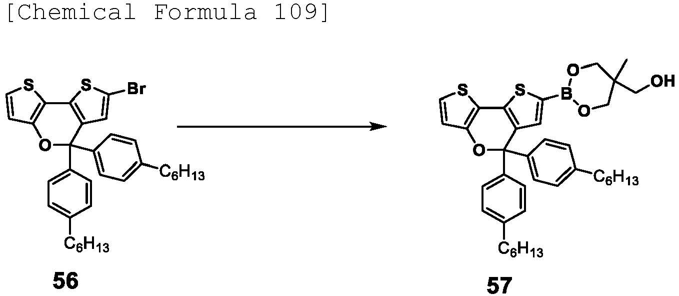

Into a 100 mL three-necked flask whose internal atmosphere was replaced with a nitrogen gas, 4.13 g (6.96 mmol) of the compound 56 and 70 mL of a dehydrated tetrahydrofuran were added, and the obtained solution was cooled down to -70°C, and then with 4.13 mL of an n-butyllithium solution (1.64 mol/L, hexane solution) added thereto, stirred for 1 hour.

Next, with the reaction liquid kept at -70°C, 1.01 g (9.74 mmol) of a trimethoxyborane was added, and stirred for 2 hours.

Next, a 10% by mass acetic acid aqueous solution (30 mL) was put into the obtained reaction liquid, and a separation operation was performed with the use of an ethyl acetate to extract the organic layer. The obtained organic layer was, with toluene (20 mL) and 1.25 g (10.4 mmol) of a 2-hydroxymethylene-2-methyl-1,3-propanediol added thereto, subjected to a dehydration operation with a Dean-Stark tube used, for 30 minutes. Furthermore, the solvent was removed with a rotary evaporator, thereby providing, as a green oil, 4.47 g of a crude product for the compound 57.

### <Synthesis Example 27> (Synthesis of Compound 59)

The compound 57 and compound 58 represented by the following formula were used to synthesize a compound 59 represented by the following formula.

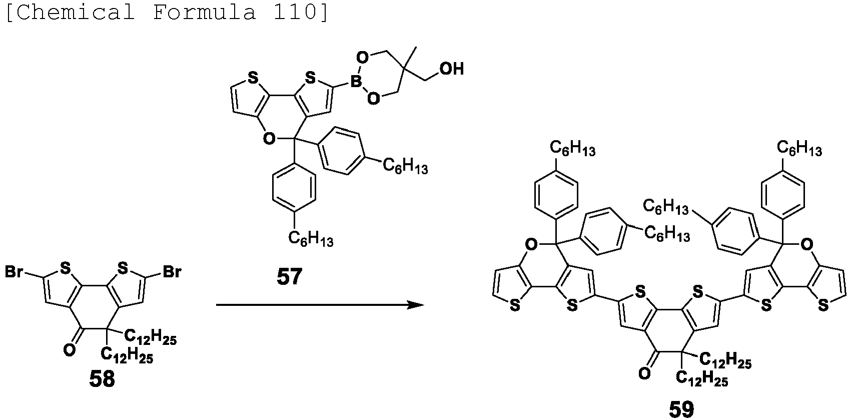

In a 300 mL four-necked flask whose internal atmosphere was replaced with a nitrogen gas, 1.00 g (1.43 mmol) of the compound 58 synthesized by the method described in JP-A-2013 -43818, 2.20 g (3.43 mmol) of the compound 57, and 14 mL of a tetrahydrofuran were put, and subjected to degassing by bubbling with an argon gas for 30 minutes.

Next, in the four-necked flask, 0.105 g (0.11 mmol, 8 mol%) of tris(dibenzylideneacetone) dipalladium (0), 0.070 g (0.23 mmol, 16 mol%) of tri-tert-butylphosphonium tetrafluoroborate, and 5 mL of a tetrahydrofuran were put, and stirred for 5 minutes.

Next, 14 mL of a 3.0 M aqueous potassium phosphate solution was added to the four-necked flask, and the reaction liquid was stirred on heating for 3 hours in an oil bath at a set temperature of 50°C.

After cooling the reaction liquid, 100 mL of water and 100 mL of hexane were added to the four-necked flask, and the organic layer was separated and washed with water three times and with a saturated aqueous sodium chloride solution one time.

The obtained solution was dried with the use of an anhydrous sodium sulfate, and then filtered, and the solvent was distilled away under reduced pressure.

The obtained crude product was purified with a silica gel column to obtain 1.74 g (1.11 mmol, 78% in yield) of the compound 59 as a red oil.

The obtained compound 59 was analyzed for an NMR spectrum. Here is the result.
[¹H NMR (CDCl₃)]

δ 7.38 (s, 1H), 7.23-7.18 (m, 8H),7.14-7.10 (m, 8H),7.03-7.00 (m, 2H),6.92 (s, 1H),6.79-6.77 (m, 2H), 6.58 (s, 1H), 6.53 (s, 1H),2.62-2.57 (m, 8H), 2.19-2.11 (m, 2H), 1.71-1.56 (m, 10H), 1.37-1.25 (m, 28H), 1.25-1.08 (br, 36H), 0.89-0.83 (m, 18H).

### <Synthesis Example 28> (Synthesis of Compound 60)

The compound 59 represented by the following formula was used to synthesize a compound 60 represented by the following formula.

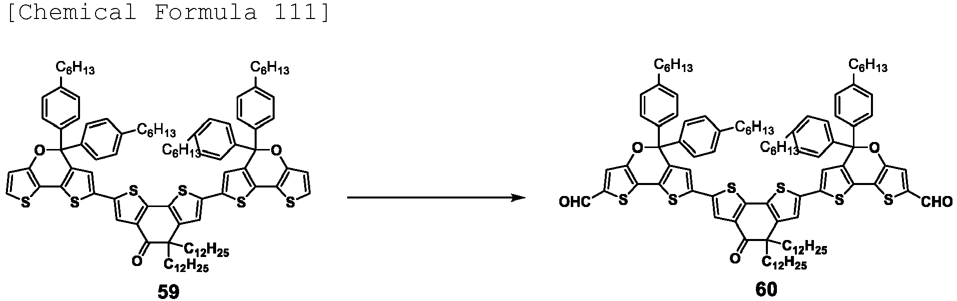

In a 100 mL three-necked flask whose internal atmosphere was replaced with a nitrogen gas, 1.74 g (1.11 mmol) of the compound 59 and 12 mL of a chloroform were put, and stirred at ordinary temperature for 10 minutes to dissolve the compound 59.

Next, in the three-necked flask, 0.43 g (3.33 mmol) of a (chloromethylene)dimethyliminium chloride was put, and stirred on heating for 2 hours in an oil bath at a set temperature of 65°C.

Then, after cooling the reaction liquid, 20 mL of water was added thereto, and the organic layer was separated and washed two times with a saturated aqueous sodium chloride solution. The obtained solution was dried with the use of an anhydrous sodium sulfate, and then subjected to filtration, and the solvent was distilled away under reduced pressure.

The obtained crude product was purified with a silica gel column to obtain 1.63 g (1.01 mmol, 90.6% in yield) of the compound 60 as a dark red oil.

The obtained compound 60 was analyzed for an NMR spectrum. Here is the result.
[¹H NMR (CDCl₃)]

δ 9.76 (s, 1H), 9.75 (s, 1H), 7.46 (s, 1H), 7.37 (s, 1H), 7.36 (s, 1H), 7.21-7.12 (m, 16H), 7.00 (s, 1H), 6.61 (s, 1H), 6.58 (s, 1H), 2.63-2.58 (m, 8H), 2.21-2.13(m, 2H), 1.73-1.57 (m, 10H), 1.37-1.26 (m, 28H), 1.24-1.09 (br, 36H), 0.89-0.83 (m, 18H).

### <Example 15> (Synthesis of Compound N-15)

The compound 12 and compound 60 represented by the following formula were used to synthesize a compound 61 (compound N-15) represented by the following formula.

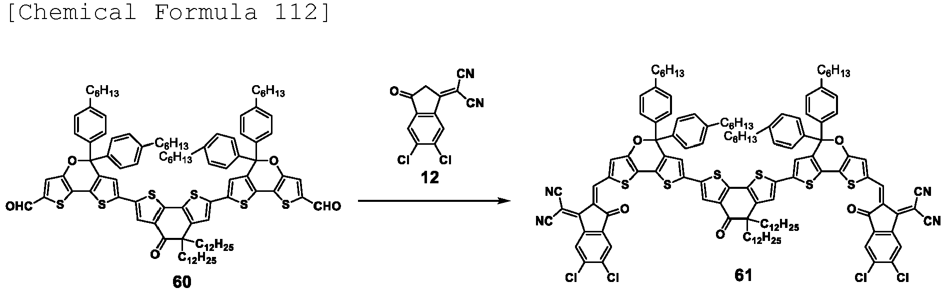

In a 100 mL four-necked flask whose internal atmosphere was replaced with a nitrogen gas, 0.57 g (0.35 mmol) of the compound 60, 0.28 g (1.05 mmol) of the compound 12, 10 mL of a chloroform, and 0.003 g (0.04 mmol) of a pyridine were put, and stirred on heating for 2 hours in an oil bath at 65°C.

The obtained solution was cooled down to ordinary temperature, and water was added to the solution to stop the reaction. The obtained solution was extracted with a chloroform, and then washed two times with water and one time with a saturated aqueous sodium chloride solution to obtain an organic layer.

Next, the obtained organic layer was dried with a magnesium sulfate, and subjected to filtration, and the solvent was further distilled away under reduced pressure.

The obtained crude product was purified with a silica gel column to obtain 0.64 g (0.30 mmol, 86.3% in yield) of the compound 61 (compound N-15) as a dark blue-green solid.

The obtained compound N-15 was analyzed for an NMR spectrum. Here is the result.
[¹H NMR (CDCl₃)]

δ 8.74 (s, 1H), 8.73 (s, 1H), 8.69 (s, 1H), 8.68 (s, 1H), 7.91 (s, 1H), 7.90 (s, 1H), 7.49 (s, 2H), 7.46 (s, 1H), 7.21-7.14 (m, 16H), 7.03 (s, 1H), 6.67 (s, 1H), 6.64 (s, 1H), 2.64-2.59 (m, 8H), 2.24-2.17(m, 2H), 1.99-1.86 (m, 8H), 1.76-1.59 (m, 10H), 1.38-1.26 (m, 28H), 1.24-1.12 (br, 36H), 0.89-0.83 (m, 18H).

### <Synthesis Example 29> (Synthesis of Compound 63)

A compound 62 represented by the following formula was used to synthesize a compound 63 represented by the following formula.

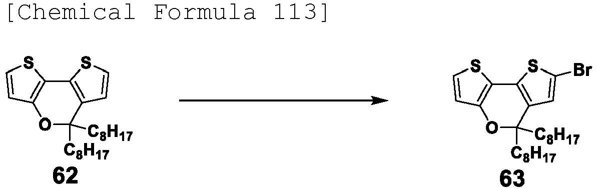

In a 300 mL four-necked flask whose internal atmosphere was replaced with a nitrogen gas, 5.02 g (12.0 mmol) of the compound 62 synthesized by the method described in paragraphs [0261] to [0271] of WO 2011/052709 A and 100 mL of a tetrahydrofuran were put, and cooled down to -30°C.

Next, 2.11 g (11.9 mmol) of N-bromosuccinimide was added to the four-necked flask, and stirred at -30°C for 6 hours.

Then, the temperature of the obtained solution was increased up to ordinary temperature, and the solution was reacted by further stirring for 3 hours at ordinary temperature, and a 3% aqueous sodium sulfite solution was then further added to stop the reaction.

The obtained reaction liquid was extracted with a hexane, and then washed with water and a saturated aqueous sodium chloride solution to obtain an organic layer.

Next, the obtained organic layer was dried with a magnesium sulfate, and then subjected to filtration, and the solvent was further distilled away under reduced pressure.

The obtained crude product was purified with a silica gel column to obtain 4.66 g (9.36 mmol, 78.0% in yield) of the compound 63 as a light yellow oil.

The obtained compound 63 was analyzed for an NMR spectrum. Here is the result.
[¹H NMR (CDCl₃)]

δ 6.98 (d, J_{HH}=4.8Hz, 1H), 6.66 (s, 1H),6.65 (d, J_{HH}=4.8Hz, 1H), 1.87-1.74(m, 4H), 1.41-1.23 (br, 24H), 0.86 (t, 6H) .

### <Synthesis Example 30> (Synthesis of Compound 64)

The compound 63 represented by the following formula was used to synthesize a compound 64 represented by the following formula.

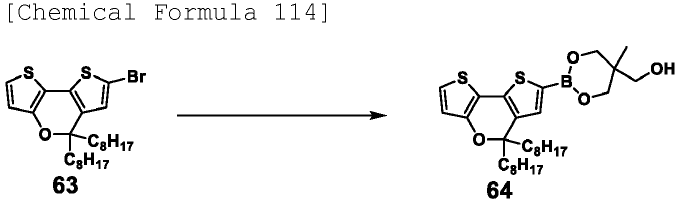

Into a 100 mL three-necked flask whose internal atmosphere was replaced with a nitrogen gas, 4.59 g (9.23 mmol) of the compound 63, and 90 mL of a dehydrated tetrahydrofuran were added, and the obtained solution was cooled down to -70°C, and then with 5.8 mL of an n-butyllithium solution (1.64 mol/L, hexane solution) added thereto, stirred for 1 hour.

Next, with the reaction liquid kept at -70°C, 2.43 g (12.9 mmol) of a trimethoxyborane was added, and stirred for 2 hours.

Next, a 10% by mass acetic acid aqueous solution (30 mL) was put into the obtained reaction liquid, and a separation operation was performed with the use of an ethyl acetate to extract the organic layer. The obtained organic layer was, with toluene (20 mL) and 1.66 g (13.82 mmol) of a 2-hydroxymethylene-2-methyl-1,3-propanediol added thereto, subjected to a dehydration operation with a Dean-Stark tube used, for 30 minutes. Furthermore, the solvent was removed with a rotary evaporator, thereby providing, as a green oil, 5.00 g of a crude product for the compound 64.

### <Synthesis Example 31> (Synthesis of Compound 66)

The compound 64 and a compound 65 represented by the following formula were used to synthesize a compound 66 represented by the following formula.

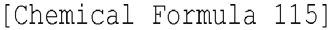

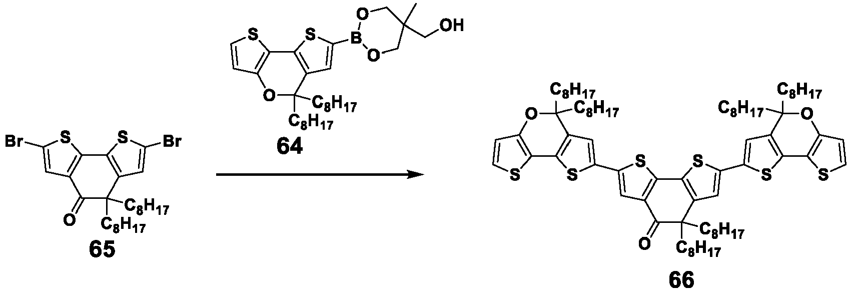

In a 200 mL four-necked flask whose internal atmosphere was replaced with a nitrogen gas, 1.65 g (2.80 mmol) of the compound 65 synthesized by the method described in the literature (ACS Omega 2017, 2, 4347.), 3.83 g (7.00 mmol) of the compound 64 and 93 mL of a tetrahydrofuran were put, and subjected to degassing by bubbling with an argon gas for 30 minutes.

Then, in the four-necked flask, 0.128 g (0.140 mmol, 5 mol%) of tris(dibenzylideneacetone) dipalladium (0), 0.170 g (0.560 mmol, 20 mol%) of a tri-tert-butylphosphonium tetrafluoroborate, and 5 mL of a tetrahydrofuran were put, and stirred for 5 minutes.

Next, 9.3 mL of a 3.0 M aqueous potassium phosphate solution was added to the four-necked flask, and the reaction liquid was stirred on heating for 3 hours in an oil bath at a set temperature of 50°C.

After cooling the reaction liquid, 100 mL of water and 100 mL of hexane were added to the four-necked flask, and the organic layer was separated and washed with water three times and with a saturated aqueous sodium chloride solution one time.

The obtained solution was dried with the use of an anhydrous sodium sulfate, and then filtered, and the solvent was distilled away under reduced pressure. The obtained crude product was purified with a silica gel column to obtain 3.21 g (2.54 mmol, 90.6% in yield) of the compound 66 as a red oil.

The obtained compound 66 was analyzed for an NMR spectrum. Here is the result.
[¹H NMR (CDCl₃)]

δ 7.44 (s, 1H), 7.03 (d, J_{HH}=4.0Hz, 1H),7.02 (d, J_{HH}=4.0Hz, 1H), 6.97 (s, 1H), 6.83 (s, 1H), 6.80 (s, 1H), 6.89 (d, J_{HH}=4.8Hz, 1H), 6.68 (d, J_{HH}=4.8Hz, 1H),2.23-2.16(m, 2H), 1.94-1.81 (m, 8H), 1.77-1.69 (m, 2H), 1.44-1.11 (br, 72H), 0.86-0.80 (m, 18H).

### <Synthesis Example 32> (Synthesis of Compound 67)

The compound 66 represented by the following formula was used to synthesize a compound 67 represented by the following formula.

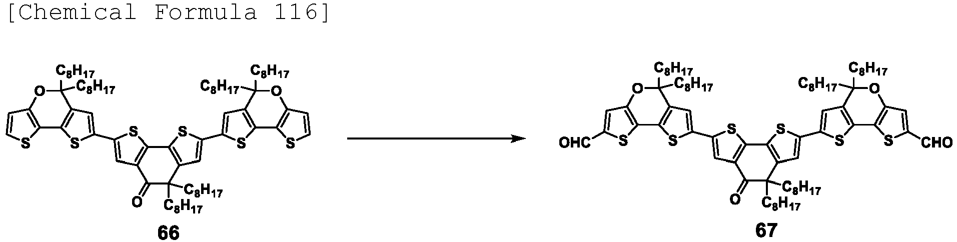

In a 100 mL three-necked flask whose internal atmosphere was replaced with a nitrogen gas, 3.41 g (2.70 mmol) of the compound 66 and 80 mL of a chloroform were put, and stirred at ordinary temperature for 10 minutes to dissolve the compound 66.

Next, in the three-necked flask, 1.04 g (8.10 mmol) of a (chloromethylene)dimethyliminium chloride was put, and stirred on heating for 2 hours in an oil bath at a set temperature of 65°C.

Then, after cooling the reaction liquid, 20 mL of water was added thereto, and the organic layer was separated and washed two times with a saturated aqueous sodium chloride solution. The obtained solution was dried with the use of an anhydrous sodium sulfate, and then subjected to filtration, and the solvent was distilled away under reduced pressure.

The obtained crude product was purified with a silica gel column to obtain 2.97 g (2.25 mmol, 83.3% in yield) of the compound 67 as a dark red solid.

The obtained compound 67 was analyzed for an NMR spectrum. Here is the result.
[¹H NMR (CDCl₃)]

δ 9.79 (s, 1H), 9.78 (s, 1H), 7.53 (s, 1H), 7.29 (s, 1H), 7.28 (s, 1H), 7.06 (s, 1H), 6.87 (s, 1H), 2.25-2.17(m, 2H), 1.96-1.84 (m, 8H), 1.78-1.71 (m, 2H), 1.44-1.12 (br, 72H), 0.87-0.79 (m, 18H).

### <Example 16> (Synthesis of Compound N-16)

The compound 12 and compound 67 represented by the following formula were used to synthesize a compound 68 (N-16) represented by the following formula.

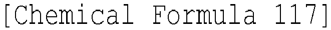

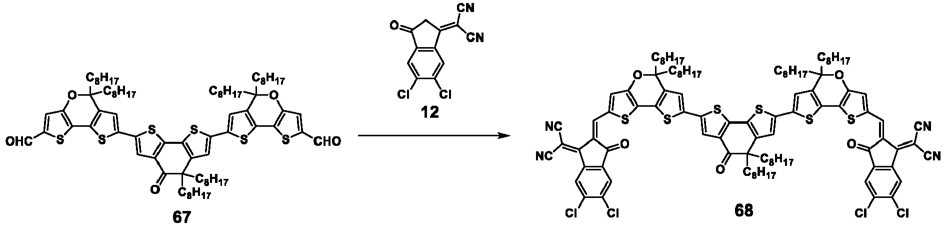

In a 100 mL four-necked flask whose internal atmosphere was replaced with a nitrogen gas, 0.858 g (0.650 mmol) of the compound 67, 0.513 g (1.95 mmol) of the compound 12, 30 mL of a chloroform, and 0.514 g (6.50 mmol) of a pyridine were put, and stirred on heating for 2 hours in an oil bath at 65°C.

The obtained solution was cooled down to ordinary temperature, and water was added to the solution to stop the reaction. The obtained solution was extracted with a chloroform, and then washed two times with water and one time with a saturated aqueous sodium chloride solution to obtain an organic layer.

Next, the obtained organic layer was dried with a magnesium sulfate, and subjected to filtration, and the solvent was further distilled away under reduced pressure.

The obtained crude product was purified with a silica gel column to obtain 0.967 g (0.534 mmol, 82.2% in yield) of the compound 68 (compound N-16) as a dark blue-green solid.

The obtained compound N-16 was analyzed for an NMR spectrum. Here is the result.
[¹H NMR (CDCl₃)]

δ 8.74 (s, 4H), 7.92 (s, 1H), 7.91 (s, 1H), 7.63 (s, 1H), 7.40 (s, 1H), 7.38 (s, 1H), 7.15 (s, 1H), 6.94 (s, 1H), 6.91 (s, 1H), 2.29-2.22(m, 2H), 1.99-1.86 (m, 8H), 1.82-1.75 (m, 2H), 1.44-1.14 (br, 72H), 0.92-0.80 (m, 18H).

### <Preparation Example 1> (Preparation of Ink I-1)

The polymer compound P-1 as a p-type semiconductor material and the compound N-1 as an n-type semiconductor material were mixed with an o-dichlorobenzene (ODCB) as a solvent respectively so as to reach a concentration of 0.8% by weight with respect to the total ink weight and a concentration of 0.8% by weight with respect to the ink total weight (p-type semiconductor material/n-type semiconductor material = 1/1), and the mixture liquid obtained by stirring at 60°C for 8 hours was filtered with the use of a filter to obtain an ink (I-1).

### <Preparation Example 2> (Preparation of Ink I-2)

The polymer compound P-1 as a p-type semiconductor material and the compound N-2 as an n-type semiconductor material were mixed with an ODCB as a solvent respectively so as to reach a concentration of 1.2% by weight with respect to the total ink weight and a concentration of 1.2% by weight with respect to the ink total weight (p-type semiconductor material/n-type semiconductor material = 1/1), and the mixture liquid obtained by stirring at 60°C for 8 hours was filtered with the use of a filter to obtain an ink (I-2).

### <Preparation Examples 3 to 17> (Preparation of inks (1-3) to (1-17))

Inks (I-3) to (I-17) were prepared in the same manner as in Preparation Example 2 except that a p-type semiconductor material and an n-type semiconductor material were used in combination as shown in Table 5 below.

### <Preparation Example 18>

The polymer compound P-1 as a p-type semiconductor material and the compound N-2 as an n-type semiconductor material were mixed with an o-xylene (oXY) respectively so as to reach a concentration of 1.2% by weight with respect to the total ink weight and a concentration of 1.2% by weight with respect to the ink total weight (p-type semiconductor material/n-type semiconductor material = 1/1), and the mixture liquid obtained by stirring at 60°C for 8 hours was filtered with the use of a filter to obtain an ink (I-18).

### <Preparation Example 19>

A mixed solvent was prepared with the use of an o-xylene as a first solvent and an acetophenone (AP) as a second solvent at a volume ratio of 97 : 3 between the first solvent to the second solvent. The polymer compound P-1 as a p-type semiconductor material and the compound N-2 as an n-type semiconductor material were mixed with the mixed solvent prepared, respectively so as to reach a concentration of 1.2% by weight with respect to the total ink weight and a concentration of 1.2% by weight with respect to the ink total weight (p-type semiconductor material/n-type semiconductor material = 1/1), and the mixture liquid obtained by stirring at 60°C for 8 hours was filtered with the use of a filter to obtain an ink (I-19).

### <Preparation Example 20>

A mixed solvent was prepared with the use of an o-xylene as a first solvent and a methyl benzoate (MBZ) as a second solvent at a volume ratio of 97 : 3 between the first solvent to the second solvent. The polymer compound P-1 as a p-type semiconductor material and the compound N-12 as an n-type semiconductor material were mixed with the mixed solvent prepared, respectively so as to reach a concentration of 1.2% by weight with respect to the total ink weight and a concentration of 1.2% by weight with respect to the ink total weight (p-type semiconductor material/n-type semiconductor material = 1/1), and the mixture liquid obtained by stirring at 60°C for 8 hours was filtered with the use of a filter to obtain an ink (I-20).

### <Preparation Examples 21 to 24> (Preparation of inks (I-21) to (1-24))

Inks (I-21) to (I-24) were prepared in the same manner as in Preparation Example 20 except that a p-type semiconductor material and an n-type semiconductor material were used in combination as shown in Table 5 below.

### [Table 5]

**(Table 5)**

| | Ink | Solvent | p-type semiconductor material | n-type semiconductor material |
|---|---|---|---|---|
| Preparation Example 1 | I-1 | ODCB | P-1 | N-1 |
| Preparation Example 2 | I-2 | ODCB | P-1 | N-2 |
| Preparation Example 3 | I-3 | ODCB | P-2 | N-3 |
| Preparation Example 4 | I-4 | ODCB | P-1 | N-3 |
| Preparation Example 5 | I-5 | ODCB | P-2 | N-3 |
| Preparation Example 6 | I-6 | ODCB | P-1 | N-4 |
| Preparation Example 7 | I-7 | ODCB | P-2 | N-4 |
| Preparation Example 8 | I-8 | ODCB | P-1 | N-5 |
| Preparation Example 9 | I-9 | ODCB | P-1 | N-6 |
| Preparation Example 10 | I-10 | ODCB | P-1 | N-7 |
| Preparation Example 11 | I-11 | ODCB | P-1 | N-8 |
| Preparation Example 12 | I-12 | ODCB | P-3 | N-2 |
| Preparation Example 13 | I-13 | ODCB | P-4 | N-2 |
| Preparation Example 14 | I-14 | ODCB | P-5 | N-2 |
| Preparation Example 15 | I-15 | ODCB | P-6 | N-2 |
| Preparation Example 16 | I-16 | ODCB | P-7 | N-2 |
| Preparation Example 17 | I-17 | ODCB | P-1 | N-11 |
| Preparation Example 18 | I-18 | oXY | P-1 | N-2 |
| Preparation Example 19 | I-19 | oXY/AP | P-1 | N-2 |
| Preparation Example 20 | I-20 | oXY/MBZ | P-1 | N-12 |
| Preparation Example 21 | I-21 | oXY/MBZ | P-1 | N-13 |
| Preparation Example 22 | I-22 | oXY/MBZ | P-1 | N-14 |
| Preparation Example 23 | I-23 | oXY/MBZ | P-1 | N-15 |
| Preparation Example 24 | I-24 | oXY/MBZ | P-1 | N-16 |

### <Example 17> (Manufacture and Evaluation of Photoelectric Conversion Element)

### (1) Manufacture of Photoelectric Conversion Element and Sealed Body thereof

A glass substrate with an ITO thin film (anode) of 50 nm in thickness formed by a sputtering method was prepared, and the glass substrate was subjected to an ozone UV treatment as a surface treatment.

Next, the ink (I-1) was applied onto the thin film of ITO by a spin coating method to form a coating film, and then dried by a heat treatment for 10 minutes with the use of a hot plate heated to 100°C under a nitrogen gas atmosphere to form an active layer (pre-baking step). The thickness of the active layer formed was about 150 nm.

Next, in a resistance heating vapor deposition apparatus, a calcium (Ca) layer with a thickness of about 5 nm was formed on the formed active layer to provide an electron transport layer.

Then, a silver (Ag) layer with a thickness of about 60 nm was formed on the formed electron transport layer to provide a cathode.

Through the foregoing steps, a photoelectric conversion element was manufactured on the glass substrate.

Next, a UV curable sealing agent as a sealing material was applied onto the glass substrate as a support substrate so as to surround the periphery of the manufactured photoelectric conversion element, a glass substrate as a sealing substrate was bonded to the support substrate, and the photodetection element was then sealed by UV light irradiation in the gap between the support substrate and the sealing substrate to obtain a sealed body of the photoelectric conversion element. The planar shape of the photoelectric conversion element sealed in the gap between the support substrate and the sealing substrate as viewed from the thickness direction was a square of 2 mm × 2 mm. The sealed body obtained was provided as a sample 1.

### (2) Evaluation of Photoelectric Conversion Element

### (External Quantum Efficiency)

A reverse bias voltage of -5 V was applied to the manufactured Sample 1, and the external quantum efficiency (EQE) at the applied voltage was measured and then evaluated with the use of a solar simulator (CEP-2000, manufactured by Bunkoukeiki Co., Ltd). The result is shown in Table 6 below.

With respect to the EQE, first, the current value of a current generated in the case of irradiation with light of a constant number of photons (1.0 × 10¹⁶) every 20 nm in the wavelength range from 300 nm to 1200 nm was measured with the reverse bias voltage of -5 V applied to the sealed body of the photoelectric conversion element, and a spectrum of the EQE at wavelengths from 300 nm to 1200 nm was determined by a known method.

Next, among the multiple measured values obtained for every 20 nm, the measured value at the wavelength (λmax) closest to the absorption peak wavelength was defined as the value (%) of the EQE.

### <Example 18> (Manufacture and Evaluation of Photoelectric Conversion Element)

A sealed body of a photoelectric conversion element was manufactured and evaluated in the same manner as in Example 17 described already, except that the ink (I-2) was used instead of the ink (I-1), and that the thickness of the formed active layer was about 300 nm. The result is shown in Table 6 below.

### <Examples 19 to 40> (Manufacture and Evaluation of Photoelectric Conversion Elements)

Sealed bodies of photoelectric conversion elements were manufactured and evaluated in the same manner as in Example 18 described already, except that the inks (I-3) to (I-24) were used instead of the ink (1-2). The result is shown in Table 6 below.

### [Table 6]

**(Table 6)**

| | Ink | p-type semiconductor material | n-type semiconductor material | EQE |
|---|---|---|---|---|
| Example 17 | I-1 | P-1 | N-1 | 4% |
| Example 18 | I-2 | P-1 | N-2 | 14% |
| Example 19 | I-3 | P-2 | N-2 | 50% |
| Example 20 | I-4 | P-1 | N-3 | 4% |
| Example 21 | I-5 | P-2 | N-3 | 8% |
| Example 22 | I-6 | P-1 | N-4 | 8% |
| Example 23 | I-7 | P-2 | N-4 | 3% |
| Example 24 | I-8 | P-1 | N-5 | 11% |
| Example 25 | I-9 | P-1 | N-6 | 10% |
| Example 26 | I-10 | P-1 | N-7 | 30% |
| Example 27 | I-11 | P-1 | N-8 | 11% |
| Example 28 | I-12 | P-3 | N-2 | 4% |
| Example 29 | I-13 | P-4 | N-2 | 27% |
| Example 30 | I-14 | P-5 | N-2 | 21% |
| Example 31 | I-15 | P-6 | N-2 | 12% |
| Example 32 | I-16 | P-7 | N-2 | 23% |
| Example 33 | I-17 | P-1 | N-11 | 4% |
| Example 34 | I-18 | P-1 | N-2 | 31% |
| Example 35 | I-19 | P-1 | N-2 | 38% |
| Example 36 | I-20 | P-1 | N-12 | 13% |
| Example 37 | I-21 | P-1 | N-13 | 22% |
| Example 38 | I-22 | P-1 | N-14 | 5% |
| Example 39 | I-23 | P-1 | N-15 | 32% |
| Example 40 | I-24 | P-1 | N-16 | 39% |

### <Examples 41 to 48> Evaluation of Photoelectric Conversion Elements (Heat Resistance)

Each of the photoelectric conversion elements according to Examples 17 to 21, 24 to 25, and 33 manufactured as mentioned above was evaluated for heat resistance with the EQE as an index.

Specifically, a value (EQE220°C/EQE100°C) was evaluated, obtained from normalization by dividing the value of the EQE in the photoelectric conversion element (sample 1) subjected to only the already described pre-baking step as a reference, with the heating temperature of 100°C in the pre-baking step, by the value of the EQE in the photoelectric conversion element (sample 2) further subjected to the post-baking step at a heating temperature of 220°C. The results are shown in Table 7 below.

In this regard, the post-baking step was performed specifically by heat-treating the already described sample 1 for 50 minutes on a hot plate heated to 220°C under a nitrogen gas atmosphere.

### [Table 7]

**(Table 7)**

| | Ink | Heat Resistance | EQE_{220°C}/EQE_{100°C} |
|---|---|---|---|
| Example 41 | I-1 | ○ | 1.75 |
| Example 42 | I-2 | ○ | 1.14 |
| Example 43 | I-3 | ○ | 1.06 |
| Example 44 | I-4 | ○ | 2.25 |
| Example 45 | I-5 | ○ | 4.50 |
| Example 46 | I-8 | ○ | 1.15 |
| Example 47 | I-9 | ○ | 1.14 |
| Example 48 | I-17 | ○ | 1.50 |

As is clear from Table 7, for each of the "samples 2" according to Examples 41 to 48, the EQE_{220°C}/EQE_{100°C} is 0.85 or more, which reaches 1.06 to 4.50, and it has been thus determined that the EQE rather has a tendency to be improved without any decrease in the value of the EQE when the heating temperature in the post-baking step is 220°C. Accordingly, the evaluation of heat resistance can be considered as "good (o)" in the range of 100°C to 220°C.

### <Examples 49 to 53> Evaluation of Photoelectric Conversion Elements (Heat Resistance)

Each of the photoelectric conversion elements according to Examples 36 to 40 manufactured as mentioned above was evaluated for heat resistance with the EQE as an index.

Specifically, a value (EQE200°C/EQE100°C) was evaluated, obtained from normalization by dividing the value of the EQE in the photoelectric conversion element (sample 1) subjected to only the already described pre-baking step as a reference, with the heating temperature of 100°C in the pre-baking step, by the value of the EQE in the photoelectric conversion element (sample 3) further subjected to the post-baking step at a heating temperature of 200°C. The results are shown in Table 8 below.

In this regard, the post-baking step was performed specifically by heat-treating the already described sample 1 for 50 minutes on a hot plate heated to 200°C under a nitrogen gas atmosphere.

### [Table 8]

**(Table 8)**

| | Ink | Heat Resistance | EQE_{200°C}/EQE_{100°C} |
|---|---|---|---|
| Example 49 | I-20 | ○ | 1.54 |
| Example 50 | I-21 | ○ | 0.86 |
| Example 51 | I-22 | ○ | 1.20 |
| Example 52 | I-23 | ○ | 0.91 |
| Example 53 | I-24 | ○ | 0.85 |

As is clear from Table 8, for each of the "samples 3" according to Examples 49 to 53, the EQE_{200°C}/EQE_{100°C} is 0.85 or more, which reaches 0.85 to 1.54, and it has been thus determined that the EQE may be rather improved in some cases almost without any decrease in the value of the EQE when the heating temperature in the post-baking step is 200°C. Accordingly, the evaluation of heat resistance can be considered as "good (o)" in the range of 100°C to 200°C.

### <Comparative Adjustment Examples>

Inks (C-1) to (C-2) were prepared by the same method as in Preparation Example 1 except that an n-type semiconductor material and a p-type semiconductor material were used in combination as shown in Table 9 below.

### [Table 9]

**(Table 9)**

| | Ink | p-type semiconductor material | n-type semiconductor material |
|---|---|---|---|
| Comparative Adjustment Example 1 | C-1 | P-1 | N-17 |
| Comparative Adjustment Example 2 | C-2 | P-1 | N-18 |

### <Comparative Examples 1 to 2> Evaluation of Photoelectric Conversion Elements (Heat Resistance)

Sealed bodies of photoelectric conversion elements were manufactured in the same manner as in Example 18 described already, except that the inks (C-1) to (C-2) were used instead of the ink (I-2), and each of the photoelectric conversion elements was evaluated for heat resistance with the EQE as an index in the same manner as in Examples 27 to 31 described already. The results are shown in Table 10 below.

### [Table 10]

**(Table 10)**

| | Ink | Heat Resistance | EQE_{220°C}/EQE_{100°C} |
|---|---|---|---|
| Comparative Example 1 | C-1 | × | 0.54 |
| Comparative Example 2 | C-2 | × | 0.50 |

In the evaluation of the heat resistance of "samples 2" according to Comparative Examples 1 to 2, as is clear from Table 10, the value of "EQE_{220°C}/EQE_{100°C}" is less than 0.8 in the case of at least heating at 220°C, and it has been thus determined that the value of the EQE is decreased by the heating treatment at the heating temperature of 220°C in the post-baking step. Accordingly, the evaluation of the heat resistance with the EQE as an index according to Comparative Examples 1 to 2 is "defective (×)".

### <Comparative Preparation Examples 3 to 4>

Inks (C-3) to (C-4) were prepared by the same method as in Preparation Example 20 except that an n-type semiconductor material and a p-type semiconductor material were used in combination as shown in Table 11 below.

### [Table 11]

**(Table 11)**

| | Ink | p-type semiconductor material | n-type semiconductor material |
|---|---|---|---|
| Comparative Preparation Example 3 | C-3 | P-1 | N-17 |
| Comparative Preparation Example 4 | C-4 | P-1 | N-18 |

### <Comparative Examples 3 to 4> Evaluation of Photoelectric Conversion Elements (Heat Resistance)

Sealed bodies of photoelectric conversion elements were manufactured in the same manner as in Example 18 described already, except that the inks (C-3) to (C-4) were used instead of the ink (I-2), and each of the photoelectric conversion elements was evaluated for heat resistance with the EQE as an index in the same manner as in Examples 49 to 53 described already. The results are shown in Table 12 below.

### [Table 12]

**(Table 12)**

| | Ink | Heat Resistance | EQE_{200°C}/EQE_{100°C} |
|---|---|---|---|
| Comparative Example 3 | C-3 | × | 0.49 |
| Comparative Example 4 | C-4 | × | 0.33 |

In the evaluation of the heat resistance of "samples 4" according to Comparative Examples 3 to 4, as is clear from Table 12, the value of "EQE_{200°C}/EQE_{100°C}" is less than 0.5 in the case of at least heating at 200°C, and it has been thus determined that the value of the EQE is decreased by the heating treatment at the heating temperature of 200°C in the post-baking step. Accordingly, the evaluation of the heat resistance with the EQE as an index according to Comparative Examples 3 to 4 is "defective (×)".

### DESCRIPTION OF REFERENCE SIGNS

- 1: image detection unit
- 2: display device
- 10: photoelectric conversion element
- 11, 210: support substrate
- 12: anode
- 13: hole transport layer
- 14: active layer
- 15: electron transport layer
- 16: cathode
- 17: sealing member
- 20: CMOS transistor substrate
- 30: interlayer insulating film
- 32: Interlayer wiring part
- 40: sealing layer
- 42: scintillator
- 44: reflective layer
- 46: protective layer
- 50: color filter
- 100: fingerprint detection unit
- 200: display panel unit
- 200a: display area
- 220: organic EL element
- 230: touch sensor panel
- 240: sealing substrate
- 300: vein detection unit
- 302: glass substrate
- 304: light source part
- 306: cover part
- 310: insertion part
- 400: image detection unit for TOF-type distance measuring device
- 402: floating diffusion layer
- 404: photogate
- 406: light-shielding part

## Claims

1. A compound represented by the following formula (I) :
A¹-B¹-A¹ (I)
(In the formula (I),
A¹ represents an electron-withdrawing group,
B¹ is a divalent group including two or more constituent units that are linked by a single bond to constitute a π-conjugated system, at least one of the two or more constituent units is a first constituent unit represented by the following formula (II), and the remaining second constituent unit other than the first constituent unit is a divalent group including an unsaturated bond, an arylene group, or a heteroarylene group.
The two groups A¹ may be identical to or different from each other. In the case of having two or more first constituent units, the two or more first constituent units may be identical to or different from each other. In the case of having two or more second constituent units, the two or more second constituent units may be identical to or different from each other.) 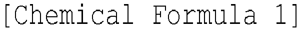 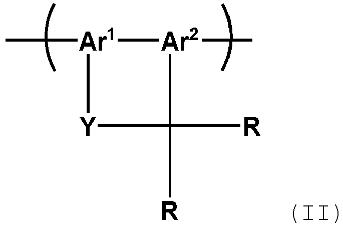 (In the formula (II),
Ar¹ and Ar² each independently represent an aromatic carbocyclic ring optionally having a substituent or an aromatic heterocyclic ring optionally having a substituent,
Y represents a group represented by -C(=O)- or an oxygen atom,
the groups R each independently represent
a hydrogen atom,
a halogen atom,
an alkyl group optionally having a substituent,
a cycloalkyl group optionally having a substituent,
an aryl group optionally having a substituent,
an alkyloxy group optionally having a substituent,
a cycloalkyloxy group optionally having a substituent,
an aryloxy group optionally having a substituent,
an alkylthio group optionally having a substituent,
a cycloalkylthio group optionally having a substituent,
an arylthio group optionally having a substituent,
a monovalent heterocyclic group optionally having a substituent,
a substituted amino group optionally having a substituent,
an acyl group optionally having a substituent,
an imine residue optionally having a substituent,
an amide group optionally having a substituent,
an acid imide group optionally having a substituent,
a substituted oxycarbonyl group optionally having a substituent,
an alkenyl group optionally having a substituent,
a cycloalkenyl group optionally having a substituent,
an alkynyl group optionally having a substituent,
a cycloalkynyl group optionally having a substituent,
a cyano group,
a nitro group,
a group represented by -C(=O)-R^{a}, or
a group represented by -SO₂-R^{b}, and
R^{a} and R^{b} each independently represent
a hydrogen atom,
an alkyl group optionally having a substituent,
an aryl group optionally having a substituent,
an alkyloxy group optionally having a substituent,
an aryloxy group optionally having a substituent, or
a monovalent heterocyclic group optionally having a substituent.
the multiple groups R may be identical to or different from each other.).

2. The compound according to claim 1, wherein B¹ comprises two or more of the first constituent units.

3. The compound according to claim 1 or 2, wherein the first constituent unit is a constituent unit represented by the following formula (III): 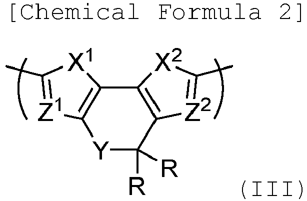 (In the formula (III),
Y and R are as defined above,
X¹ and X² each independently represent a sulfur atom or an oxygen atom, and
Z¹ and Z² each independently represent a group represented by =C(R)- or a nitrogen atom.).

4. The compound according to any one of claims 1 to 3, wherein B¹ comprises three or more of the first constituent units.

5. The compound according to any one of claims 1 to 4, wherein the second constituent unit is selected from the group consisting of a divalent group including an unsaturated bond and groups represented by the following formulas (IV-1) to (IV-9): 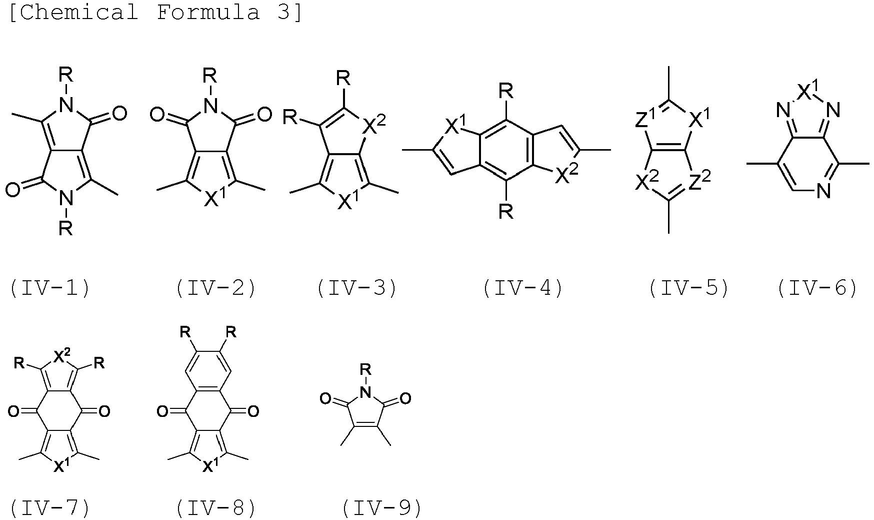
(In the formulas (IV-1) to (IV-9), X¹, X², Z¹, Z², and R are as defined above.
In the case of having two groups R, the two groups R may be identical to or different from each other.).

6. The compound according to any one of claims 1 to 3, wherein B¹ is a divalent group having the two or more and four or less first constituent units linked.

7. The compound according to claim 5, wherein B¹ is a divalent group that has any one structure selected from the group consisting of structures represented by the following formulas (V-1) to (V-9):
-CU1-CU2-CU1- (V-1)
-CU1-CU2-CU1-CU2-CU1- (V-2)
-CU2-CU1-CU2-CU1-CU2- (V-3)
-CU1-CU2-CU1-CU2-CU1-CU2-CU1- (V-4)
-CU1-CU1- (V-5)
-CU2-CU1-CU2- (V-6)
-CU1-CU1-CU1- (V-7)
-CU2-CU1-CU1-CU2- (V-8)
-CU2-CU1-CU1-CU1-CU2- (V-9)
(In the formulas (V-1) to (V-9),
CU1 represents the first constituent unit, and
CU2 represents the second constituent unit.
In the case of having two or more units CU1, the two or more units CU1 may be identical to or different from each other, and in the case of having two or more units CU2, the two or more units CU2 may be identical to or different from each other.).

8. A composition comprising a p-type semiconductor material and an n-type semiconductor material, wherein the n-type semiconductor material comprises the compound according to any one of claims 1 to 7.

9. An ink comprising the composition according to claim 8 and a solvent.

10. A photoelectric conversion element comprising: an anode; a cathode; and an active layer provided between the anode and the cathode and comprising a p-type semiconductor material and an n-type semiconductor material, wherein the n-type semiconductor material comprises the compound according to any one of claims 1 to 7.

11. The photoelectric conversion element according to claim 10, wherein the photoelectric conversion element is a photodetection element.

12. An image sensor comprising the photoelectric conversion element according to claim 11.

13. A fingerprint authentication device comprising the photoelectric conversion element according to claim 11.

14. A vein authentication device comprising the photoelectric conversion element according to claim 11.
